(19) Europäisches Patentamt
European Patent Office
Office européen des brevets

(11) EP 4 722 203 A1

(12) EUROPEAN PATENT APPLICATION
published in accordance with Art. 153(4) EPC

(43) Date of publication:
**08.04.2026 Bulletin 2026/15**

(21) Application number: **24818612.4**

(22) Date of filing: **03.06.2024**

(51) International Patent Classification (IPC):
***C07D 401/04*** (2006.01) ***C07D 403/04*** (2006.01)
***A61K 31/502*** (2006.01) ***A61K 31/5377*** (2006.01)
***A61K 45/06*** (2006.01) ***A61P 35/00*** (2006.01)
***A61P 35/02*** (2006.01) ***A61P 29/00*** (2006.01)
***A61P 25/00*** (2006.01) ***A61P 37/02*** (2006.01)

(86) International application number:
**PCT/CN2024/097038**

(87) International publication number:
**WO 2024/251080 (12.12.2024 Gazette 2024/50)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **05.06.2023 CN 202310656044**

(71) Applicant: **Academy of Military Medical Sciences**
**Beijing 100850 (CN)**

(72) Inventors:
- **ZHENG, Zhibing**
  **Beijing 100850 (CN)**
- **LI, Pengyun**
  **Beijing 100850 (CN)**
- **LI, Song**
  **Beijing 100850 (CN)**
- **HU, Xiaotong**
  **Beijing 100850 (CN)**
- **XIAO, Junhai**
  **Beijing 100850 (CN)**
- **ZHONG, Wu**
  **Beijing 100850 (CN)**
- **ZHOU, Xinbo**
  **Beijing 100850 (CN)**

(74) Representative: **PGA S.p.A., Milano, Succursale di Lugano**
**Via Castagnola, 21c**
**6900 Lugano (CH)**

# (54) 3,4-DIHYDROPHTHALAZIN-1(2H)-ONE DERIVATIVE, AND PREPARATION METHOD THEREFOR AND USE THEREOF

(57) The present invention relates to a 3,4-dihydrophthalazin-1(2H)-one compound as represented by formula (I), a pharmaceutically acceptable salt, a prodrug, a stable isotope derivative, an isomer, a solvate, or a polymorph form thereof, and a pharmaceutical composition comprising the compound described above. Further provided in the present invention is a method for preparing the compound as represented by formula (I). The compound is used as a modulator of targeted ubiquitination, especially for the treatment of diseases associated with a CRBN protein. The compound can also be used as a ligand of the CRL4[CRBN] E3 ubiquitin ligase for the preparation of a related proteolysis targeting chimera (PROTAC),

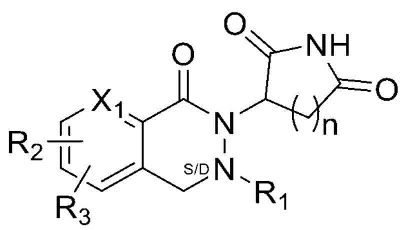

(I).

EP 4 722 203 A1

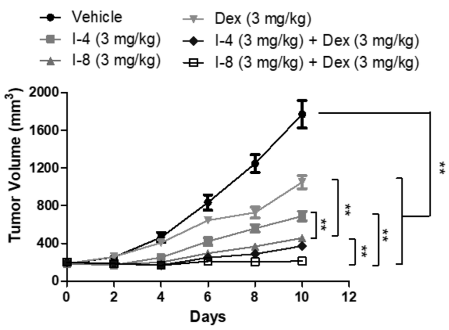
Vehicle
I-4 (3 mg/kg)
I-8 (3 mg/kg)
Dex (3 mg/kg)
I-4 (3 mg/kg) + Dex (3 mg/kg)
I-8 (3 mg/kg) + Dex (3 mg/kg)
Tumor Volume (mm3)
2000
1600
1200
800
400
0
0
2
4
6
8
10
12
Days
**
**
**
**
**

Figure 3

## Description

The present application is based on and claims priority to the Chinese application with application number 202310656044.9 (filed on June 5, 2023). The disclosure of the Chinese application is incorporated into the present application in its entirety.

## Technical Field

The present invention relates to a 3,4-dihydrophthalazin-1(2*H*)-one compound and structural derivative thereof, a pharmaceutically acceptable salt, prodrug, stable isotope derivative, isomer, solvate, or polymorph thereof, a preparation method therefor, and a use thereof as medicament. The compound can be used as a modulator targeting ubiquitination, particularly for the treatment of a disease related to CRBN protein.

## Background Art

Thalidomide and structural derivatives thereof, lenalidomide and pomalidomide, are known as immunomodulators (IMiDs). Thalidomide was initially marketed in Germany as a sedative but was banned due to its potent teratogenic effects. Scientists later discovered that it could effectively alleviate symptoms in patients with erythema nodosum leprosum and inhibit the expression of tumor necrosis factor during leprosy treatment. Thalidomide, lenalidomide, and pomalidomide were subsequently approved by the FDA for the treatment of multiple myeloma patients. Multiple studies have confirmed that CRBN (Cereblon) is the target of this class of immunomodulators.

CRBN is a "substrate acceptor" of an E3 ubiquitin ligase. After IMiDs act on CRBN, they can activate the E3 ubiquitin ligase, causing rapid ubiquitination of the substrate protein, which is then recognized and degraded by proteasome. Studies have shown that immunomodulators such as lenalidomide exert their antitumor effects in multiple myeloma cell lines by regulating the function of $CRL4^{CRBN}$ E3 ubiquitin ligase. After acting on CRBN, the immunomodulators induce the E3 ubiquitin ligase to recruit substrate proteins such as IKZF1/IKZF3 and label them with ubiquitin, leading to the recognition and degradation of these proteins by 26S proteasome. The degradation of IKZF1 and IKZF3 directly reduces the expression of transcription factors such as IRF4 and Myc, thus exerting cytotoxic effects on myeloma cells. On the other hand, the degradation of IKZF1 and IKZF3 increases the expression of IL-2 in T cells, thereby activating the T cell immune response and suppressing B cell function, achieving the effects of killing tumor and inhibiting tumor proliferation.

Research indicates that different immunomodulators exhibit different substrate protein degradation characteristics after binding to CRBN proteins. Lenalidomide, for example, primarily achieves its therapeutic effect in treating multiple myeloma by selectively degrading IKZF1 and IKZF3; while in treating myelodysplastic syndrome (del(5q)MDS), it mainly achieves its therapeutic effect by degrading CK1$\alpha$. With the research and development of new immunomodulators and the progress of clinical trials, the indications for thalidomide, lenalidomide and pomalidomide are constantly expanding. For instance, thalidomide is approved by FDA for the treatment of erythema nodosum leprosum, lenalidomide is used in clinical trials for the treatment of prostate cancer, and pomalidomide is used in clinical trials for the treatment of myelofibrosis.

However, domides drugs have many side effects. Lenalidomide's prescribing information clearly states that the drug has risks of bone marrow suppression, deep venous thrombosis, pulmonary embolism, and teratogenicity. During clinical trials, domides drugs exhibited significant hematologic toxicity, necessitating dose reduction in patient use. In other words, while lenalidomide possesses beneficial activity, its effectiveness is limited by the significant occurrence of side effects. Therefore, there is an urgent need in the field for a novel and highly efficient CRBN modulator to optimize, on the one hand, the performance of existing domides IMiDs and improve, on the other hand, the severe drug resistance problem.

## Contents of the invention

The present invention describes a 3,4-dihydrophthalazin-1(2*H*)-one compound, a preparation method therefor, and a use thereof. This compound can recruit endogenous proteins to E3 ubiquitin ligases for degradation, generating diverse physiological activities related to substrate proteins through protein degradation, thereby effectively preventing, improving, and treating cancers such as multiple myeloma. The compound of the present invention is used as a ligand for $CRL4^{CRBN}$ E3 ubiquitin ligase to prepare a related proteolysis targeting chimera (PROTAC).

A first aspect of the present invention provides a compound represented by formula (I), or pharmaceutically acceptable salt, prodrug, stable isotope derivative, isomer, solvate, or polymorph thereof:

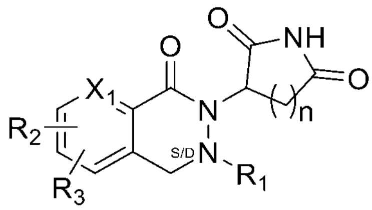

(I)

wherein: $X_1$ is N or CH; n is 1 or 2;

$R_1$ is selected from the group consisting of H, $C_{1-10}$ linear or branched alkyl and $C_{3-10}$ cycloalkyl;

S/D is a single bond or a double bond, and when it is a double bond, $R_1$ is absent;

$R_2$ and $R_3$ are each independently selected from the group consisting of H, D, halogen, substituted or unsubstituted alkyl, substituted or unsubstituted alkenyl, substituted or unsubstituted alkynyl, substituted or unsubstituted cycloalkyl, substituted or unsubstituted aryl, substituted or unsubstituted heteroaryl, substituted or unsubstituted heterocyclyl, -S-alkyl, -CN, $-NO_2$, $-N_3$, $-CH(Ph)_2$, perfluoro-$C_1$-$C_4$ alkyl, perfluoro-$C_1$-$C_4$ alkoxy, $-NR_4R_5$, $-OR_4$, $-COR_4$, $-CO_2R_4$, $-CONR_4R_5$, $-C(=NR_4)NR_5R_6$, $-NR_4COR_5$, $-NR_4CO_2R_5$, $-SO_2R_4$, $-NR_4SO_2NR_5R_6$, and $-NR_4SO_2R_5$;

$R_4$, $R_5$, and $R_6$ are each independently H, D, alkyl, heteroalkyl, alkenyl, alkynyl, cycloalkyl, saturated or unsaturated heterocyclyl, aryl, or heterocyclylaryl; wherein ($R_4$ and $R_5$) and/or ($R_5$ and $R_6$), together with the atoms to which they are connected, may each form a ring, and the ring is selected from the group consisting of optionally substituted or unsubstituted cycloalkyl, saturated or unsaturated heterocyclyl, aryl, and heterocyclylaryl.

In some embodiments, the $X_1$ is CH.
In some embodiments, the n is 2.
In some embodiments, the $R_1$ is selected from the group consisting of H and $C_{1-6}$ linear or branched alkyl.
In some embodiments, the $R_1$ is selected from the group consisting of H and $C_{1-4}$ linear or branched alkyl.
In some embodiments, the $R_1$ is a $C_{1-4}$ linear or branched alkyl.
In some embodiments, the $R_1$ is selected from the group consisting of methyl, ethyl, n-propyl, isopropyl, n-butyl, isobutyl, and tert-butyl.
In some embodiments, the $R_1$ is H.
In some embodiments, the $R_1$ is $CH_3$.
In some embodiments, the $R_2$ is selected from the group consisting of H and halogen.
In some embodiments, the $R_2$ is selected from the group consisting of H, F, Cl, Br, and I.
In some embodiments, the $R_2$ is selected from the group consisting of H and F.
In some embodiments, the $R_2$ is H.
In some embodiments, the S/D is a single bond.
In some embodiments, the $R_3$ is selected from the group consisting of H, D, halogen, substituted or unsubstituted alkyl, substituted or unsubstituted alkenyl, substituted or unsubstituted alkynyl, substituted or unsubstituted cycloalkyl, substituted or unsubstituted aryl, substituted or unsubstituted heteroaryl, substituted or unsubstituted heterocyclyl, -S-alkyl, -CN, $-NO_2$, $-N_3$, $-CH(Ph)_2$, perfluoro-$C_1$-$C_4$ alkyl, perfluoro-$C_1$-$C_4$ alkoxy, $-NR_4R_5$, $-OR_4$, $-COR_4$, $-CO_2R_4$, $-CONR_4R_5$, $-C(=NR_4)NR_5R_6$, $-NR_4COR_5$, $-NR_4CO_2R_5$, $-SO_2R_4$, $-NR_4SO_2NR_5R_6$, and $-NR_4SO_2R_5$;
$R_4$, $R_5$, and $R_6$ are each independently H, D, alkyl, heteroalkyl, alkenyl, alkynyl, cycloalkyl, saturated or unsaturated heterocyclyl, aryl, or heterocyclylaryl; wherein ($R_4$ and $R_5$) and/or ($R_5$ and $R_6$), together with the atoms to which they are connected, may each form a ring, and the ring is selected from the group consisting of optionally substituted or unsubstituted cycloalkyl, saturated or unsaturated heterocyclyl, aryl, and heterocyclylaryl.
In some embodiments, the substituents and chemical bonds in formula (I) are selected from the following:

(a) $X_1$ is CH, n is 2;

(b) $R_1$ is $CH_3$;

(c) $R_2$ is H;

(d) S/D is a single bond;

(e) $R_3$ is independently selected from the group consisting of H, D, halogen, substituted or unsubstituted alkyl, substituted or unsubstituted alkenyl, substituted or unsubstituted alkynyl, substituted or unsubstituted cycloalkyl, substituted or unsubstituted aryl, substituted or unsubstituted heteroaryl, substituted or unsubstituted heterocyclyl, -S-alkyl, -CN, $-NO_2$, $-N_3$, $-CH(Ph)_2$, perfluoro-$C_1$-$C_4$ alkyl, perfluoro-$C_1$-$C_4$ alkoxy, $-NR_4R_5$, $-OR_4$, $-COR_4$, $-CO_2R_4$, $-CONR_4R_5$, $-C(=NR_4)NR_5R_6$, $-NR_4COR_5$, $-NR_4CO_2R_5$, $-SO_2R_4$, $-NR_4SO_2NR_5R_6$, and $-NR_4SO_2R_5$;

$R_4$, $R_5$, and $R_6$ are each independently H, D, alkyl, heteroalkyl, alkenyl, alkynyl, cycloalkyl, saturated or unsaturated heterocyclyl, aryl, or heterocyclylaryl; wherein ($R_4$ and $R_5$) and/or ($R_5$ and $R_6$), together with the atoms to which they are connected, may each form a ring, and the ring is selected from the group consisting of optionally substituted or unsubstituted cycloalkyl, saturated or unsaturated heterocyclyl, aryl, and heterocyclylaryl.

In some embodiments, in formula (I), $R_3$ is further selected from the following: H, D, halogen, substituted or unsubstituted $C_1$-$C_4$ alkyl, substituted or unsubstituted $C_2$-$C_6$ alkenyl, substituted or unsubstituted $C_2$-$C_6$ alkynyl, substituted or unsubstituted $C_3$-$C_8$ cycloalkyl, substituted or unsubstituted aryl, substituted or unsubstituted heteroaryl, substituted or unsubstituted heterocyclyl, $-S-R_4$, -CN, $-NO_2$, perfluoro-$C_1$-$C_4$ alkyl, perfluoro-$C_1$-$C_4$ alkoxy, $-NR_4R_5$, $-OR_4$, $-COR_4$, $-CO_2R_4$, $-CONR_4R_5$, $-C(=NR_4)NR_5R_6$, $-NR_4COR_5$, $-NR_4CO_2R_5$, $-SO_2R_4$, $-NR_4SO_2NR_5R_6$, and $-NR_4SO_2R_5$; $R_4$, $R_5$, and $R_6$ are each independently H, D, alkyl, heteroalkyl, alkenyl, alkynyl, cycloalkyl, saturated or unsaturated heterocyclyl, aryl, or heterocyclylaryl; wherein ($R_4$ and $R_5$) and/or ($R_5$ and $R_6$), together with the atoms to which they are connected, may each form a ring, and the ring is selected from the group consisting of optionally substituted or unsubstituted cycloalkyl, saturated or unsaturated heterocyclyl, aryl, and heterocyclylaryl.

In some embodiments, the $R_3$ is selected from the group consisting of H, halogen, -OH, $-S-R_4$, -CN, $-NO_2$, and $-NR_4R_5$.

In some embodiments, the $R_3$ is selected from the group consisting of H, halogen, $-NO_2$, and $-NR_4R_5$.

In some embodiments, the $R_3$ is $-NR_4R_5$.

In some embodiments, the $R_4$ and $R_5$ are each independently H, D, $C_{1-4}$ alkyl, or $R_4$ and $R_5$ together with the atoms to which they are connected form a ring selected from the group consisting of optionally substituted or unsubstituted cycloalkyl, saturated or unsaturated heterocyclyl, aryl, and heterocyclylaryl.

In some embodiments, the $R_4$ and $R_5$ are each independently H, D, $C_{1-4}$ alkyl.

In some embodiments, the $R_4$ is H.

In some embodiments, the $R_5$ is $C_{1-4}$ alkyl.

In some embodiments, the $R_5$ is selected from the group consisting of methyl, ethyl, n-propyl, isopropyl, n-butyl, isobutyl, and tert-butyl.

In some embodiments, the $R_5$ is methyl.

In some embodiments, in formula (I), $R_3$ is further selected from the following: H, D, halogen, substituted or unsubstituted $C_1$-$C_4$ alkyl, substituted or unsubstituted $C_2$-$C_6$ alkenyl, substituted or unsubstituted $C_2$-$C_6$ alkynyl, substituted or unsubstituted $C_3$-$C_8$ cycloalkyl, substituted or unsubstituted aryl, substituted or unsubstituted heteroaryl, substituted or unsubstituted heterocyclyl, $-S-R_4$, -CN, $-NO_2$, perfluoro-$C_1$-$C_4$ alkyl, perfluoro-$C_1$-$C_4$ alkoxy, $-NR_4R_5$, $-OR_4$, $-COR_4$, $-CO_2R_4$, $-CONR_4R_5$, $-C(=NR_4)NR_5R_6$, $-NR_4COR_5$, $-NR_4CO_2R_5$, $-SO_2R_4$, $-NR_4SO_2NR_5R_6$, and $-NR_4SO_2R_5$;

$R_4$, $R_5$, and $R_6$ are each independently H, D, alkyl, heteroalkyl, alkenyl, alkynyl, cycloalkyl, saturated or unsaturated heterocyclyl, aryl, or heterocyclylaryl; wherein, ($R_4$ and $R_5$) and/or ($R_5$ and $R_6$), together with the atoms to which they are connected, may each form a ring as represented by formula (II),

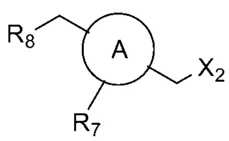

(II)

wherein, $X_2$ is selected from the group consisting of NH, $CH_2$, and O;

ring A is a 5- to 6-membered aromatic ring containing 0-3 heteroatoms selected from N, S, or O;

$R_7$ is selected from the group consisting of H, D, halogen, alkyl, heteroalkyl, alkenyl, alkynyl, cycloalkyl, saturated or unsaturated heterocyclyl, aryl, and heterocyclylaryl;

$R_8$ is selected from the group consisting of H, D, substituted or unsubstituted alkyl, substituted or unsubstituted alkenyl, substituted or unsubstituted alkynyl, substituted or unsubstituted cycloalkyl, substituted or unsubstituted aryl, substituted or unsubstituted heteroaryl, substituted or unsubstituted heterocyclyl, -S-alkyl, -CN, $-NO_2$, $-N_3$, $-CH(ph)_2$, perfluoro-$C_1$-$C_4$ alkyl, perfluoro-$C_1$-$C_4$ alkoxy, $-NR_9R_{10}$, $-OR_9$, $-COR_9$, $-CO_2R_9$, $-CONR_9R_{10}$, $-C(=NR_9)NR_9R_{10}$, $-NR_9COR_{10}$, $-NR_9CO_2R_{10}$, $-SO_2R_9$, $-NR_9SO_2NR_{10}R_{11}$, and $-NR_9SO_2R_{10}$;

$R_9$, $R_{10}$, and $R_{11}$ are each independently H, D, alkyl, heteroalkyl, alkenyl, alkynyl, cycloalkyl, saturated or unsaturated heterocyclyl, aryl, or heterocyclylaryl; wherein ($R_9$ and $R_{10}$) and/or ($R_{10}$ and $R_{11}$), together with the atoms to which they are connected, may each form a ring, and the ring is selected from the group consisting of optionally substituted or unsubstituted cycloalkyl, saturated or unsaturated heterocyclyl, aryl, and heterocyclylaryl.

In some embodiments, the $X_2$ end is a connecting site of formula (II).
In some embodiments, the $X_2$ is N.
In some embodiments, the ring A is a benzene ring.
In some embodiments, the $R_7$ is selected from the group consisting of H, D, and halogen.
In some embodiments, the $R_7$ is H.
In some embodiments, the $R_8$ is selected from the group consisting of H, D, substituted or unsubstituted 5- to 9-membered heterocyclyl and $-NR_9R_{10}$, wherein $R_9$ and $R_{10}$ are each independently H, D, and $C_{1-4}$ alkyl, or $R_9$ and $R_{10}$ together with the atoms to which they are connected form a substituted or unsubstituted 5- to 9-membered cycloalkyl. In some embodiments, $R_8$ is selected from the group consisting of substituted or unsubstituted 5- to 9-membered heterocyclyl. In some embodiments, the substituted 5- to 9-membered heterocyclyl is a 5- to 9-membered heterocyclyl substituted with one or more $R_{12}$, wherein the $R_{12}$ is as defined in any embodiment. In some embodiments, the 5- to 9-membered heterocyclyl comprises 1, 2, or 3 N atoms. In some embodiments, the 5- to 9-membered heterocyclyl is selected from the group consisting of morpholinyl and piperazinyl. In some embodiments, the 5- to 9-membered heterocyclyl is piperazinyl.
In some embodiments, $R_4$ and $R_5$ together with the atoms to which they are connected form a structural unit as represented by formula (II),

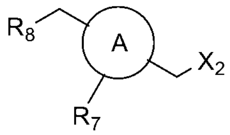

(II)

wherein, $R_7$, $R_8$, $X_2$, and ring A are as defined in any embodiment.
In some embodiments, the $R_3$ is selected from the group consisting of H, halogen, -OH, -SH, -CN, $-NO_2$, $-NH_2$, $-NH(C_{1-4}$ alkyl), and

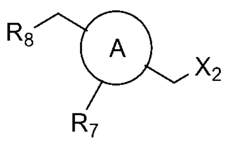 (formula (II)),

and the $R_7$, $R_8$, $X_2$, and ring A are as defined in any embodiment.
In some embodiments, $R_3$ is selected from the group consisting of H, halogen, $-NO_2$, $-NH_2$, $-NH(CH_3)$, and

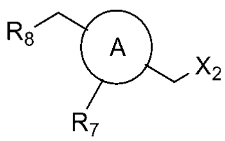 (formula (II)),

and the $R_7$, $R_8$, $X_2$, and ring A are as defined in any embodiment.
In some embodiments, the ring represented by formula (II) is further selected from the following:

ring A is a benzene ring;

$R_7$ is H;
$X_2$ is NH;
$R_8$ is a structure represented by formula (III):

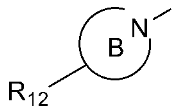

(III)

wherein ring B is an N-substituted non-aromatic ring, which is selected from the group consisting of any of the following structures:

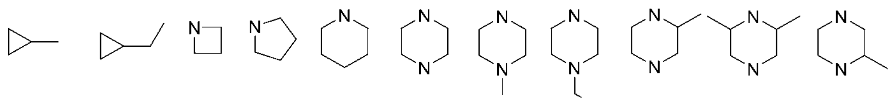

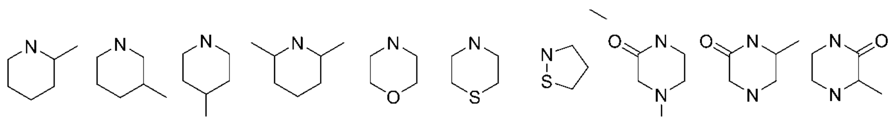

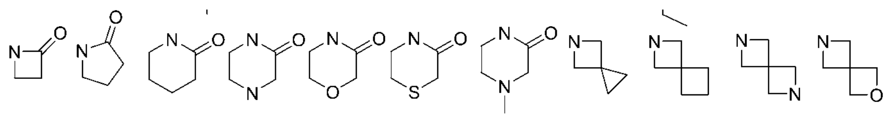

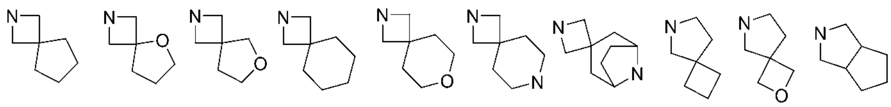

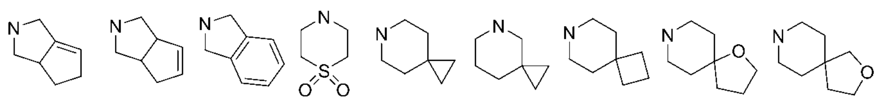

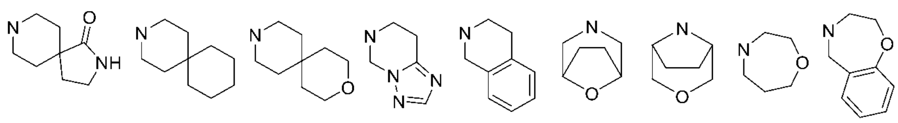 ;

$R_{12}$ is selected from the group consisting of H, D, halogen, -CN, $-NO_2$, alkyl, heteroalkyl, alkenyl, alkynyl, cycloalkyl, saturated or unsaturated heterocyclyl, aryl and heterocyclylaryl; wherein if the alkyl, heteroalkyl, alkenyl, alkynyl, cycloalkyl, saturated or unsaturated heterocyclyl, aryl or heterocyclylaryl is substituted, it is optionally substituted by at least one of substituent $R_{13}$, wherein $R_{13}$ is halogen, lower alkyl, lower alkoxy, cyano, or nitro.

In some embodiments, the N atom of ring B is a connecting site of formula (III).

In some embodiments, ring B is selected from the group consisting of any of the following structures:

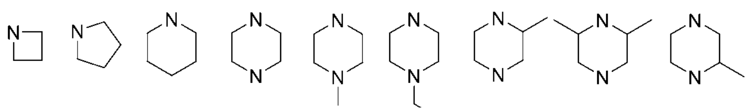

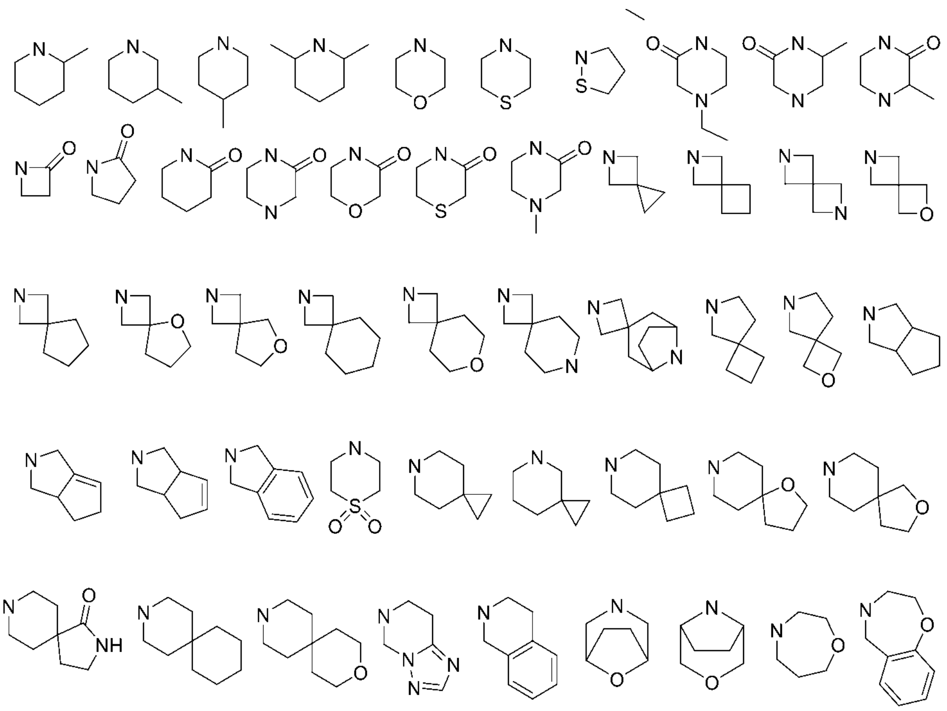

In some embodiments, ring B is selected from the group consisting of

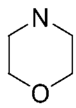 and 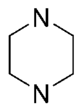.

In some embodiments, ring B is

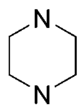.

In some embodiments, the $R_{12}$ is selected from the group consisting of H, D, halogen, -C(=O)-$C_{1-6}$ alkyl, phenyl, and 5- to 6-membered heteroaryl, wherein the phenyl and 5- to 6-membered heteroaryl are optionally substituted with one or more $R_{13}$, wherein the $R_{13}$ is selected from the group consisting of halogen, $C_{1-4}$ alkyl, cyano, and nitro.

In some embodiments, the $R_{12}$ is selected from the group consisting of H, D, halogen, -C(=O)-$C_{1-4}$ alkyl, and phenyl, wherein the phenyl is optionally substituted with one, two, or three $R_{13}$, wherein the $R_{13}$ is selected from the group consisting of F and cyano.

In some embodiments, the $R_{12}$ is selected from the group consisting of -C(=O)OC($CH_3$)$_3$, phenyl,

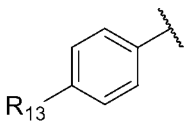 and 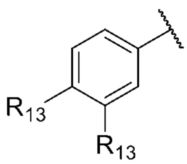,

wherein the $R_{13}$ is selected from the group consisting of F and cyano.

In some embodiments, the $R_{12}$ is selected from the group consisting of -C(=O)OC($CH_3$)$_3$, phenyl,

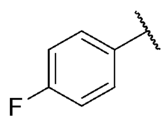, 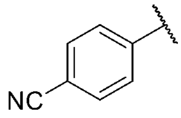 and 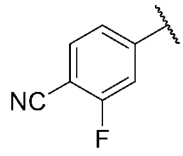 .

In some embodiments, in formula (III), ring B is a piperazine ring substituted with a lower alkyl, alkenyl, alkynyl, halogen, nitro, etc., or a substituted piperazine as represented by formula (IV):

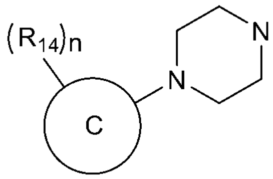

(IV)

wherein, ring C is a 5- to 6-membered aromatic ring, or an aromatic heterocyclyl containing 0-3 heteroatoms selected from N, S, or O;
$R_{14}$ is selected from the group consisting of H, D, halogen, lower alkyl, lower alkoxy, cyano, nitro, and hydroxyl;
n = 0, 1, 2, 3, 4, or 5.

In some embodiments, in formula (III), ring B is a substituted piperazine as represented by formula (IV):

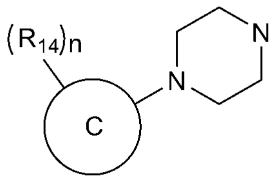

(IV)

wherein, ring C, $R_{14}$, and n are as defined in any embodiment of the present application.
In some embodiments, the N atom of piperazine is a connecting site of formula (IV).
In some embodiments, in formula (III), ring B is a piperazine ring substituted with $-C(=O)OC(CH_3)_3$ or a substituted piperazine as represented by formula (IV).
In some embodiments, the structural unit

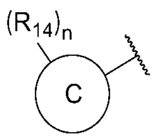

is selected from the group consisting of

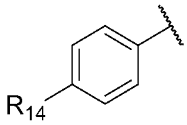 and 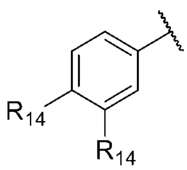 .

In some embodiments, the ring C is phenyl.
In some embodiments, the $R_{14}$ is selected from the group consisting of H, F, and cyano.
In some embodiments, the $R_{14}$ is selected from the group consisting of F and cyano.

In some embodiments, the structural unit

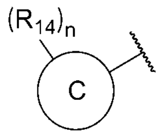

is selected from the group consisting of phenyl,

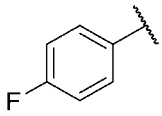, 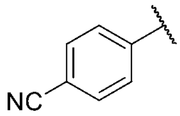 and 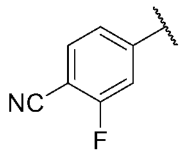.

In some embodiments, the structural unit

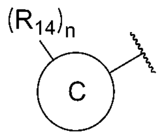

is

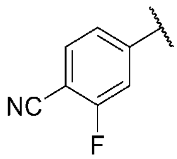.

In some embodiments, the compound is a compound represented by formula (I-1),

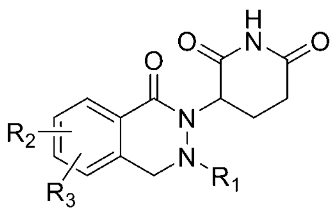

(I-1)

$R_1$, $R_2$, and $R_3$ are as defined in any embodiment.

In some embodiments, the structural unit

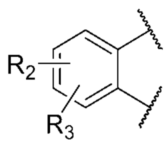

is selected from the group consisting of

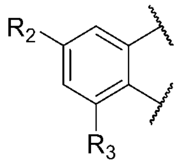

and

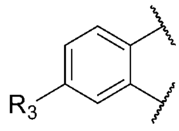

.

In some embodiments, the structural unit

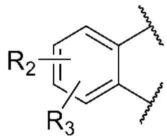

is

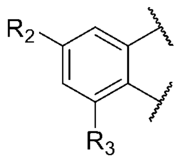

.

In some embodiments, the structural unit

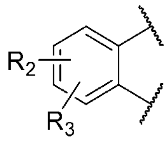

is

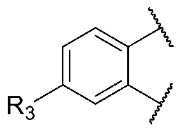

.

In some embodiments, the $R_3$ is selected from the group consisting of H, halogen, $-NO_2$, and $-NR_4R_5$, wherein $R_4$ and $R_5$ are as defined in any embodiment.

In some embodiments, the $R_3$ is selected from the group consisting of H, $-NO_2$, $-NH_2$, and $-NH(C_{1-4}$ alkyl).

In some embodiments, the $R_3$ is selected from the group consisting of H, $-NO_2$, $-NH_2$, and $-NH(CH_3)$.

In some embodiments, the $R_3$ is $-NR_4R_5$, wherein $R_4$ and $R_5$ are as defined in any embodiment.

In some embodiments, the $R_3$ is selected from the group consisting of $-NH_2$ and $-NH(CH_3)$.

In some embodiments, the $R_3$ is $-NO_2$.

In some embodiments, the $R_3$ is

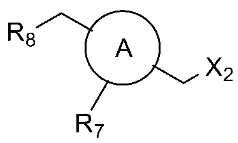

,

wherein $X_2$, ring A, $R_7$, and $R_8$ are as defined in any embodiment.

In some embodiments, the $R_8$ is selected from the group consisting of H, D, substituted or unsubstituted 5- to 9-membered heterocyclyl, and

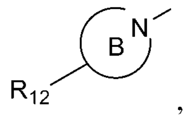

wherein ring B, and $R_{12}$ are as defined in any embodiment.

In some embodiments, the 5- to 9-membered heterocyclyl comprises 1, 2, or 3 N atoms.

In some embodiments, the 5- to 9-membered heterocyclyl is selected from the group consisting of morpholinyl and piperazinyl.

In some embodiments, the 5- to 9-membered heterocyclyl is piperazinyl.

In some embodiments, the 5- to 9-membered heterocyclyl is optionally substituted with one, two, or three groups selected from the group consisting of H, D, halogen, nitro, $C_{1-4}$ alkyl, $-C(=O)OC_{1-4}$ alkyl, and

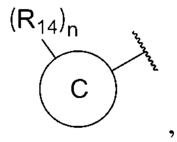

wherein ring C, $R_{14}$, and n are as defined in any embodiment.

In some embodiments, the $R_{12}$ is selected from the group consisting of H, D, halogen, nitro, $C_{1-4}$ alkyl, $-C(=O)OC_{1-4}$ alkyl, and

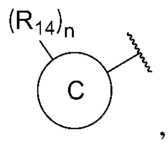

wherein ring C, $R_{14}$, and n are as defined in any embodiment.

In some embodiments, the compound is a compound represented by formula (II-1),

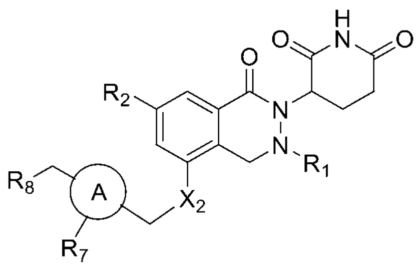

(II-1)

wherein $R_1$, $R_2$, $R_7$, $R_8$, $X_2$, and ring A are as defined in any embodiment.

In some embodiments, the compound is a compound represented by formula (III-1),

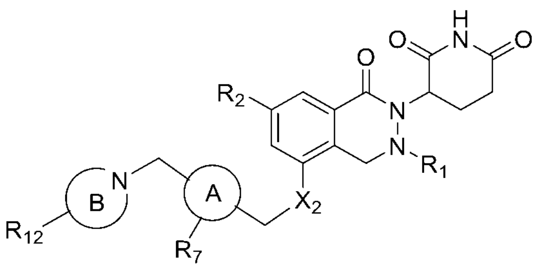

(III-1)

wherein $R_1$, $R_2$, $R_7$, $R_{12}$, $X_2$, ring A, and ring B are as defined in any embodiment.

In some embodiments, the compound is a compound represented by formula (IV-1),

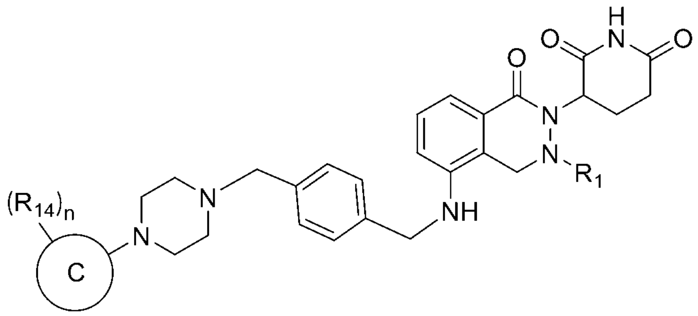

(IV-1)

wherein $R_1$, ring C, $R_{14}$, and n are as defined in any embodiment.

A second aspect of the present invention provides a pharmaceutical composition, which comprises the compound (e.g., the compound represented by formula (I)), or pharmaceutically acceptable salt, prodrug, stable isotope derivative, isomer, solvate, or polymorph thereof, and one or more pharmaceutically acceptable carriers or excipients. The pharmaceutically acceptable carriers or excipients include, but are not limited to: ion exchanger, alumina, aluminum stearate, lecithin, serum proteins such as human serum albumin, buffering substances such as phosphates, glycerol, sorbic acid, potassium sorbate, mixture of partial glycerides of saturated vegetable fatty acids, water, salts or electrolytes such as protamine sulfate, disodium hydrogen phosphate, potassium hydrogen phosphate, sodium chloride, zinc salts, colloidal silica, magnesium trisilicate, polyvinylpyrrolidone, cellulose substances, polyethylene glycol, sodium carboxymethyl cellulose, polyacrylate, beeswax, and lanolin.

A third aspect of the present invention provides a combination drug, which comprises the compound (e.g., the compound represented by formula (I)), or pharmaceutically acceptable salt, prodrug, stable isotope derivative, isomer, solvate, or polymorph thereof, and at least one additional drug, wherein the at least one additional drug is a chemotherapeutic agent or immunomodulator.

In some embodiments, the chemotherapeutic agent or immunomodulator is selected from the group consisting of immune checkpoint inhibitor, tyrosine kinase inhibitor, proteasome inhibitor, antibiotic, alkylating agent, antimetabolite, hormonal drug, immunomodulator, interferon-like active agent, and mixed active agent.

In some embodiments, the chemotherapeutic agent or immunomodulator is selected from the group consisting of dexamethasone, bortezomib, and tazemetostat.

In some embodiments, the chemotherapeutic agent or immunomodulator is tazemetostat.

A fourth aspect of the present invention provides a use of the compound (e.g., the compound represented by formula (I)), or pharmaceutically acceptable salt, prodrug, stable isotope derivative, isomer, solvate, or polymorph thereof, or the pharmaceutical composition thereof, in the manufacture of a medicament for the treatment and/or prevention of a disease associated with $CRL4^{CRBN}$ E3 ubiquitin ligase. The present invention also relates to the compound (e.g., the compound represented by formula (I)), or pharmaceutically acceptable salt, prodrug, stable isotope derivative, isomer, solvate, or polymorph thereof, or the pharmaceutical composition thereof, for use in the treatment and/or prevention of a disease associated with $CRL4^{CRBN}$ E3 ubiquitin ligase. The present invention further relates to a method of treating and/or preventing a disease associated with $CRL4^{CRBN}$ E3 ubiquitin ligase, comprising administering to an individual in need thereof an effective amount of the compound (e.g., the compound represented by formula (I)), or pharmaceutically acceptable salt, prodrug, stable isotope derivative, isomer, solvate, or polymorph thereof, or the pharmaceutical composition thereof.

In some embodiments, the disease includes but is not limited to cancer, pain, neurological disorder, and immune system disorder.

In some embodiments, the disease is hematologic malignancy.

In some embodiments, the disease is myeloma.

In some embodiments, the disease is multiple myeloma.

A fifth aspect of the present invention provides a use of the compound (e.g., the compound represented by formula (I)) or pharmaceutically acceptable salt, prodrug, stable isotope derivative, isomer, solvate, or polymorph thereof, or the pharmaceutical composition thereof, in the manufacture of a medicament for the treatment and/or prevention of a cancerous disease. The present invention also relates to the compound (e.g., the compound represented by formula (I)) or pharmaceutically acceptable salt, prodrug, stable isotope derivative, isomer, solvate, or polymorph thereof, or the

pharmaceutical composition thereof, for use in the treatment and/or prevention of a cancerous disease. The present invention further relates to a method of treating and/or preventing a cancerous disease, comprising administering to an individual in need thereof an effective amount of the compound (e.g., the compound represented by formula (I)), or pharmaceutically acceptable salt, prodrug, stable isotope derivative, isomer, solvate, or polymorph thereof, or the pharmaceutical composition thereof.

In some embodiments, the cancerous disease includes, but is not limited to: one or more of various types of leukemia, multiple myeloma, various malignant lymphomas such as non-Hodgkin's lymphoma, diffuse large B-cell lymphoma, follicular lymphoma, mantle cell lymphoma, marginal zone lymphoma, Waldenström macroglobulinemia, etc., erythema nodosum, autoimmune diseases such as systemic lupus erythematosus, myelodysplastic syndrome, breast cancer, gastrointestinal cancer, various types of lung cancer, liver cancer, pancreatic cancer, skin cancer, head and neck cancer, melanoma, uterine cancer, ovarian cancer, various endocrine gland cancers, kidney or ureter cancer, and CNS tumors. In some embodiments, the lung cancer is non-small cell lung cancer. In some embodiments, the endocrine gland disease is selected from the group consisting of breast cancer, thyroid cancer, and gastric cancer.

In some embodiments, the cancerous disease is hematologic malignancy.

In some embodiments, the cancerous disease is myeloma.

In some embodiments, the cancerous disease is multiple myeloma.

The sixth aspect of the present invention provides the compound, or pharmaceutically acceptable salt, prodrug, stable isotope derivative, isomer, solvate, or polymorph thereof, or the pharmaceutical composition or the combination drug as described herein, which can be administered via a suitable route. In some embodiments, the suitable route includes oral, sublingual, rectal, parenteral, (intradermal, subcutaneous, intramuscular, intravenous, arterial) injection, pulmonary, nasal, tongue, buccal, skin, mucous membrane, conjunctival, local administration, or administration via implantation.

The seventh aspect of the present invention provides a use of the compound (e.g., the compound represented by formula (I)), or pharmaceutically acceptable salt, prodrug, stable isotope derivative, isomer, solvate, or polymorph thereof in the manufacture of a proteolysis targeting chimera (PROTAC).

In some embodiments, the compound, or pharmaceutically acceptable salt, prodrug, stable isotope derivative, isomer, solvate, or polymorph thereof is used as a ligand for $CRL4^{CRBN}$ E3 ubiquitin ligase in the manufacture of a proteolysis targeting chimera (PROTAC).

The eighth aspect of the present invention provides a synthetic method for preparing the compound (e.g., the compound represented by formula (I)), in which the compound can be synthesized from commercially available starting materials using known methods. In specific practice, the steps in the method may be expanded or combined as needed, and exemplary methods for preparing these compounds may include (but are not limited to) the processes described below.

In some embodiments, the compound of the present invention (e.g., the compound represented by formula (I)) is synthesized according to the route of Scheme A: reacting the compound represented by formula $A_1$ with benzaldehyde and methylhydrazine to prepare $A_2$; cyclizing $A_2$ under the catalysis of a Lewis acid to form $A_3$; substituting $A_3$ with 3-bromocycloglutarimide to generate the target product $A_4$,

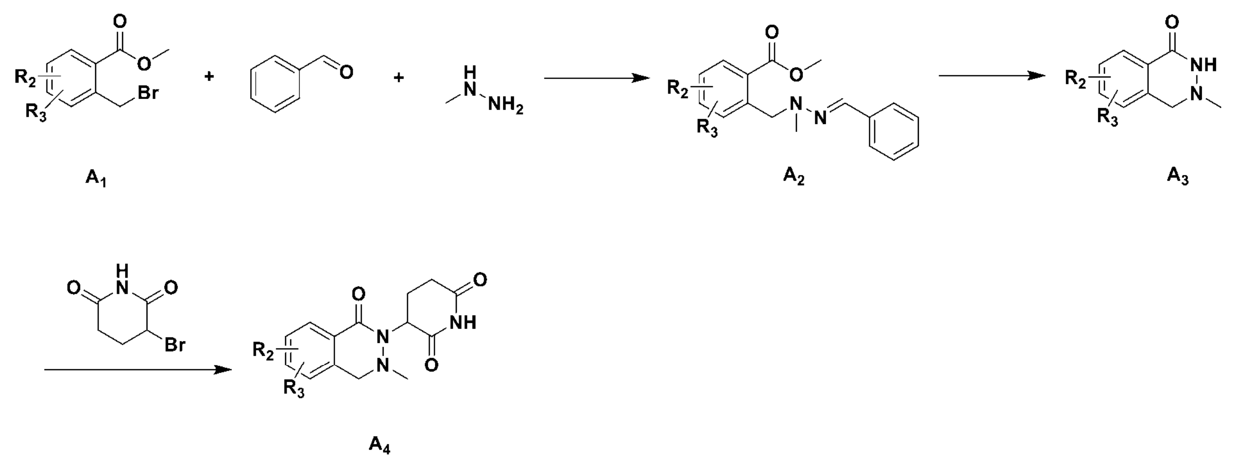

$A_1$ $A_2$ $A_3$

$A_4$

wherein: $R_2$ and $R_3$ are as defined in the present invention.

In some embodiments, the Lewis acid is $ZnCl_2$.

In some embodiments, the synthetic route of Scheme A is as follows:

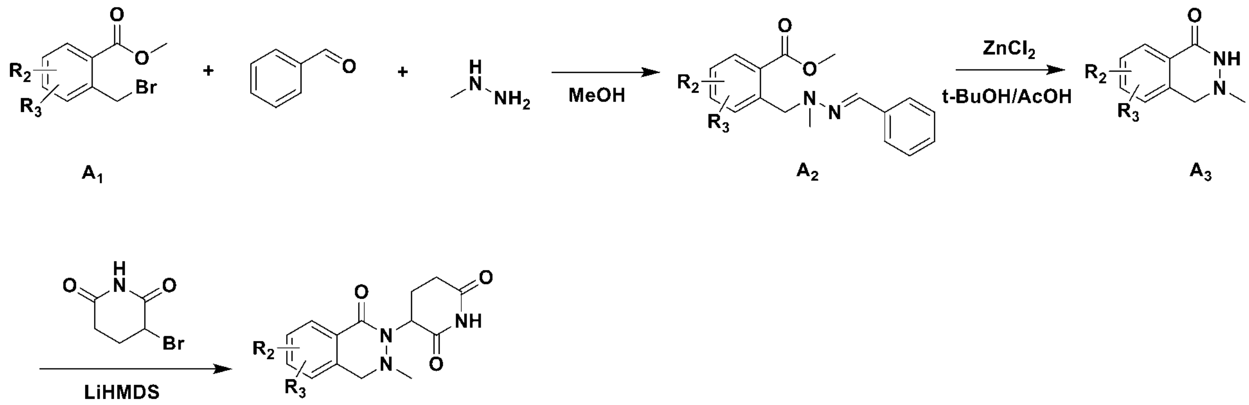


A1 A2 A3

A4

In some embodiments, the compound of the present invention (e.g., the compound represented by formula (I)) is synthesized according to the route of Scheme B: firstly, using 2-formylbenzoic acid $B_1$ as starting material and performing a condensation reaction with hydrazine hydrate under heating conditions to obtain phthalazinone $B_2$; subjecting $B_2$ to a substitution reaction with dimethyl 2-bromoglutarate ($B_{10}$) to yield $B_3$, followed by performing a cyclization in the presence of sodium amide as base catalyst to give $B_4$; wherein the key intermediate $B_{10}$ is derived from glutaric acid $B_7$ as raw material via a one-pot process by successively carrying out thionyl chloride acylation, liquid bromine substitution, and methanol esterification to yield $B_8$, $B_9$, and $B_{10}$; in some embodiments, reducing $B_4$ to give $B_5$; in some embodiments, performing the reduction via zinc; in some embodiments, methylating $B_5$ to give $B_6$; in some embodiments, carrying out the methylation in the presence of formaldehyde and formic acid.

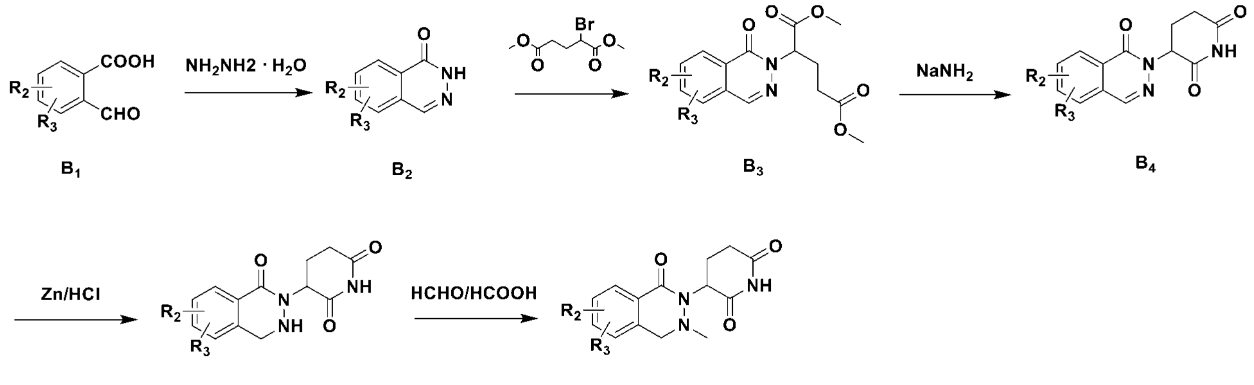


B1 B2 B3 B4

B5 B6

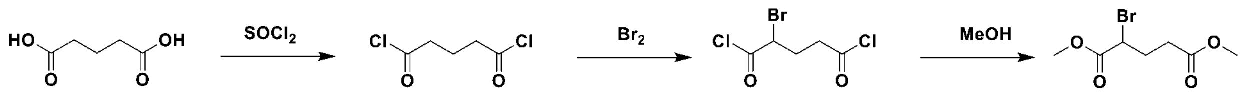


B7 B8 B9 B10

In some embodiments, the compound of the present invention (e.g., the compound represented by formula (I)) is synthesized according to the route of Scheme C: synthesizing compound $A_4$ according to Scheme A; substituting the unsaturated nitrogen-containing non-aromatic compound R with methyl 4-bromomethylbenzoate to give $C_5$; firstly fully reducing the methyl benzoate moiety of $C_5$ to generate an alcohol (compound $C_6$), and then partially oxidizing $C_6$ to generate an aldehyde $C_7$; finally, conducting a reductive amination reaction between $C_7$ and the aromatic amino group of the aforementioned compound $A_4$ to form an imine therebetween, thereby obtaining the target product,

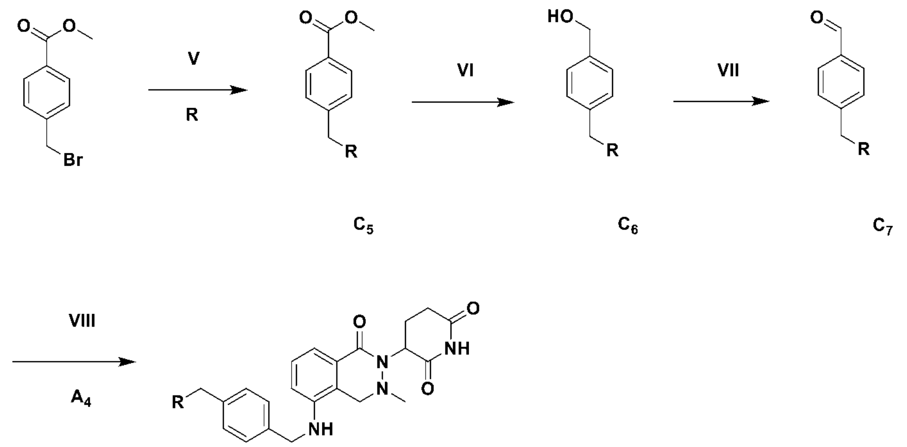


$C_5$ $C_6$ $C_7$

wherein, R is the ring B as defined in formula (III).

In some embodiments, the compound of the present invention (e.g., the compound represented by formula (I)) is synthesized according to the route of Scheme D: under the conditions of $Pd_2(dba)_3$ as a catalyst, X-Phos as a ligand, and NaOtBu as a base, carrying out a Buchwald-Hartwig carbon-nitrogen coupling reaction between 1-tert-butyloxycarbonylpiperazine and a halogenated aromatic compound R to generate compound $D_5$; deprotecting the Boc protecting group on N of compound $D_5$ to obtain $D_6$; the subsequent synthetic steps are consistent with the synthetic route of Scheme C: successively performing methyl 4-bromomethylbenzoate substitution, DIBAL reduction, and manganese dioxide oxidation to obtain compounds $D_7$, $D_8$, and $D_9$, respectively; and conducting a reductive amination reaction between compounds $D_9$ and $A_4$ to obtain the target compound.

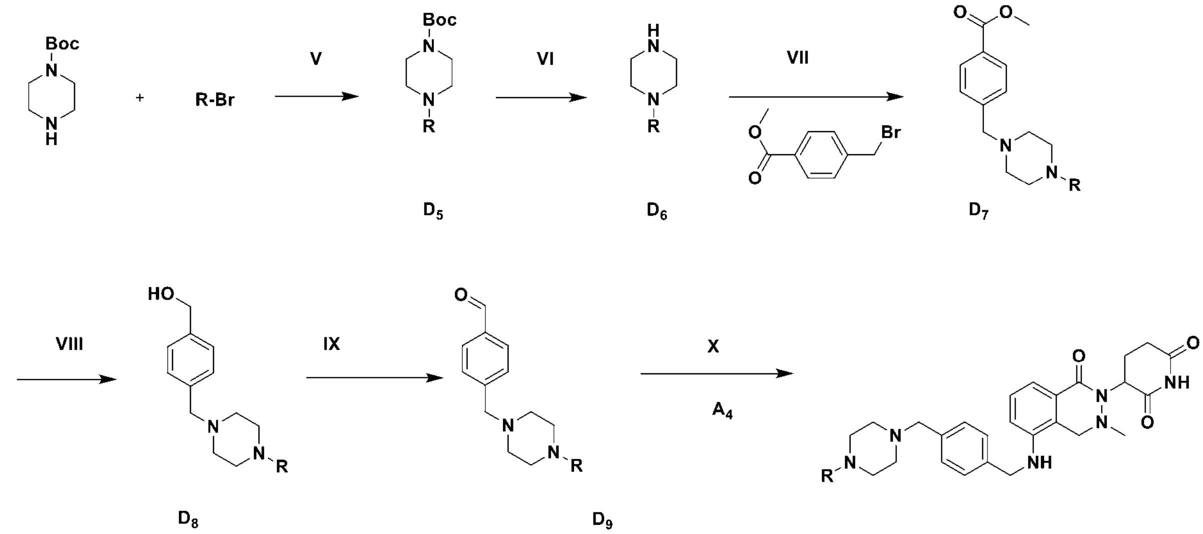


$D_5$ $D_6$ $D_7$ $D_8$ $D_9$

For the compound represented by formula (I), as well as the method for preparing the compound, the pharmaceutical composition, and the treatment regimen as disclosed in the present invention, those skilled in the art can refer to the contents of the present application to appropriately improve the process parameters. It is particularly important to note that all similar substitutions and modifications will be obvious to those skilled in the art and are considered to be included in the present invention. The products, methods, and uses of the present invention have been described through preferred examples, and those skilled in the art will readily be able to modify or appropriately alter and combine the methods and uses described herein without departing from the content, spirit, and scope of the present invention to realize and apply the technology of the present invention.

In some embodiments, the present invention provides the compound represented by formula (I) and its pharmaceutically acceptable salt, prodrug, stable isotope derivative, isomer, solvate, polymorph, and pharmaceutical composition, and the compound is selected from, but not limited to:

| Example No. | Compound Code | Chemical Structure | Compound Name |
|---|---|---|---|
| 1 | **I-11** | 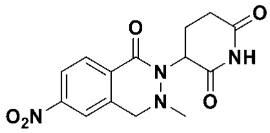 | 3-(3-Methyl-6-nitro-1-oxo-3,4 -dihydrophthalazin-2(1*H*)-yl)p iperidin-2,6-dione |
| 3 | **I-9** | 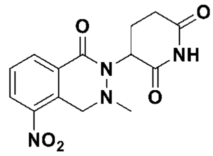 | 3-(3-Methyl-5-nitro-1-oxo-3,4-dihydrophthalazin-2(1*H*)-yl)pip eridin-2,6-dione |
| 2 | **I-10** | 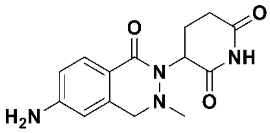 | 3-(6-Amino-3-methyl-1-oxo-3 ,4-dihydrophthalazin-2(1*H*)-yl )piperidin-2,6-dione |
| 4 | **I-4** | 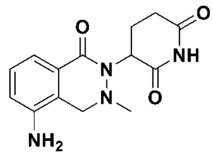 | 3-(5-Amino-3-methyl-1-oxo-3 ,4-dihydrophthalazin-2(1*H*)-yl )piperidin-2,6-dione |
| 5 | **I-2** | 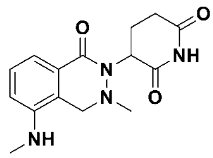 | 3-(3-Methyl-5-(methylamino) -1-oxo-3,4-dihydrophthalazin-2(1*H*)-yl) piperidin-2,6-dione |
| 6 | **YJ-1a** | 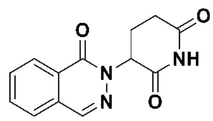 | 3-(1-Oxophthalazin-2(1*H*)-yl) piperidin-2,6-dione |
| 7 | **YJ-2a** | 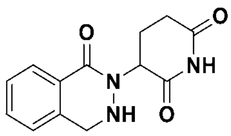 | 3-(1-Oxo-3,4-dihydrophthalazin -2(1*H*)-yl)piperidin-2,6-dione |
| 8 | **YJ-3a** | 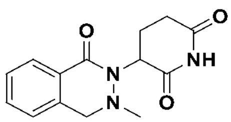 | 3-(3-Methyl-1-oxo-3,4-dihydr ophthalazin-2(1*H*)-yl)piperidi n-2,6-dione |
| 9 | **YJ-1b** | 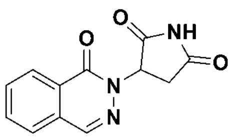 | 3-(1-Oxophthalazin-2(1*H*)-yl) pyrrolidin-2,5-dione |
| 10 | **YJ-2b** | 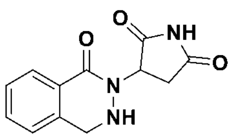 | 3-(1-Oxo-3,4-dihydrophthalaz in-2(1*H*)-yl)pyrrolidin-2,5-dio ne |
| 11 | **I-13** | 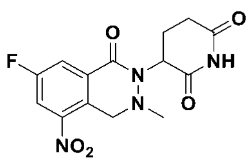 | 3-(7-Fluoro-3-methyl-5-nitro-1-oxo-3,4-dihydrophthalazin-2(1*H*)-yl) piperidin-2,6-dione |

(continued)

| Example No. | Compound Code | Chemical Structure | Compound Name |
|---|---|---|---|
| 12 | **I-12** | 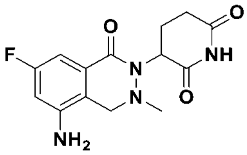 | 3-(5-Amino-7-fluoro-3-methy 1-1-oxo-3,4-dihydrophthalazin -2(1*H*)-yl) piperidin-2,6-dione |
| 13 | **I-5** | 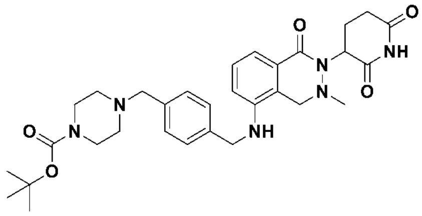 | Tert-butyl 4-(4-(((2-(2,6-dioxopiperidin-3-yl)-3-methyl-1-oxo-1,2,3,4-t etrahydrophthalazin-5-yl)amin o) methyl)benzyl)piperidin-1-c arboxy-late |
| 14 | **I-1** | 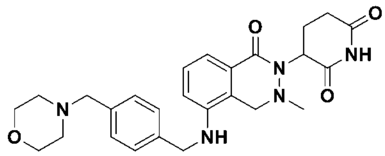 | 3-(3-Methyl-5-((4-(morpholin omethyl)benzyl)amino)-1-oxo -3,4-dihydrophthalazin-2(1*H*)-yl)-piperidin-2,6-dione |
| 15 | **I-3** | 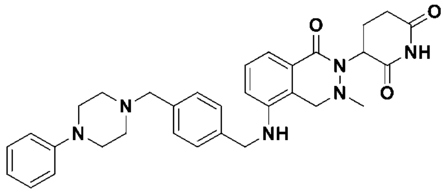 | 3-(3-Methyl-1-oxo-5-((4-((4-p henylpiperidin-1-yl)methyl)be nzyl)amino)-3,4-dihydrophtha lazin-2(1*H*)-yl) piperidin-2,6-d ione |
| 16 | **I-6** | 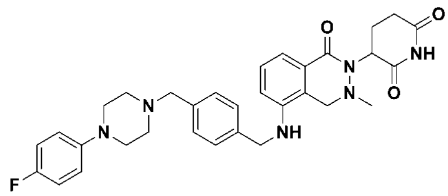 | 3-(5-((4-(4-(4-Fluorophenyl)p iperazin-1-yl)methyl)benzyl)a mino)-3-methyl-1-oxo-3,4-dih ydrophthalazin-2(1*H*)-yl)piper idin-2,6-dione |
| 17 | **I-7** | 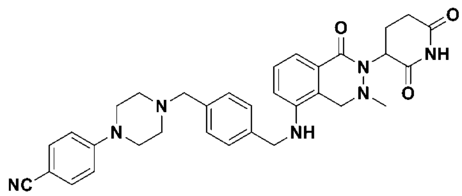 | 3-(5-((4-(4-(4-Cyanophenyl)pi perazin-1-yl)methyl)benzyl)a mino)-3-methyl-1-oxo-3,4-dih ydrophthalazin-2(1*H*)-yl)piper idin-2,6-dione |
| 18 | **I-8** | 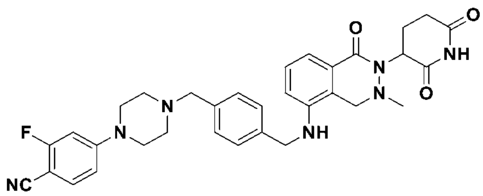 | 3-(5-((4-(4-(3-Fluoro-4-cyano phenyl) piperazin-1-yl)methyl) benzyl)amino)-3-methyl-1-ox o-3,4-dihydrophthalazin-2(1*H* )-yl)piperidin-2,6-dione |

In the present invention, unless the context otherwise requires, the words, phrases, and symbols used below have the following meanings. When the compound names used in the present invention differ from the chemical structural formulas, the chemical structural formulas shall prevail. The following abbreviations and terms have the following meanings throughout the text:

The term "alkyl" refers to a hydrocarbon group selected from the group consisting of saturated linear and branched hydrocarbonyl groups. Examples of alkyl groups include methyl, ethyl, 1-propyl or n-propyl ("n-Pr"), 2-propyl or isopropyl ("i-Pr"), 1-butyl or n-butyl ("n-Bu"), 2-methyl-1-propyl or isobutyl ("i-Bu"), 1-methylpropyl or sec-butyl ("s-Bu"), and 1,1-dimethylethyl or tert-butyl ("t-Bu"). Examples of other alkyl groups include 1-pentyl, 2-pentyl, 3-pentyl, 2-methyl-2-butyl, 3-methyl-2-butyl, 3-methyl-1-butyl, 2-methyl-1-butyl, 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, and 3,3-dimethyl-2-butyl.

Lower alkyl refers to an alkyl having 1-8, preferably 1-6, more preferably 1-4 carbon atoms; lower alkenyl or alkynyl refers to an alkenyl or alkynyl having 2-8, 2-6, or 2-4 carbon atoms.

The term "alkenyl" refers to a hydrocarbon group selected from the group consisting of linear and branched hydrocarbonyl groups, comprising at least one C=C double bond and 2-18 carbon atoms. Examples of alkenyl groups can be selected from the group consisting of vinyl (ethenyl or vinyl), prop-1-enyl, prop-2-enyl, 2-methylprop-1-enyl, but-1-enyl, but-2-enyl, but-3-enyl, but-1,3-dienyl, 2-methylbut-1,3-dienyl, hex-1-enyl, hex-2-enyl, hex-3-enyl, hex-4-enyl, and hex-1,3-dienyl.

The term "alkynyl" refers to a hydrocarbon group selected from the group consisting of linear and branched hydrocarbonyl groups, comprising at least one C≡C triple bond and 2-18 carbon atoms. Examples of alkynyl groups include ethynyl, prop-1-ynyl, prop-2-ynyl (propargyl), but-1-ynyl, buty-2-nyl, and but-3-ynyl.

The term "cycloalkyl" refers to a hydrocarbon group selected from the group consisting of saturated and partially unsaturated cycloalkyl groups, including monocyclic and polycyclic (e.g., bicyclic and tricyclic) groups, which can have 3-12 carbon atoms. For example, a cycloalkyl can be a monocyclic group having 3-12 carbon atoms. Examples of monocyclic cycloalkyl groups include cyclopropyl, cyclobutyl, cyclopentyl, cyclopent-1-en-1-yl, cyclopent-2-en-1-yl, cyclopent-3-en-1-yl, cyclohexyl, cyclohex-1-en-1-yl, cyclohex-2-en-1-yl, cyclohex-3-en-1-yl, cyclohexadienyl, cycloheptyl, cyclooctyl, cyclononyl, cyclodecyl, cycloundecyl, and cyclododecyl. Examples of bicyclic cycloalkyl groups include bicyclic structures consisting of 7-12 ring atoms arranged in a ring system selected from the group consisting of [4,4], [4,5], [5,5], [5,6], and [6,6] ring systems, or bridged bicyclic structures selected from the group consisting of bicyclic [2.2.1] heptane, bicyclic [2.2.2]octane, and bicyclic [3.2.2]nonane. The rings can be saturated or have at least one double bond (i.e., partially unsaturated), but are not fully conjugated and are not aromatic (as defined herein).

The term "aryl" as used herein refers to a group selected from following: 5- and 6-membered carbocyclic aromatic rings, such as phenyl; bicyclic systems such as 7-12-membered bicyclic systems, wherein at least one ring is a carbocyclic and aromatic ring, such as naphthalene, 1,2-dihydroindene, and 1,2,3,4-tetrahydroquinoline; and tricyclic systems such as 10- and 15-membered tricyclic systems, wherein at least one ring is a carbocyclic and aromatic ring, such as fluorene.

For example, the aryl is selected from the group consisting of 5- and 6-membered carbocyclic aromatic rings, in which the carbocyclic aromatic rings are each fused to a 5- or 7-membered cycloalkyl or heterocyclic ring optionally containing at least one heteroatom selected from the group consisting of N, O, and S, provided that when the carbocyclic aromatic ring is fused to a heterocyclic ring, the connecting site is on the carbocyclic aromatic ring; and when the carbocyclic aromatic ring is fused to a cycloalkyl, the connecting site may be on the carbocyclic aromatic ring or on the cycloalkyl. A divalent group formed from a substituted benzene derivative and having a free valence on a ring atom is called a substituted phenylene group. A divalent group derived from a monovalent polycyclic hydrocarbon whose name ends in "-yl" by removing one hydrogen atom from a carbon atom having a free valence is named by adding "-ene" to the name of the corresponding monovalent group; for example, a naphthyl group with two connecting sites is called a naphthylene group. However, aryl groups do not include heteroaryl groups or groups overlapping with heteroaryl groups, which are defined separately below. Therefore, if one or more carbocyclic aromatic rings are fused with a heterocyclic aromatic ring, the resulting ring system is a heteroaryl as defined herein, not an aryl.

The term "halogen" refers to F, C1, Br, or I.

The term "heteroalkyl" refers to an alkyl containing at least one heteroatom.

The term "heteroaryl" refers to a group selected from the following:

5- to 7-membered aromatic monocyclic rings containing 1, 2, 3, or 4 heteroatoms selected from the group consisting of N, O, and S, with the remaining ring atoms being carbon;

8- to 12-membered bicyclic rings containing 1, 2, 3, or 4 heteroatoms selected from the group consisting of N, O, and S, with the remaining ring atoms being carbon, wherein at least one ring is an aromatic ring, and at least one heteroatom is present in the aromatic ring;

11- to 14-membered tricyclic rings containing 1, 2, 3, or 4 heteroatoms selected from the group consisting of N, O, and S, with the remaining ring atoms being carbon, wherein at least one ring is an aromatic ring, and at least one heteroatom is present in the aromatic ring.

For example, the heteroaryl comprises a 5- to 7-membered heterocyclic aromatic ring fused to a 5- to 7-membered cycloalkyl ring. In this fused bicyclic heteroaryl ring system, only one ring contains at least one heteroatom, and the connection site can be on the heteroaromatic ring or the cycloalkyl ring.

When the total number of S and O atoms in a heteroaryl exceeds 1, these heteroatoms are not adjacent to each other. In some embodiments, the total number of S and O atoms in the heteroaryl does not exceed 2. In some

embodiments, the total number of S and O atoms in the aromatic heterocyclyl does not exceed 1.

Examples of heteroaryl groups include, but are not limited to (numbered from the preferred linking position 1), pyridyl (e.g., 2-pyridinyl, 3-pyridinyl, or 4-pyridinyl), cinnolinyl, pyrazinyl, 2,4-pyrimidinyl, 3,5-pyrimidinyl, 2,4-imidazolyl, imidazopyridyl, isoxazolyl, oxazolyl, thiazolyl, isothiazolyl, thiadiazolyl, tetrazolyl, thiophenyl, triazinyl, benzothiophenyl, furanyl, benzofuranyl, benzimidazolyl, indolyl, isoindolyl, dihydroindolyl, phthalazinyl, pyrazinyl, pyridazinyl, pyrrolyl, triazolyl, quinolinyl, isoquinolinyl, pyrazolyl, pyrrolopyridyl (e.g., 1*H*-pyrrolo[2,3-*b*]pyridin-5-yl), pyrazolopyridyl (e.g., 1*H*-pyrazolo[3,4-*b*]pyridin-5-yl), benzoxazolyl (e.g., benzo[*d*]oxazol-6-yl), pteridinyl, purinyl, 1-oxa-2,3-diazolyl, 1-oxa-2,4-diazolyl, 1-oxa-2,5-diazolyl, 1-oxa-3,4-diazolyl, 1-thia-2,3-diazolyl, 1-thia-2,4-diazolyl, 1-thia-2,5-diazolyl, 1-thia-3,4-diazolyl, furazanyl, benzofurazanyl, benzothiophenyl, benzothiazolyl, benzooxazolyl, quinazolinyl, quinoxalinyl, naphthyridinyl, furopyridyl, benzothiazolyl (e.g., benzo[*d*]thiazolyl-6-yl), indazolyl (e.g., 1*H*-indazol-5-yl), and 5,6,7,8-tetrahydroisoquinolinyl.

The terms "heterocyclic," "heterocyclic ring," or "heterocyclyl" refer to a ring selected from the group consisting of 4- to 12-membered monocyclic, bicyclic, and tricyclic saturated or partially unsaturated rings containing at least one carbon atom in addition to 1, 2, 3, or 4 heteroatoms selected from the group consisting of oxygen, sulfur, and nitrogen. "Heterocyclic ring" also refers to a 5- to 7-membered heterocyclic ring containing at least one heteroatom selected from the group consisting of N, O, and S and fused with a 5, 6, and/or 7-membered cycloalkyl, carbocyclic aromatic, or heteroaromatic ring, provided that when the heterocyclic ring is fused with a carbocyclic aromatic or heteroaromatic ring, the connecting site is on the heterocyclic ring, and when the heterocyclic is fused with a cycloalkyl, the connecting site is on the cycloalkyl or heterocyclic ring.

"Heterocyclic ring" also refers to an aliphatic spirocycle containing at least one heteroatom selected from the group consisting of N, O, and S, provided that the connecting site is on the heterocyclic ring. The ring may be saturated or contain at least one double bond (i.e., partially unsaturated). The heterocyclic ring may be substituted by oxo. The connecting site may be a carbon atom or heteroatom in the heterocyclic ring. The heterocyclic ring is not a heteroaryl as defined herein.

Examples of heterocyclic rings include, but are not limited to (numbered from the preferred connection position 1), 1-pyrrolidinyl, 2-pyrrolidinyl, 2,4-imidazolidinyl, 2,3-pyrazolidinyl, 1-piperidinyl, 2-piperidinyl, 3-piperidinyl, 4-piperidinyl, 2,5-piperazinyl, pyranyl, 2-morpholinyl, 3-morpholinyl, oxiranyl, aziridinyl, thiiranyl, azetidinyl, oxetanyl, thietanyl, 1,2-dithietanyl, 1,3-dithietanyl, dihydropyridinyl, tetrahydropyridinyl, thiomorpholinyl, thioxanyl, piperazinyl, homopiperazinyl, homopiperidinyl, azepanyl, oxepanyl, thiepanyl, 1,4-oxathianyl, 1,4-dioxepanyl, 1,4-oxothiepanyl, 1,4-oxazepanyl, 1,4-dithiepanyl, 1,4-thiazepanyl, 1,4-diazepanyl, 1,4-dithianyl, 1,4-thiazinyl, oxazepinyl, diazepinyl, thiazepinyl, dihydrothienyl, dihydropyranyl, dihydrofuranyl, tetrahydrofuranyl, tetrahydrothienyl, tetrahydropyranyl, tetrahydrothiopyranyl, 1-pyrrolinyl, 2-pyrrolinyl, 3-pyrrolinyl, indolinyl, 2*H*-pyranyl, 4*H*-pyranyl, 1,4-dioxanyl, 1,3-dioxolanyl, pyrazolinyl, pyrazolidinyl, dithianyl, dithiolanyl, pyrazolidinylimidazolinyl, pyrimidinonyl, 1,1-dioxo-thiomorpholinyl, 3-azabicyclo[3.1.0]hexanyl, 3-azabicyclo[4.1.0]heptanyl, azabicyclo[2.2.2]hexanyl. Substituted heterocyclic rings also include ring systems substituted with one or more oxo moieties, such as piperidinyl N-oxide, morpholinyl-N-oxide, 1-oxo-1-thiomorpholinyl, and 1,1-dioxo-1-thiomorpholinyl.

The term "fused ring" as used herein refers to a polycyclic system, such as a bicyclic or tricyclic system, in which the two rings share only two ring atoms and one bond. Examples of fused rings can include fused bicyclic alkyl rings, such as bicyclic rings composed of 7-12 ring atoms selected from the group consisting of the above-mentioned [4,4], [4,5], [5,5], [5,6], and [6,6] bicyclic ring systems; fused bicyclic aryl rings, for example, the above-mentioned 7- to 12-membered bicyclic aryl ring systems, fused tricyclic aryl rings, such as the above-described 10- to 15-membered tricyclic aryl ring systems; fused bicyclic heteroaryl rings, such as the above-mentioned 8- to 12-membered bicyclic heteroaryl rings; fused tricyclic heteroaryl rings, such as the above-mentioned 11- to 14-membered tricyclic heteroaryl rings; and the above-mentioned fused bicyclic or tricyclic heterocyclic rings.

The compounds may contain one asymmetric center and thus may exist as enantiomers. When the compounds have two or more asymmetric centers, they may also exist as diastereomers. Enantiomers and diastereomers fall into the broader category of stereoisomers. All these possible stereoisomers include substantially pure resolved enantiomers, their racemic mixtures, and mixtures of diastereomers, including all stereoisomers of the compounds and/or their pharmaceutically acceptable salts. Unless otherwise stated, the mention of one isomer applies to any possible isomers. When isomer components are not specifically specified, all possible isomers are included.

When a compound contains alkene double bonds, unless otherwise stated, these double bonds refer to both E and Z geometric isomers.

Some compounds can exist in tautomer forms. For example, compounds containing a carbonyl $-CH_2C(O)-$ group (keto form) can undergo tautomerism to form a hydroxyl $-CH=C(OH)-$ group (enol form). In application of the present invention, both keto and enol forms, as well as mixtures thereof, are also included.

The compounds represented by formulas (I), (I-1), (II), (II-1), (III), (III-1), (IV), (IV-1) of the present invention can also be in the form of their salts, generally salts formed with organic or inorganic bases or acids. Physiologically acceptable salts are preferred in the present invention. Physiologically acceptable salts of the compounds of the present invention can

be salts of the substances of the present invention with inorganic acids, carboxylic acids, or sulfonic acids, particularly preferred being salts formed, for example, with hydrochloric acid, hydrobromic acid, sulfuric acid, phosphoric acid, nitric acid, perchloric acid, fumaric acid, acetic acid, propionic acid, succinic acid, glycolic acid, formic acid, lactic acid, maleic acid, tartaric acid, citric acid, pamoic acid, malonic acid, hydroxymaleic acid, phenylacetic acid, glutamic acid, benzoic acid, salicylic acid, fumaric acid, p-toluenesulfonic acid, methanesulfonic acid, ethanesulfonic acid, naphthalene-2-sulfonic acid, benzenesulfonic acid, hydroxynaphthoic acid, hydroiodic acid, malic acid, and tannic acid. Other acids, such as oxalic acid, although not pharmaceutically acceptable on their own, can be used to prepare salts as intermediates to obtain the compounds of the present invention and their pharmaceutically acceptable salts.

Physiologically acceptable salts can also be metal or ammonium salts of the compounds of the present invention having a free carboxyl group. Particularly preferred are sodium, potassium, magnesium or calcium salts, as well as ammonium salts of inorganic ammonia or organic amines such as ethylamine, diethylamine, triethylamine, N,N'-dibenzylethylenediamine, chloroprocaine, choline, N-methylglucosamine, procaine, diethanolamine, triethanolamine, dicyclohexylamine, dimethylaminoethanol, arginine, lysine, or ethylenediamine.

The compounds of the present invention can exist in tautomeric forms, and the present invention also includes such forms.

The compounds of the present invention can also be their possible solvates.

The present invention also relates to a medicament comprising at least one compound of the present invention, preferably also comprising one or more pharmacologically acceptable excipients or carriers, and further relates to its use for the aforementioned purposes. The pharmaceutical carriers used here include, but are not limited to: ion exchanger, alumina, aluminum stearate, lecithin, serum proteins such as human serum albumin, buffering substances such as phosphates, glycerol, sorbic acid, potassium sorbate, mixture of partial glycerides of saturated vegetable fatty acids, water, salts or electrolytes such as protamine sulfate, disodium hydrogen phosphate, potassium hydrogen phosphate, sodium chloride, zinc salts, colloidal silica, magnesium trisilicate, polyvinylpyrrolidone, cellulose substances, polyethylene glycol, sodium carboxymethyl cellulose, polyacrylate, beeswax, and lanolin. The active ingredient can have systemic and/or local effects, and therefore can be administered via suitable routes, such as oral, parenteral, pulmonary, nasal, sublingual, lingual, buccal, rectal, transdermal, conjunctival, local administration, or implantation.

The active ingredient can also be administered in a form suitable for these routes of administration:
Known administration forms suitable for oral administration that can rapidly and/or in a modified manner deliver the active ingredient, such as tablets (uncoated or coated tablets, such as tablets with enteric or film coating), capsules, sugar-coated tablets, granules, pills, powders, emulsions, suspensions, and aerosols.

Parenteral administration may avoid absorption steps (intravenous, intraarterial, intracardiac, intraspinal, or intralumbar administration) or involve absorption steps (intramuscular, subcutaneous, intradermal, transdermal, or intraperitoneal administration). Forms suitable for parenteral administration are particularly preparations in the form of solutions, suspensions, emulsions, lyophilized products, and sterile powders intended for injection and infusion.

Forms suitable for alternative routes of administration include, for example, inhaled (especially powder inhalation, spray) medications, nasal drops/solutions, sprays; tablets, capsules, or suppositories for tongue, sublingual or buccal administration; preparations for ear and eye administration; vaginal capsules; aqueous suspensions (lotions, shaken mixtures); lipophilic suspensions; ointments, creams, lotions, pastes, powders, or implants, such as stent fixation molds.

The active ingredient can be converted into the described administration form using methods known per se. This can be achieved using inert, non-toxic, suitable pharmaceutical excipients. These particularly include carriers (e.g., microcrystalline cellulose), solvents (e.g., liquid polyethylene glycol), emulsifiers (e.g., sodium lauryl sulfate), dispersants (e.g., polyvinylpyrrolidone), synthetic and natural biopolymers (e.g., proteins), stabilizers (e.g., antioxidants and ascorbic acid), colorants (e.g., inorganic pigments such as iron oxide), or flavoring agents and/or flavor masking agents. Where suitable, the active ingredient may be present in one or more of the above carriers in the form of microencapsulated capsules.

The present application covers all possible crystalline forms or polymorphs of the compounds, which may be a single polymorph or a mixture of more than one polymorph in any proportion.

The compounds of the present application can exist as solvates (e.g., hydrates), wherein the compounds of the present application contain a solvent, such as water, methanol, or ethanol, as a structural element of the lattice of the compounds. The amount of the solvent may be stoichiometric or non-stoichiometric.

The term "prodrug" refers to a derivative of the compounds of the present invention that can be hydrolyzed, oxidized, or otherwise reacted under biological conditions (in vitro or in vivo) to provide the compounds of the present invention. Prodrugs become active compounds only after undergoing this reaction under biological conditions, or they do not have or only have low activity in their unreacted forms. Prodrugs can generally be prepared using known methods, such as those described in Burger's Medicinal Chemistry and Drug Discovery (1995) 172-178, 949-982 (Manfred E. Wolff, ed., 5th edition).

The term "isotope derivative" refers to a compound in which one or more atoms are replaced by atoms of the same atomic number but with a different atomic mass or mass number than the dominant atomic mass or mass number

found in nature. Examples of isotopes of the compounds of the present invention suitable for inclusion in include, but are not limited to, hydrogen isotopes such as $^{2}H$ and $^{3}H$; carbon isotopes such as $^{11}C$, $^{13}C$, and $^{14}C$; chlorine isotopes such as $^{36}Cl$; fluorine isotopes such as $^{18}F$; iodine isotopes such as $^{123}I$ and $^{125}I$; nitrogen isotopes such as $^{13}N$ and $^{15}N$; oxygen isotopes such as $^{15}O$, $^{17}O$, and $^{18}O$; and sulfur isotopes such as $^{35}S$.

The term "effective amount" refers to a dose that can achieve treatment and/or prevention of the disease or symptom described in the present invention in a subject.

The term "treatment" aims to alleviate, reduce, improve, or eliminate the targeted disease state or symptom. For example, a subject is successfully "treated" if, after administration of the relevant drug, one or more indicators and symptoms show an observable and/or detectable reduction or improvement. It should also be understood that the treatment of the aforementioned disease state or symptom includes not only complete treatment but also the achievement of some biological or medically relevant results without complete treatment.

The term "prevention" aims to avoid, reduce, prevent, or delay the onset of a disease or disease-related symptom, in which the disease or disease-related symptom does not appear before the administration of the relevant drug. "Prevention" does not necessarily require the complete prevention of the onset of the disease or disease-related symptom; for example, reducing the risk of a subject developing a specific disease or disease-related symptom after administration of the relevant drug, or lessening the severity of subsequently occurring related symptom, can be considered as "prevention" of the onset or development of the disease.

Beneficial effects:

The compounds of the present invention exhibit excellent inhibitory effects on the proliferation of hematological tumor cells and can effectively inhibit the proliferation of drug-resistant cells, and possess excellent in vivo efficacy.

## Brief Description of the Drawings

Figure 1 shows the inhibitory effect of compounds **I-4** and **I-8** on human multiple myeloma RPMI-8226 xenograft tumors.

Figure 2 shows the inhibitory effect of compounds **I-4** and **I-8** on human multiple myeloma NCI-H929 xenograft tumors.

Figure 3 shows the inhibitory effects of compounds **I-4** and **I-8** in combination with dexamethasone on human multiple myeloma NCI-H929 xenograft tumors.

Figure 4 shows the inhibitory effects of compound **I-8** in combination with bortezomib and tazemetostat, respectively, on human multiple myeloma NCI-H929 xenograft tumors.

Note: ** in the above figures indicates $p < 0.01$, and ns indicates no significant difference.

## Specific Models for Carrying Out the Invention

More detailed information on the preparation of the compounds of general formula (I) is provided in the following examples, but these examples are merely illustrative of preferred embodiments of the present invention, and are intended to illustrate the present invention rather than limit the scope of the present invention.

For all the following examples, standard operation and purification methods known to those skilled in the art can be used. Unless otherwise stated, all temperatures are expressed in °C (degrees Celsius). The structures of the compounds were confirmed by nuclear magnetic resonance (NMR) or mass spectrometry (MS). The melting points of the compounds were determined by an RY-1 melting point apparatus; the thermometer was uncalibrated and is given in °C. $^{1}H$ NMR was determined by a JNM-ECA-600 NMR spectrometer (JEOL). Mass spectrometry was determined by an API 3000 (ESI) mass spectrometer. All solvents used in the reactions were not standardized unless otherwise specified. In the examples below, % refers to mass percentage unless otherwise specified. Silica gel for column chromatography was produced by Qingdao Marine Chemical Plant (200-300 mesh); silica gel plates for thin-layer chromatography were pre-made silica gel plates for thin-layer chromatography produced by Yantai Chemical Industry Research Institute. The compounds of the present invention can be prepared using conventional methods in the art, employing suitable reagents, raw materials, and purification methods known to those skilled in the art.

Example 1: Synthesis of 3-(3-methyl-6-nitro-1-oxo-3,4-dihydrophthalazin-2(1*H*)-yl)piperidin-2,6-dione (compound **I-11**)

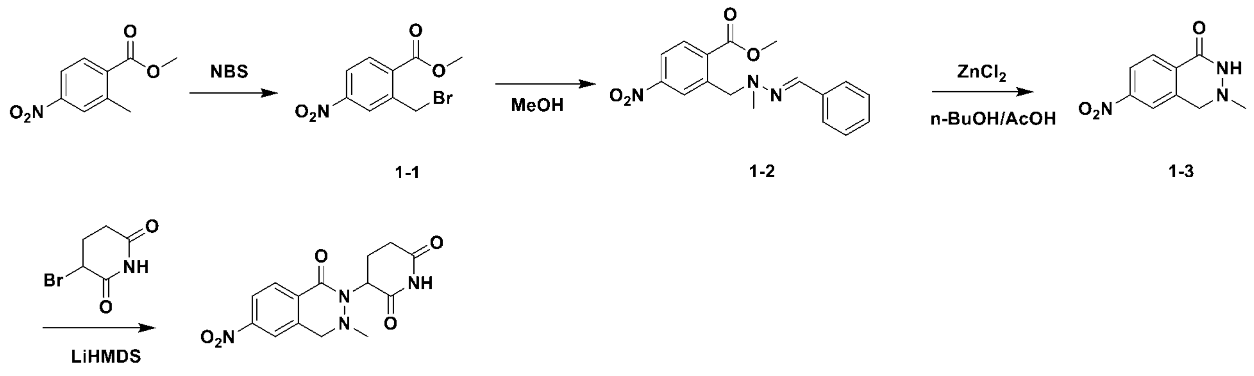


1-1 1-2 1-3

I-11

[1-1] Synthesis of methyl 2-(bromomethyl)-4-nitrobenzoate (compound <**1-1**>)

10 g (51.23 mmol) of methyl 2-methyl-4-nitrobenzoate was weighed and dissolved in 20 mL of dichloroethane, added with 420.4 mg (2.56 mmol) of AIBN and stirred until it was completely dissolved. The mixture was heated to 80°C, and a total of 10.94 g (61.48 mmol) of NBS was added in portions. After the reaction was performed for 1 hour, TLC showed that the reaction was completed. Heating was stopped, and the reaction solution was gradually cooled to 0°C. After 1 hour, a large amount of white crystals precipitated. The crystals were removed by filtration, and the filtrate was concentrated and separated by silica gel column chromatography (eluent: $V_{petroleum\ ether}/V_{ethyl\ acetate}$ = 100/1) to give 10.20 g of intermediate **1-1** as a colorless oily liquid, with a yield of 72.65%. ESI-MS m/z: 274.04 $[M+H]^+$.

[1-2] Synthesis of methyl (*E*)-2-((2-benzylidene-1-methylhydrazineyl)methyl)-4-nitrobenzoate (Compound <**1-2**>)

1.89 g (13.14 mmol) of methylhydrazine sulfate and 1.39 g (13.14 mmol) of anhydrous sodium carbonate were weighed and suspended in 20 mL of methanol, stirred, and heated to 60 °C. After 1 hour, 1.35 mL (13.14 mmol) of benzaldehyde and 3.00 g (10.95 mmol) of compound **1-1** were added to the reaction system, and stirring was continued at 60 °C for 3 hours. After TLC monitoring showed that the reaction was completed, the solid was removed by filtration. The filtrate was concentrated and purified by silica gel column chromatography (eluent: $V_{petroleum\ ether}/V_{ethyl\ acetate}$ = 15/1) to give 2.21 g of compound **1-2** as an orange-yellow solid, with a yield of 61.73%. ESI-MS m/z: 328.12 $[M+H]^+$.

[1-3] Synthesis of 3-methyl-6-nitro-3,4-dihydrophthalazin-1(2*H*)-one (compound <**1-3**>)

1.17 g (3.57 mmol) of compound **1-2** was weighed and added to 5 mL of a mixed solvent ($V_{n\text{-}butanol}/V_{glacial\ acetic\ acid}$ = 1/1), stirred until it was completely dissolved, then added with 24.4 mg (0.18 mmol) of anhydrous zinc chloride, heated to 120 °C and reacted for 6 hours. Heating and stirring were stopped, and the mixture was allowed to stand overnight. A yellow solid precipitated and was filtered. The obtained solid was washed with petroleum ether, and dried to give compound **1-3** (590 mg, yield 79.84%). ESI-HRMS m/z: 206.0560 $[M-H]^-$.

[1-4] Synthesis of 3-(3-methyl-6-nitro-1-oxo-3,4-dihydrophthalazin-2(1*H*)-yl)piperidin-2,6-dione (compound **I-11**)

500 mg of compound **1-3** (2.42 mmol) was weighed and dissolved in 10 mL of anhydrous tetrahydrofuran, stirred under nitrogen protection in an ice bath, and added with 3.6 mL of LiHMDs (3.60 mmol, 1 N in THF). After the addition was completed, the mixture was stirred in an ice bath for 30 minutes. Then, 695 mg (3.62 mmol) of 3-bromopiperidin-2,6-dione dissolved in 5 mL of anhydrous tetrahydrofuran was added. The mixture was then allowed to warm naturally to room temperature, and reacted for 3 hours. When TLC detection showed that the reaction ceased to proceed, the reaction was quenched by adding saturated ammonium chloride solution. After layer separation, the aqueous phase was extracted with ethyl acetate (3 x 10 mL). The organic phases were combined, dried over anhydrous sodium sulfate, filtered, and the filtrate was concentrated and purified by silica gel column chromatography (eluent: $V_{dichloromethane}/V_{methanol}$ = 50/1) to give 20 mg of compound **I-11** as a white to slightly yellow solid, with a yield of 2.60%. ESI-MS m/z: 319.10 $[M+H]^+$. $^1H$-NMR (600 MHz, DMSO-$d_6$) δppm: 10.99 (s, 1H), 8.35 (dd, *J* = 8.3, 1.3 Hz, 1H), 8.26 (dd, *J* = 7.7, 1.3 Hz, 1H), 7.74 (t, *J* = 7.9 Hz, 1H),

5.19 (dd, *J* = 12.4, 4.9 Hz, 1H), 4.68 (d, *J* = 17.2 Hz, 1H), 4.50 (d, *J* = 17.1 Hz, 1H), 2.86 (ddd, *J* = 17.1, 13.7, 5.3 Hz, 1H), 2.58 (d, *J* = 17.2 Hz, 1H), 2.51 (s, 3H), 2.43 (dd, *J* = 13.2, 4.4 Hz, 1H), 2.14-2.10 (m, 1H).

Example 2: Synthesis of 3-(6-amino-3-methyl-1-oxo-3,4-dihydrophthalazin-2(1*H*)-yl)piperidin-2,6-dione (compound **I-10**)

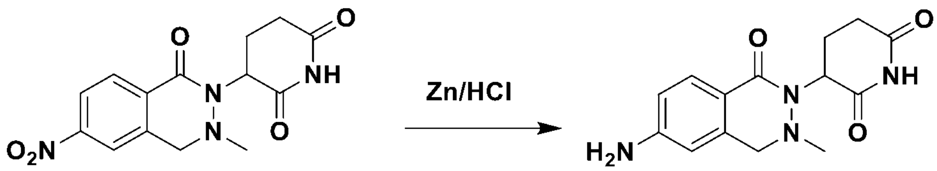


**I-11** **I-10**

[2-1] 20 mg (0.06 mmol) of compound **I-11** was weighed and added to 5 mL of methanol, and stirred, and some of the solid was dissolved. Approximately 8.05 mg (0.13 mmol) of activated zinc powder was added, followed by the dropwise addition of 300 μL of 3M HCl (aq.) under stirring, and reacted overnight at room temperature. The solid was completely dissolved on the next day. TLC detection showed that the reaction was completed. The zinc powder was removed by filtration, and the filtrate was concentrated and subjected to TLC separation to obtain 13 mg of compound **I-10** as a white solid, with a yield of 71.78%. ESI-MS m/z: 289.13 $[M+H]^+$. $^1$H-NMR (600 MHz, DMSO-$d_6$) δ 10.85 (s, 1H), 7.51 (d, *J* = 8.4 Hz, 1H), 6.53 (dd, *J* = 8.4, 2.2 Hz, 1H), 6.35 (d, *J* = 2.2 Hz, 1H), 5.93 (s, 2H), 5.09 (dd, *J* = 12.5, 5.2 Hz, 1H), 4.25 (s, 1H), 3.82 (d, *J* = 12.2 Hz, 1H), 2.82 (ddd, *J* = 17.1, 13.7, 5.3 Hz, 1H), 2.58-2.53 (m, 1H), 2.50 (s, 3H), 2.39 (qd, *J* = 13.0, 4.3 Hz, 1H), 2.02 (dtd, *J* = 12.8, 5.2, 2.7 Hz, 1H).

Example 3: Synthesis of 3-(3-methyl-5-nitro-1-oxo-3,4-dihydrophthalazin-2(1*H*)-yl)piperidin-2,6-dione (compound **I-9**)

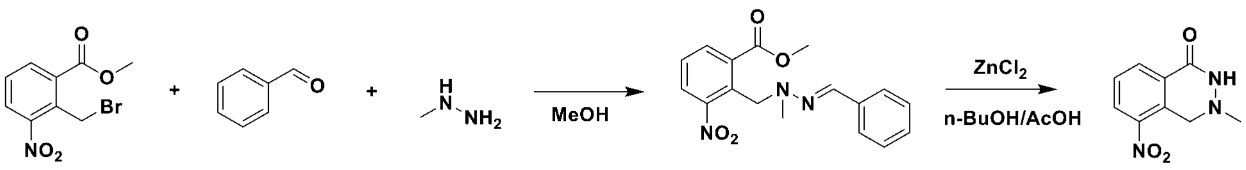


**3-1** **3-2**

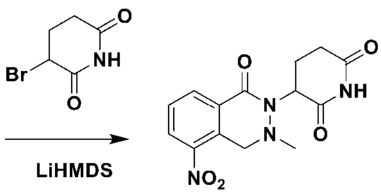


**I-9**

[3-1] Synthesis of methyl (*E*)-2-((2-benzylidene-1-methylhydrazineyl)methyl)-3-nitrobenzoate (compound <**3-1**>)

10.50 g (72.84 mmol) of methylhydrazine sulfate and 11.60 g (109.26 mmol) of anhydrous sodium carbonate were weighed and suspended in 200 mL of methanol, stirred and heated to 60 °C. One hour later, 7.29 mL (72.84 mmol) of benzaldehyde and 20.00 g (72.84 mmol) of methyl 2-bromomethyl-3-nitrobenzoate were added to the reaction system, and the mixture was stirred at 60 °C for another 3 hours. After TLC showed that the reaction of methyl 2-bromomethyl-3-nitrobenzoate was completed, the solid was removed by filtration. The filtrate was concentrated and purified by silica gel column chromatography (eluent: $V_{petroleum\ ether}/V_{ethyl\ acetate}$ = 15/1) to give 17.0 g of compound **3-1** as an orange-yellow solid, with a yield of 71.1%. ESI-MS m/z: 328.12 $[M+H]^+$.

[3-2] Synthesis of 3-methyl-5-nitro-3,4-dihydrophthalazin-1(2*H*)-one (compound <**3-2**>)

17.0 g of compound **3-1** (52.00 mmol) was weighed and added to 50 mL of a mixed solvent ($V_{n\text{-butanol}}/V_{glacial\ acetic\ acid}$ = 1/1), and stirred until it was completely dissolved. 354 mg (2.60 mmol) of anhydrous zinc chloride was added, heated to 120 °C and reacted for 6 hours. Heating and stirring were stopped, and the mixture was allowed to stand overnight. A yellow solid precipitated and was filtered. The obtained solid was washed with petroleum ether, and dried to give compound **3-2** (5.90 g, yield 54.8%). ESI-HRMS m/z: 206.0560 $[M+H]^+$.

[3-3] Synthesis of 3-(3-methyl-5-nitro-1-oxo-3,4-dihydrophthalazin-2(1*H*)-yl)piperidin-2,6-dione (compound **I-9**)

5.00 g of compound **3-2** (24.14 mmol) was weighed and dissolved in 50 mL of anhydrous tetrahydrofuran, stirred under nitrogen protection in an ice bath, and added with 36 mL of LiHMDs (36.00 mmol, 1 N in THF). After the addition was completed, the mixture was stirred in an ice bath for 30 minutes. Then, 6.95 g (36.22 mmol) of 3-bromopiperidin-2,6-dione dissolved in 20 mL of anhydrous tetrahydrofuran was added. The mixture was then allowed to warm naturally to room temperature and reacted for 3 hours. After TLC detection showed that the reaction ceased to proceed, the reaction was quenched by adding saturated ammonium chloride solution. After layer separation, the aqueous phase was extracted with ethyl acetate (3 x 10 mL). The organic phases were combined, dried over anhydrous sodium sulfate, filtered, and the filtrate was concentrated and purified by silica gel column chromatography (eluent: $V_{dichloromethane}/V_{methanol}$ = 50/1) to give 4.01 g of compound **I-9** as a white to slightly yellow solid, with a yield of 52.10%. ESI-HRMS m/z: 318.3001 [M]+. $^1$H-NMR (600 MHz, DMSO-$d_6$) δppm: 11.00 (s, 1H), 8.36 (dd, *J* = 8.2, 1.3 Hz, 1H), 8.26 (dd, *J* = 7.7, 1.3 Hz, 1H), 7.74 (t, *J* = 8.0 Hz, 1H), 5.20 (dd, *J* = 12.8, 5.1 Hz, 1H), 4.68 (d, *J* = 17.2 Hz, 1H), 4.51 (d, *J* = 18.5 Hz, 1H), 2.86 (ddd, *J* = 17.2, 13.8, 5.4 Hz, 1H), 2.60 (t, *J* = 4.0 Hz, 1H), 2.57 (d, *J* = 3.7 Hz, 2H), 2.45 (d, *J* = 10.2 Hz, 1H), 2.43-2.38 (m, 1H), 2.12 (ddq, *J* = 10.5, 5.3, 2.6 Hz, 1H).

Example 4: Synthesis of 3-(5-amino-3-methyl-1-oxo-3,4-dihydrophthalazin-2(1*H*)-yl)piperidin-2,6-dione (compound **I-4**)

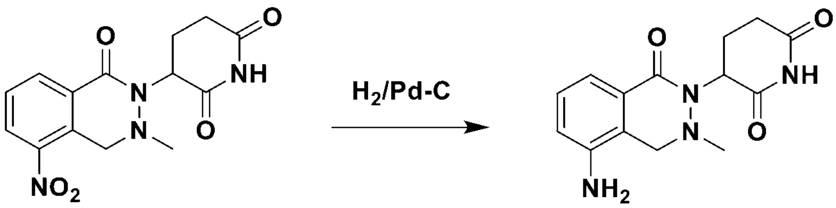


I-9 I-4

[4-1] 4.00 g (12.58 mmol) of compound **I-9** was weighed and added to 40 mL of methanol, and stirred, and some of the solid was dissolved. Approximately 133.86 mg (1.26 mmol) of 5% Pd-C was added, and the reactor was purged with hydrogen gas before being connected to a hydrogen-filled balloon. The reaction was allowed to proceed overnight at room temperature. The solid completely dissolved on the next day, and TLC detection showed that the reaction was completed. The wet palladium-on-carbon catalyst was removed and recovered by filtration with diatomaceous earth. The filtrate was concentrated and purified by silica gel column chromatography (eluent: $V_{dichloromethane}/V_{methanol}$ = 50/1 to 10/1) to obtain 1.21 g of the product **I-4** as a white solid, with a yield of 33.34%. ESI-HRMS m/z: 289.1269 $[M+H]^+$; $^1$H-NMR (600 MHz, DMSO-$d_6$) δppm: 10.90 (s, 1H), 7.13-7.06 (m, 2H), 6.86 (dd, *J* = 7.6, 1.5 Hz, 1H), 5.19 (s, 2H), 5.11 (dd, *J* = 12.7, 5.1 Hz, 1H), 4.18 (d, *J* = 16.2 Hz, 1H), 3.97 (d, *J* = 16.2 Hz, 1H), 2.83 (ddd, *J* = 17.1, 13.8, 5.3 Hz, 1H), 2.58-2.53 (m, 1H), 2.47 (s, 3H), 2.45-2.37 (m, 1H), 2.05 (dtd, *J* = 12.8, 5.2, 2.6 Hz, 1H).

Example 5: Synthesis of 3-(3-methyl-5-(methylamino)-1-oxo-3,4-dihydrophthalazin-2(1*H*)-yl)piperidin-2,6-dione (compound **1-2)**

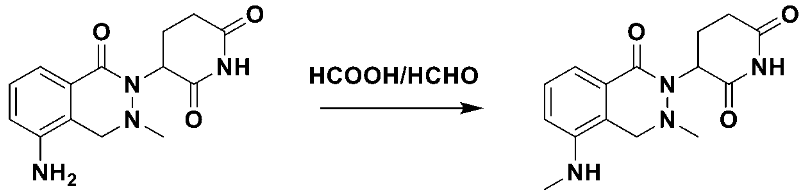


I-4

I-2

[5-1] 300 mg (1.04 mmol) of compound **I-4** and 100 mg (1.61 mmol) of paraformaldehyde were weighed and placed in a 25 mL single-necked flask, and 10 mL of water was added. The reaction flask was placed on a mixer, and formic acid (0.32 g, 7 mmol) was added under stirring. The mixture was then heated to 70 °C, and reacted for 6 hours. TLC showed that the reaction was completed. The reaction flask was cooled to room temperature, and then suction filtration was carried out. The resulting solid was washed with 10 mL of water, 10 mL of dichloromethane, and 10 mL of tetrahydrofuran, respectively. After drying under vacuum, the crude product was purified by silica gel column chromatography (eluent: $V_{dichloromethane}/V_{methanol}$ = 50/1-10/1) to give 180 mg of compound **I-2** as a white solid, with a yield of 57.32%. ESI-HRMS m/z: 303.1455 $[M+H]^+$; $^1$H-NMR (600 MHz, DMSO-$d_6$) δppm: 10.90 (s, 1H), 7.25 (t, *J* = 7.9 Hz, 1H), 7.12 (dd, *J* = 7.6, 1.1 Hz, 1H), 6.76 (dd, *J* = 8.2, 1.1 Hz, 1H), 5.42 (d, *J* = 5.0 Hz, 1H), 5.11 (dd, *J* = 12.7, 5.1 Hz, 1H), 4.19 (d, *J* = 16.2 Hz, 1H), 3.98 (d, *J* = 16.2 Hz, 1H), 2.83 (ddd, *J* = 17.1, 13.8, 5.3 Hz, 1H), 2.73 (d, *J* = 4.8 Hz, 3H), 2.60-2.54 (m, 1H), 2.46 (s, 3H), 2.42 (dd, *J* = 13.3, 4.4 Hz, 1H), 2.06 (dtd, *J* = 12.8, 5.2, 2.6 Hz, 1H).

Example 6: Synthesis of 3-(1-oxophthalazin-2(1H)-yl)piperidin-2,6-dione (compound **YJ-1a)**

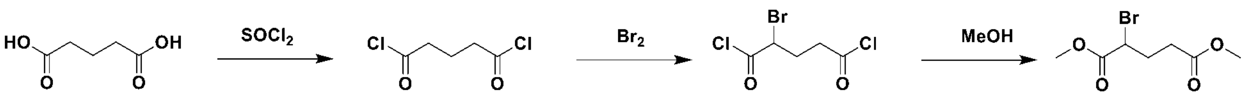


6-1

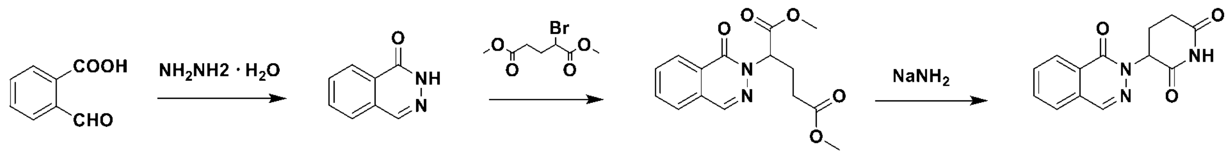


6-2

6-3

YJ-1a

[6-1] Synthesis of dimethyl 2-bromoglutarate (compound <**6-1**>)

Glutaric acid (2.64 g, 20 mmol) was weighed and placed in a 50 mL two-necked round-bottom flask, and 30 mL of dichloromethane was added. The reaction flask was placed on a mixer, and stirring was performed until a suspension was formed at room temperature. Thionyl chloride (7.13 g, 60 mmol) was then slowly added. The mixture was then heated to reflux, and reacted for 10 hours. The flask was allowed to cool to room temperature, and the solvent and unreacted thionyl chloride were evaporated to obtain a golden-yellow liquid. The golden-yellow liquid was transferred to a 100 mL three-necked round-bottom flask, and a thermometer and a serpentine condenser were equipped. The temperature was raised to 80 °C, and liquid bromine (3.20 g, 20.05 mmol) was added dropwise through a dropping funnel. After the first drop of bromine was added, the reaction mixture turned red. The second drop of bromine was added after the red color faded. After the bromine was added completely over 6 hours, the reaction was continued at 80 °C for 12 hours. After the reaction was completed, the flask was allowed to cool to room temperature. Then, 50 mL of methanol was added to a 100 mL one-necked flask, and the flask was placed in an ice bath and stirred for 10 minutes. The reaction mixture was then slowly added dropwise to the methanol. After the dropwise addition was completed, the reaction flask was allowed to stand at room temperature, and stirring was continued for 3 hours. The solvent was then evaporated using a rotary evaporator to obtain 5.22 g of a red liquid. The red liquid was washed three times with 20 mL of saturated sodium bicarbonate solution and 20 mL of saturated sodium thiosulfate solution, and then once with 30 mL of saturated brine. The washed red liquid was then distilled under reduced pressure and fractions at 80-85 °C were collected to obtain 3.63 g of **6-1** as a pale yellow liquid, with a yield of 76.1%. $^1$H-NMR (600 MHz, $CDCl_3$) δppm: 4.39 (dd, *J* = 8.5, 5.8 Hz, 1H), 3.79 (s, 3H), 3.70 (s, 3H), 2.59-2.46 (m, 2H), 2.44-2.34 (m, 2H). ESI-MS m/z: 240.99 $[M+H]^+$.

[6-2] Synthesis of phthalazin-1-(2*H*)-one (compound <**6-2**>)

2-Formylbenzoic acid (7.50 g, 50 mmol) was weighed and placed in a 200 mL single-necked round-bottom flask, and 50 mL of n-butanol was added. The reaction flask was placed on a mixer, and stirring was performed at room temperature until a suspension was formed. Then, 85% hydrazine hydrate (3.53 g, 60 mmol) was slowly added. The temperature was raised to 100 °C, the reaction was carried out for 2 hours, and the reaction mixture was allowed to stand until being cooled to room temperature. The mixture was filtered by suction. The solid was washed with dichloromethane, then with water, and dried to obtain 5.85 g of **6-2** as white needle-like crystals, with a yield of 80.5%. $^1$H-NMR (600 MHz, DMSO-$d_6$) δppm: 12.66 (s, 1H), 8.37 (d, *J* = 0.7 Hz, 1H), 8.24 (dq, *J* = 7.8, 0.9 Hz, 1H), 7.97-7.91 (m, 2H), 7.86 (dp, *J* = 8.5, 4.3 Hz, 1H); ESI-MS m/z: 147.06 $[M+H]^+$.

[6-3] Synthesis of dimethyl 2-(1-oxophthalazin-2(1H)-yl)glutarate (compound <**6-3**>)

**6-2** (0.44 g, 3 mmol) and potassium carbonate (0.46 g, 3.3 mmol) were weighed and placed in a 50 mL two-necked round-bottom flask, 10 mL of DMF was added, and the flask was placed on a mixer. **6-1** (0.87 g, 3.6 mmol) was added under rapid stirring, and the reaction temperature was then raised to 80 °C. After 18 hours of reaction, TLC showed that the reaction was completed. After the reaction flask was allowed to stand and cooled at room temperature, the reaction mixture was added dropwise to 20 mL of cold water, and extracted three times with 30 mL of ethyl acetate. The organic layers were combined and dried over anhydrous sodium sulfate. Silica gel was then added and mixed thoroughly. The mixture was then subjected to column chromatography (eluent: $V_{petroleum\ ether}/V_{ethyl\ acetate}$ = 15/1) to give 0.78 g of **6-3** as a colorless and transparent liquid, with a yield of 86.2%. $^1$H-NMR (400 MHz, $CDCl_3$) δppm: 8.43 (dd, *J* = 7.8, 1.4 Hz, 1H), 8.22 (s, 1H), 7.89-7.76 (m, 2H), 7.73 (dd, *J* = 7.9, 1.3 Hz, 1H), 5.74 (dd, *J* = 10.0, 5.0 Hz, 1H), 3.74 (s, 3H), 3.63 (s, 3H), 2.71-2.47 (m, 2H), 2.43-2.31 (m, 2H). ESI-MS m/z: 305.11 $[M+H]^+$.

[6-4] Synthesis of 3-(1-oxophthalazin-2(1*H*)-yl)piperidin-2,6-dione (compound **YJ-1a)**

Sodium amide (0.12 g, 3 mmol) was weighed and placed in a 50 mL two-necked round-bottom flask. Nitrogen protection was applied through a three-way valve. 10 mL of ultra-dry tetrahydrofuran was added via syringe, and the reaction flask was then placed in a -30°C cooling bath. After cooling for 20 minutes, **6-3** (0.60 g, 2 mmol) was dissolved in 10 mL of ultra-dry tetrahydrofuran and slowly added dropwise to the reaction flask over 10 minutes via a syringe. After reacting in the cooling bath for 6 hours, 5 mL of saturated ammonium chloride solution was slowly added, and the reaction flask was then allowed to stand at room temperature and stirring was continued for 30 minutes. The mixture was filtered by suction. The solid was washed with 40 mL of water, 30 mL of dichloromethane, and 30 mL of tetrahydrofuran, respectively and then dried to obtain 0.28 g of **YJ-1a** as a white solid, with a yield of 56.5%. $^1$H-NMR (400 MHz, DMSO-$d_6$) δppm: 11.07 (s, 1H), 8.52-8.48 (m, 1H), 8.28 (dt, *J* = 7.9, 1.0 Hz, 1H), 7.99 (dd, *J* = 3.7, 1.1 Hz, 2H), 7.91 (ddd, *J* = 8.3, 4.9, 3.6 Hz, 1H), 5.83 (dd, *J* = 12.3, 5.4 Hz, 1H), 2.94 (ddd, *J* = 17.3, 13.7, 5.4 Hz, 1H), 2.67-2.52 (m, 2H), 2.19-2.08 (m, 1H). ESI-MS m/z: 258.09 $[M+H]^+$.

Example 7: Synthesis of 3-(1-oxo-3,4-dihydrophthalazin-2(1*H*)-yl)piperidin-2,6-dione (compound **YJ-2a)**

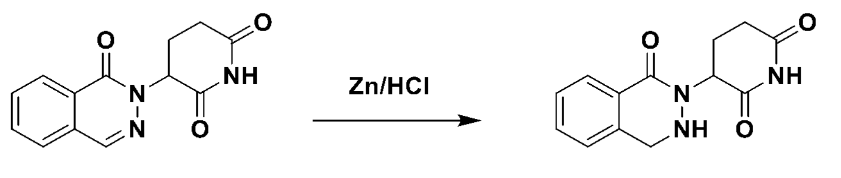


YJ-1a YJ-2a

[7-1] Compound **YJ-1a** (0.39 g, 1.5 mmol) and zinc powder (0.16 g, 2.5 mmol) were weighed and placed in a 50 mL single-necked round-bottom flask. 10 mL of water was added, and the reaction flask was placed in an ice bath. After 10 minutes, 2 mL of 3 mol/L hydrochloric acid was slowly added dropwise to the reaction flask through a dropping funnel, completing the addition over 10 minutes. The reaction was maintained in an ice bath for 3 hours, and then the mixture was heated to room temperature and stirred for 30 minutes. The mixture was filtered by suction. The solid was washed with water, and dried under vacuum. The obtained solid was then slurried in 20 mL of a mixed solvent ($V_{dichloromethane}/V_{methanol}$ = 10/1). After 30 minutes, the mixture was filtered by suction, and the filtrate was evaporated to dryness to obtain 0.14 g of

compound **YJ-2a** as a white solid, with a yield of 37.6%. $^1$H-NMR (400 MHz, DMSO-$d_6$) δppm: 10.93 (s, 1H), 7.89 (dd, *J* = 7.7, 1.4 Hz, 1H), 7.53 (td, *J* = 7.5, 1.4 Hz, 1H), 7.42 (td, *J* = 7.6, 1.3 Hz, 1H), 7.31-7.25 (m, 1H), 5.84 (t, *J* = 8.2 Hz, 1H), 5.36 (dd, *J* = 12.4, 5.3 Hz, 1H), 4.13-3.92 (m, 2H), 2.85 (ddd, *J* = 17.2, 13.8, 5.4 Hz, 1H), 2.62-2.52 (m, 1H), 2.38-2.23 (m, 1H), 1.93 (dtd, *J* = 13.1, 5.4, 2.6 Hz, 1H). ESI-MS m/z: 260.11 [M+H]$^+$.

Example 8: Synthesis of 3-(3-methyl-1-oxo-3,4-dihydrophthalazin-2(1*H*)-yl)piperidin-2,6-dione (compound **YJ-3a)**

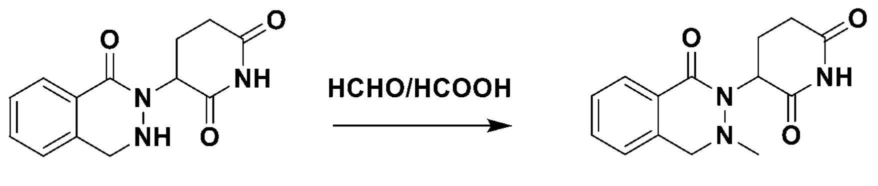


**YJ-2a** **YJ-3a**

[8-1] Compound **YJ-2a** (0.30 g, 1 mmol) and paraformaldehyde (0.05 g, 1.5 mmol) were weighed and placed in a 25 mL single-necked flask, and 10 mL of water was added. The reaction flask was placed on a mixer, and formic acid (0.32 g, 7 mmol) was added while stirring. The temperature was then raised to 70 °C, and the reaction was carried out for 6 hours. TLC showed that the reaction was completed. The reaction flask was cooled to room temperature, and the mixture was filtered by suction. The obtained solid was washed with 10 mL of water, 10 mL of dichloromethane, and 10 mL of tetrahydrofuran, respectively. The solid was dried under vacuum to give 0.25 g of compound **YJ-3a** as a white solid, with a yield of 93.3%. $^1$H-NMR (400 MHz, DMSO-$d_6$) δppm: 10.96 (s, 1H), 7.77 (td, *J* = 5.5, 2.6 Hz, 1H), 7.57 (td, *J* = 7.6, 1.8 Hz, 1H), 7.43 (t, *J* = 7.6 Hz, 2H), 4.96 (s, 2H), 4.63-4.18 (m, 3H), 3.60 (s, 2H), 2.78 (t, *J* = 15.7 Hz, 1H), 2.26-1.32 (m, 2H). ESI-MS m/z: 272.10 [M+H]$^+$.

Example 9: Synthesis of 3-(1-oxophthalazin-2(1*H*)-yl)pyrrolidin-2,5-dione (compound **YJ-1b)**

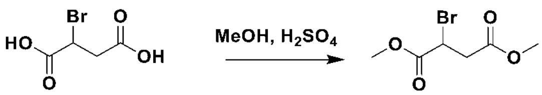


**9-1**

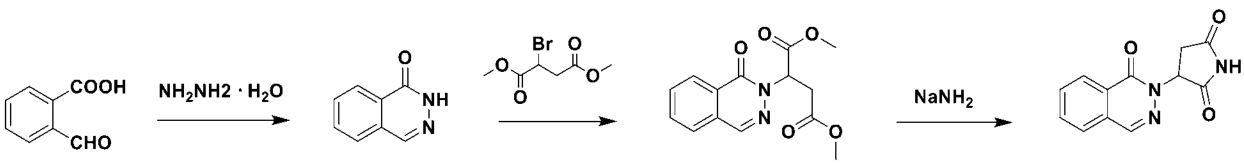


**9-2** **9-3** **YJ-1b**

[9-1] Synthesis of dimethyl 2-bromosuccinate (compound <**9-1**>)

2-Bromosuccinic acid (1.97 g, 10 mmol) was weighed and placed in a 50 mL single-necked round-bottom flask, then added with 20 mL of methanol and stirred until it was completely dissolved. The reaction flask was placed on a mixer, and 3 drops of concentrated sulfuric acid were added dropwise under stirring at room temperature. The reaction temperature was then raised to reflux. After reflux and reaction for 16 hours, the reaction flask was cooled to room temperature, and the solvent was evaporated to dryness using a rotary evaporator. 30 mL of ethyl acetate was added to the evaporated liquid, which was transferred to a separatory funnel and washed three times with 30 mL of saturated sodium bicarbonate, followed by one wash with saturated brine. The organic layer was dried over anhydrous sodium sulfate, and then evaporated to dryness using a rotary evaporator to obtain 2.05 g of **9-1** as a colorless and transparent liquid, with a yield of 91.2%. $^1$H-NMR (600 MHz, $CDCl_3$) δppm: 4.59 (dd, *J* = 8.8, 6.2 Hz, 1H), 3.81 (s, 3H), 3.72 (s, 3H), 3.32-2.97 (m,

2H). ESI-MS m/z: 226.98 $[M+H]^+$.

[9-2] Synthesis of phthalazin-1-(2*H*)-one (compound <**9-2**>)

2-Formylbenzoic acid (7.50 g, 50 mmol) was weighed and placed in a 200 mL single-necked round-bottom flask, and 50 mL of n-butanol was added. The reaction flask was placed on a mixer, and the mixture was stirred at room temperature until a suspension was formed. Then, 85% hydrazine hydrate (3.53 g, 60 mmol) was slowly added. The temperature was raised to 100 °C, the reaction was carried out for 2 hours, and then the reaction mixture was allowed to stand at and cooled to room temperature. The mixture was filtered by suction. The solid was washed with dichloromethane and then with water, and dried to obtain 5.85 g of **9-2** as white needle-like crystals, with a yield of 80.5%. $^1$H-NMR (600 MHz, DMSO-$d_6$) δppm: 12.66 (s, 1H), 8.37 (d, *J* = 0.7 Hz, 1H), 8.24 (dq, *J* = 7.8, 0.9 Hz, 1H), 7.97-7.91 (m, 2H), 7.86 (dp, *J* = 8.5, 4.3 Hz, 1H); ESI-MS m/z: 147.06 $[M+H]^+$.

[9-3] Synthesis of dimethyl 2-(1-oxophthalazin-2(1H)-yl)succinate (compound <**9-3**>)

**9-2** (0.44 g, 3 mmol) and potassium carbonate (0.46 g, 3.3 mmol) were weighed and placed in a 50 mL two-necked round-bottom flask, and 10 mL of DMF was added. The reaction flask was placed on a mixer. **9-1** (0.81 g, 3.6 mmol) was added under rapid stirring, and then heated to 80 °C. After the reaction was carried out for 24 hours, TLC showed that the reaction was completed. The reaction flask was allowed to stand at room temperature, and the reaction solution was added dropwise to 20 mL of cold water. The mixture was extracted three times with 30 mL of ethyl acetate. The organic layers were combined and dried over anhydrous sodium sulfate, then added with silica gel and stirred well, and subjected to column chromatography (eluent: $V_{\text{petroleum ether}}/V_{\text{ethyl acetate}}$ = 15/1) to obtain 0.77 g of **9-3** as a colorless and transparent liquid, with a yield of 88%. $^1$H-NMR (400 MHz, $CDCl_3$) δppm: 8.44 (dd, *J* = 7.7, 1.5 Hz, 1H), 8.19 (s, 1H), 7.88-7.69 (m, 3H), 6.08 (dd, *J* = 8.1, 6.0 Hz, 1H), 3.75 (s, 3H), 3.69 (s, 3H). 3.45-3.08 (m, 2H). ESI-MS m/z: 291.18 $[M+H]^+$.

[9-4] Synthesis of 3-(1-oxophthalazin-2(1*H*)-yl)pyrrolidin-2,5-dione (compound **YJ-1b)**

Sodium amide (0.12 g, 3 mmol) was weighed and placed in a 50 mL two-necked round-bottom flask under nitrogen protection. 10 mL of ultra-dry tetrahydrofuran was added, and the reaction flask was then placed in a -30°C cooling bath. After cooling for 20 minutes, **9-3** (0.58 g, 2 mmol) was dissolved in 10 mL of ultra-dry tetrahydrofuran, and slowly added dropwise to the reaction flask over 10 minutes using a syringe. After the reaction was carried out in the cooling bath for 6 hours, 5 mL of saturated ammonium chloride solution was slowly added. The reaction flask was then allowed to stand at room temperature and stirring was continued for 30 minutes. The mixed was filtered by suction. The obtained solid was washed with water, dichloromethane, and tetrahydrofuran, respectively, to obtain 0.24 g of compound **YJ-1b** as a white solid, with a yield of 50.2%. $^1$H-NMR (400 MHz, DMSO-$d_6$) δppm: 11.57 (s, 1H), 8.52 (s, 1H), 8.26 (dd, *J* = 8.0, 1.0 Hz, 1H), 8.03-7.96 (m, 2H), 7.92 (dq, *J* = 8.3, 4.3 Hz, 1H), 5.78 (s, 1H), 3.12 (dd, *J* = 17.8, 9.5 Hz, 1H), 2.90 (dd, *J* = 17.8, 5.2 Hz, 1H). ESI-MS m/z: 244.08 $[M+H]^+$.

Example 10: Synthesis of 3-(1-oxo-3,4-dihydrophthalazin-2(1*H*)-yl)pyrrolidin-2,5-dione (compound **YJ-2b)**

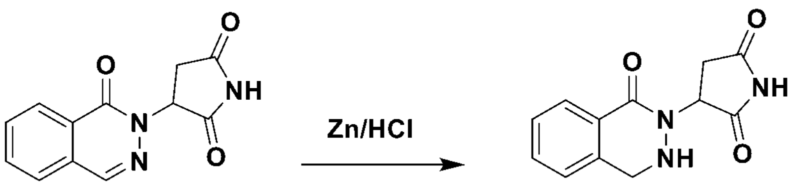


**YJ-1b** **YJ-2b**

[10-1] Compound **YJ-1b** (0.36 g, 1.5 mmol) and zinc powder (0.16 g, 2.5 mmol) were weighed and placed in a 50 mL single-necked round-bottom flask, and 10 mL of water was added. The reaction flask was placed in an ice bath. After 10 minutes, 3 mL of 3 mol/L hydrochloric acid was slowly added dropwise to the reaction flask through a dropping funnel, and the dropwise addition was completed over 10 minutes. The reaction was maintained in an ice bath for 3 hours, and then the mixture was heated to room temperature and stirred for 30 minutes. The mixture was filtered by suction. The obtained solid was washed with water, and dried under vacuum. The resulting solid was then slurried in 20 mL of a mixed solvent (V

$_{dichloromethane}$/V $_{methanol}$ = 10/1). After 30 minutes, the mixture was filtered by suction, and the filtrate was evaporated to dryness to obtain 0.19 g of compound **YJ-2b** as a white solid, with a yield of 35.1%. $^1$H-NMR (400 MHz, DMSO-$d_6$) δppm: 11.42 (s, 1H), 7.87 (dd, *J* = 7.8, 1.3 Hz, 1H), 7.54 (td, *J* = 7.5, 1.4 Hz, 1H), 7.42 (t, *J* = 7.5 Hz, 1H), 7.28 (d, *J* = 7.5 Hz, 1H), 6.12 (dd, *J* = 9.4, 7.1 Hz, 1H), 5.49 (dd, *J* = 9.3, 5.1 Hz, 1H), 4.12-3.94 (m, 2H), 2.94 (dd, *J* = 17.8, 9.2 Hz, 1H), 2.71 (dd, *J* = 17.8, 5.1 Hz, 1H). ESI-MS m/z: 246.09 $[M+H]^+$.

Example 11: Synthesis of 3-(7-fluoro-3-methyl-5-nitro-1-oxo-3,4-dihydrophthalazin-2(1*H*)-yl)piperidin-2,6-dione (compound **I-13**)

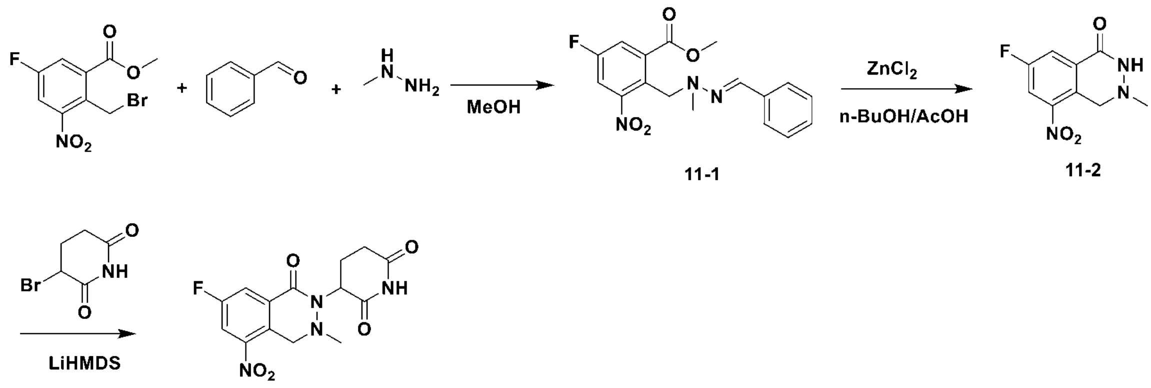


11-1

11-2

I-13

[11-1] Synthesis of methyl (*E*)-2-((2-benzylidene-1-methylhydrazineyl)methyl)-5-fluoro-3-nitrobenzoate (compound <**11-1**>)

217.7 mg (2.05 mmol) of sodium carbonate and 295.5 mg (2.05 mmol) of methylhydrazine sulfate were weighed and suspended in 10 mL of methanol. The mixture was heated to 60 °C and reacted for 1 hour. 209 μL (2.05 mmol) of benzaldehyde and 500 mg of methyl 2-(bromomethyl)-5-fluoro-3-nitrobenzoate were added, and the reaction was continued for 5 hours. After TLC detection showed that the reaction was completed, the solid was removed by filtration. The residue was purified by silica gel column chromatography to give 460 mg of **11-1** as an orange-yellow solid, with a yield of 77.80%. ESI-MS m/z: 346.09 $[M+H]^+$

[11-2] Synthesis of 7-fluoro-3-methyl-5-nitro-3,4-dihydrophthalazin-1(2*H*)-one (compound <**11-2**>)

460 mg (1.33 mmol) of **11-1** was weighed and dissolved in a mixed solvent of 3 mL n-butanol and 3 mL glacial acetic acid, and 10 mg (0.07 mmol) of anhydrous zinc chloride was added. The mixture was heated to 120 °C and reacted for 3 hours. When TLC showed that the reaction was completed, the solution was cooled to room temperature and purified by silica gel column chromatography to give 210 mg of **11-2** as a yellow solid, with a yield of 70.12%. ESI-MS m/z: 224.05 $[M-H]^-$

[11-3] Synthesis of 3-(7-fluoro-3-methyl-5-nitro-1-oxo-3,4-dihydrophthalazin-2(1*H*)-yl)piperidin-2,6-dione (compound **I-13**)

210 mg of **11-2** was weighed and dissolved in 5 mL of anhydrous tetrahydrofuran. Under nitrogen protection, the mixture was pre-cooled at -20 °C. 1.2 mL of LiHMDs (1.2 mmol, 1 M in THF) was added to the reaction system. After stirring at -20 °C for 30 minutes, 358.1 mg (1.87 mmol) of 3-bromopiperidin-2,6-dione dissolved in 5 mL of anhydrous tetrahydrofuran was added. The mixture was then allowed to react overnight at room temperature. After TLC detection showed that the reaction was completed, the reaction was quenched with water. After layer separation, the aqueous phase was extracted with ethyl acetate (2 × 10 mL). The organic phases were combined, dried, and filtered, and the filtrate was concentrated and purified by silica gel column chromatography to give 70 mg of compound **I-13** as a white solid, with a yield of 22.39%. $^1$H-NMR (600 MHz, DMSO-$d_6$) δppm: 11.01 (s, 1H), 8.31 (dd, *J* = 8.5, 2.8 Hz, 1H), 8.06 (dd, *J* = 8.0, 2.8 Hz, 1H), 5.19 (dd, *J* = 12.8, 5.0 Hz, 1H), 4.64 (d, *J* = 17.1 Hz, 1H), 4.49 (d, *J* = 17.1 Hz, 1H), 2.86 (ddd, *J* = 17.0, 13.7, 5.4 Hz, 1H), 2.58 (dt, *J* = 18.4, 4.7 Hz, 2H), 2.46-2.37 (m, 1H), 2.15-2.09 (m, 1H); ESI-MS m/z: 337.10 $[M+H]^+$.

Example 12: Synthesis of 3-(5-amino-7-fluoro-3-methyl-1-oxo-3,4-dihydrophthalazin-2(1*H*)-yl)piperidin-2,6-dione (compound **I-12**)

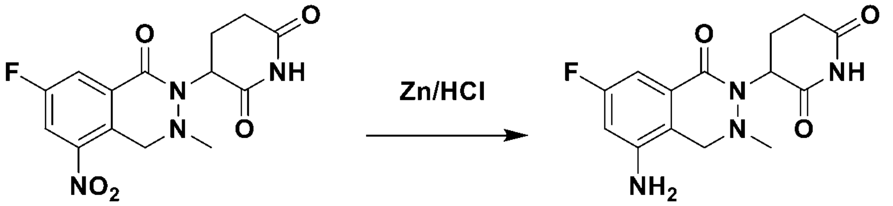


I-13 I-12

[12-1] 50 mg (0.15 mmol) of compound **I-13** was weighed into a round-bottom flask, 20 mg (0.30 mmol) of activated zinc powder and 5 mL of methanol were added, and then 2 mL of 3M hydrochloric acid aqueous solution was slowly added dropwise to the reaction solution. After the dropwise addition was completed, the mixture was stirred for 1 hour. When TLC detection showed that the reaction was completed, excess zinc powder was removed by filtration, and the filtrate was concentrated and purified by silica gel column chromatography to obtain 20 mg of compound **I-12** as a white solid, with a yield of 43.57%. $^1$H-NMR (600 MHz, DMSO-$d_6$) δppm: 10.91 (s, 1H), 6.74 (dd, *J* = 8.8, 2.6 Hz, 1H), 6.63 (dd, *J* = 11.5, 2.6 Hz, 1H), 5.56 (s, 2H), 5.10 (dd, *J* = 12.9, 5.1 Hz, 1H), 4.14 (d, *J* = 16.1 Hz, 1H), 3.95 (d, *J* = 16.2 Hz, 1H), 2.87-2.79 (m, 1H), 2.57 (t, *J* = 3.7 Hz, 1H), 2.47 (s, 3H), 2.41 (qd, *J* = 13.0, 4.2 Hz, 1H), 2.10-2.01 (m, 1H); ESI-MS m/z: 307.12 [M+H]$^+$

Example 13: Synthesis of tert-butyl 4-(4-(((2-(2,6-dioxopiperidin-3-yl)-3-methyl-1-oxo-1,2,3,4-tetrahydrophthalazin-5-yl)amino)methyl)benzyl)piperazin-1-carboxylate (compound **I-5**)

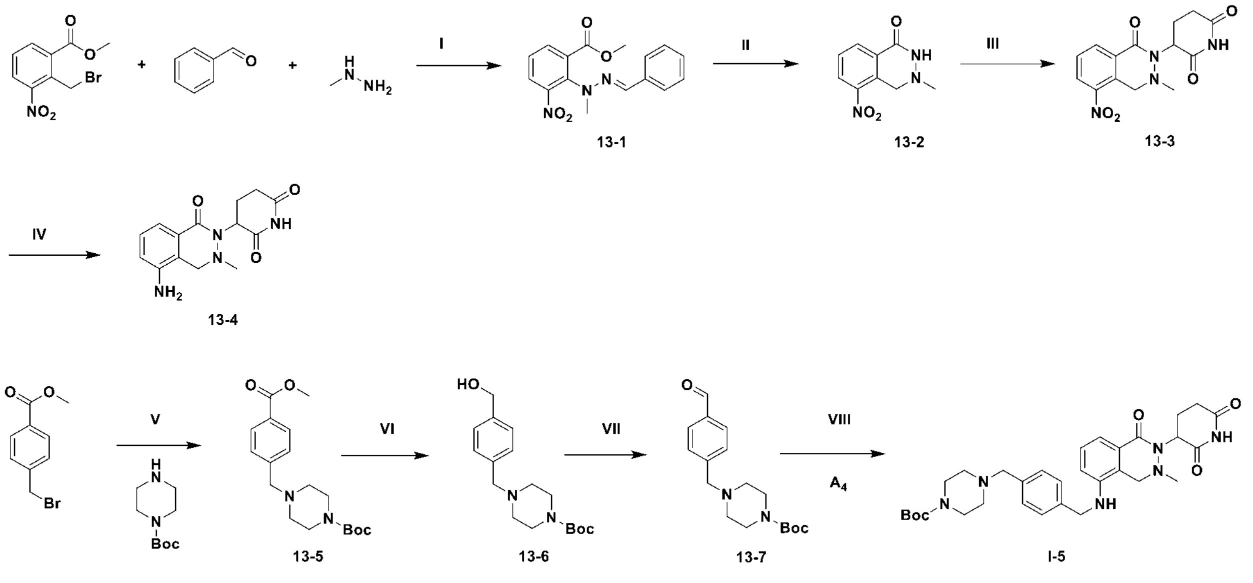


13-1 13-2 13-3 13-4 13-5 13-6 13-7 I-5

[13-1] Synthesis of methyl (*E*)-2-((2-benzylidene-1-methylhydrazineyl)methyl)-3-nitrobenzoate (compound <**13-1**>)

10.50 g (72.84 mmol) of methylhydrazine sulfate and 11.60 g (109.26 mmol) of anhydrous sodium carbonate were weighed and suspended in 200 mL of methanol, stirred, and heated to 60 °C. One hour later, 7.29 mL (72.84 mmol) of benzaldehyde and 20.00 g (72.84 mmol) of methyl 2-bromomethyl-3-nitrobenzoate were added to the reaction system, and the mixture was stirred at 60 °C for another 3 hours. After TLC detection showed that the reaction of methyl 2-bromomethyl-3-nitrobenzoate was completed, the solid was removed by filtration. The filtrate was concentrated and purified by silica gel column chromatography (eluent: $V_{petroleum\ ether}/V_{ethyl\ acetate}$ = 15/1) to give 17.0 g of compound **13-1** as an orange-yellow solid, with a yield of 71.1%. ESI-MS m/z: 328.12 [M+H]$^+$.

[13-2] Synthesis of 3-methyl-5-nitro-3,4-dihydrophthalazin-1(2*H*)-one (compound <**13-2**>)

17.0 g of compound **13-1** (52.00 mmol) was weighed and added to 50 mL of a mixed solvent ($V_{n\text{-bucanol}}/V_{\text{glacial acetic acid}}$ = 1/1), and stirred until it was completely dissolved. 354 mg (2.60 mmol) of anhydrous zinc chloride was added. The mixture was heated to 120 °C and reacted for 6 hours. Heating and stirring were stopped, and the mixture was allowed to stand overnight. A yellow solid precipitated and was filtered. The obtained solid was washed with petroleum ether, and dried to give compound **13-2** (5.90 g, yield 54.8%). ESI-HRMS m/z: 206.0560 $[M+H]^+$.

[13-3] Synthesis of 3-(3-methyl-5-nitro-1-oxo-3,4-dihydrophthalazin-2(1*H*)-yl)piperidin-2,6-dione (compound <**13-3**>)

5.00 g of compound **13-2** (24.14 mmol) was weighed and dissolved in 50 mL of anhydrous tetrahydrofuran under nitrogen protection. The mixture was stirred in an ice bath, and 36 mL of LiHMDs (36.00 mmol, 1 N in THF) was added. After the addition was completed, the mixture was stirred in an ice bath for 30 minutes. Then, 6.95 g (36.22 mmol) of 3-bromopiperidin-2,6-dione dissolved in 20 mL of anhydrous tetrahydrofuran was added. The mixture was allowed to warm naturally to room temperature and reacted for 3 hours. After TLC detection showed that the reaction ceased to proceed, the reaction was quenched by adding saturated ammonium chloride solution. After layer separation, the aqueous phase was extracted with ethyl acetate (3 × 10 mL). The organic phases were combined, dried over anhydrous sodium sulfate, and filtered, and the filtrate was concentrated and purified by silica gel column chromatography (eluent: $V_{\text{dichloromethane}}/V_{\text{methanol}}$ = 50/1) to give 4.01 g of compound **13-3** as a white to slightly yellow solid, with a yield of 52.10%. ESI-HRMS m/z: 318.3001 [M]+. $^1$H-NMR (600 MHz, DMSO-$d_6$) δppm: 11.00 (s, 1H), 8.36 (dd, *J* = 8.2, 1.3 Hz, 1H), 8.26 (dd, *J* = 7.7, 1.3 Hz, 1H), 7.74 (t, *J* = 8.0 Hz, 1H), 5.20 (dd, *J* = 12.8, 5.1 Hz, 1H), 4.68 (d, *J* = 17.2 Hz, 1H), 4.51 (d, *J* = 18.5 Hz, 1H), 2.86 (ddd, *J* = 17.2, 13.8, 5.4 Hz, 1H), 2.60 (t, *J* = 4.0 Hz, 1H), 2.57 (d, *J* = 3.7 Hz, 2H), 2.45 (d, *J* = 10.2 Hz, 1H), 2.43-2.38 (m, 1H), 2.12 (ddq, *J* = 10.5, 5.3, 2.6 Hz, 1H).

[13-4] Synthesis of 3-(5-amino-3-methyl-1-oxo-3,4-dihydrophthalazin-2(1*H*)-yl)piperidin-2,6-dione (compound <**13-4**>)

4.00 g (12.58 mmol) of compound **13-3** was weighed and added to 40 mL of methanol, and stirred, and some of the solid was dissolved. Approximately 133.86 mg (1.26 mmol) of 5% Pd-C was added. The reactor was then purged with hydrogen gas and connected to a hydrogen-filled balloon. The reaction was allowed to proceed overnight at room temperature. The solid completely dissolved on the next day. TLC detection showed that the reaction was completed. The wet palladium-on-carbon catalyst was removed and recovered by filtration with diatomaceous earth. The filtrate was concentrated and purified by silica gel column chromatography (eluent: $V_{\text{dichloromethane}}/V_{\text{methanol}}$ = 50/1-10/1) to give 1.21 g of compound **13-4** as a white solid, with a yield of 33.34%. ESI-HRMS m/z: 289.1269 $[M+H]^+$; $^1$H-NMR (600 MHz, DMSO-$d_6$) δppm: 10.90 (s, 1H), 7.13-7.06 (m, 2H), 6.86 (dd, *J* = 7.6, 1.5 Hz, 1H), 5.19 (s, 2H), 5.11 (dd, *J* = 12.7, 5.1 Hz, 1H), 4.18 (d, *J* = 16.2 Hz, 1H), 3.97 (d, *J* = 16.2 Hz, 1H), 2.83 (ddd, *J* = 17.1, 13.8, 5.3 Hz, 1H), 2.58-2.53 (m, 1H), 2.47 (s, 3H), 2.45-2.37 (m, 1H), 2.05 (dtd, *J* = 12.8, 5.2, 2.6 Hz, 1H).

[13-5] Synthesis of tert-butyl 4-(4-(methoxycarbonyl)benzyl)piperazine-1-carboxylate (compound <**13-5**>)

1.00 g (4.37 mmol) of methyl 4-bromomethylbenzoate was dissolved in 20 mL of ethyl acetate. 1.21 g (8.74 mmol) of potassium carbonate and 0.81 g (4.37 mmol) of 1-tert-butyloxycarbonylpiperazine were added, and the mixture was heated to 60 °C and reacted for 1 hour. TLC detection showed that the reaction was completed, and a large amount of white solid produced. The solid was removed by filtration, and the filtrate was concentrated to obtain a colorless oily liquid, which was compound **13-5,** and could be used directly in the next step without further purification. ESI-HRMS m/z: 335.1965 $[M+H]^+$; $^1$H-NMR (600 MHz, DMSO-$d_6$) δppm: 7.97-7.91 (m, 2H), 7.50-7.43 (m, 2H), 3.86 (s, 3H), 3.55 (s, 2H), 3.37-3.29 (m, 4H), 2.32 (t, *J* = 5.1 Hz, 4H), 1.40 (s, 9H).

[13-6] Synthesis of tert-butyl 4-(4-(hydroxymethyl)benzyl)piperazine-1-carboxylate (compound <**13-6**>)

Compound **13-5** (4.37 mmol) obtained in the previous step was added to a 100 mL three-necked flask, and 20 mL of anhydrous tetrahydrofuran was added to dissolve it. The flask was purged with nitrogen, and connected with a nitrogen-filled balloon. The reaction flask was pre-cooled to -78°C, and then 10.9 mL of DIBAL-H (10.91 mmol, 1 M in THF) was slowly added. After the addition was completed, the reaction was carried out at -78°C. After 6 hours, TLC detection showed that the reaction was completed. $H_2O$ was carefully added to quench the reaction. The reaction solution released heat and solidified into a gel. The reaction flask was brought to room temperature, sufficient saturated potassium sodium tartrate solution was added until the gel was dissolved, and then the mixture was extract with ethyl acetate (3 × 10 mL). The organic phases were combined, dried over anhydrous sodium sulfate, and filtered, and the filtrate was evaporated to dryness to obtain compound **13-6** as a colorless oily liquid, which could be directly used for the next reaction. ESI-MS m/z: 307.20

$[M+H]^+$; $^1H$-NMR (600 MHz, DMSO-$d_6$) δppm: 7.27 (d, *J* = 8.0 Hz, 2H), 7.24 (d, *J* = 8.2 Hz, 2H), 5.14 (t, *J* = 5.7 Hz, 1H), 4.48 (d, *J* = 4.2 Hz, 2H), 3.30 (t, *J* = 5.1 Hz, 4H), 2.29 (t, *J* = 5.1 Hz, 4H), 1.39 (s, 9H).

[13-7] Synthesis of tert-butyl 4-(4-formylbenzyl)piperazine-1-carboxylate (compound <**13-7**>)

The oily compound **13-6** (4.37 mmol) obtained in the previous step was dissolved in 20 mL of dichloromethane, added with 3.81 g (43.70 mmol) of activated manganese dioxide powder, heated and refluxed for 2 h under stirring. TLC detection showed that the reaction was completed. The solid manganese dioxide was removed by filtration with diatomaceous earth. The filtrate was concentrated and separated by silica gel column chromatography (eluent: $V_{\text{petroleum ether}}/V_{\text{ethyl acetate}}$ = 10/1) to obtain 300 mg of compound **13-7** as a colorless oily liquid, in which the overall yield of the three-step reaction was 22.56%. ESI-HRMS m/z: 305.1861 $[M+H]^+$; $^1H$-NMR (600 MHz, DMSO-$d_6$) δppm: 9.99 (s, 1H), 7.87 (d, *J* = 7.9 Hz, 2H), 7.54 (d, *J* = 7.9 Hz, 2H), 3.58 (s, 2H).3.35-3.28 (m, 4H), 2.32 (t, *J* = 5.0 Hz, 4H), 1.39 (s, 9H).

[13-8] Synthesis of tert-butyl 4-(4-(((2-(2,6-dioxopiperidin-3-yl)-3-methyl-1-oxo-1,2,3,4-tetrahydrophthalazin-5-yl)amino)methyl)benzyl)piperazine-1-carboxylate (compound **I-5**)

181.63 mg (0.63 mmol) of **13-4** and 287.5 mg (0.95 mmol) of compound **13-7** were weighed and placed in a 100 mL round-bottom flask, added with 10 mL of anhydrous methanol, and stirred until some of the solid was dissolved. 0.5 mL of glacial acetic acid was added dropwise as a catalyst, and the mixture was heated to 50 °C and reacted overnight. On the next day, the solid in the reaction flask was completely dissolved. Heating was stopped, and after the mixture was cooled to room temperature, 79.18 mg (1.26 mmol) of sodium cyanoborohydride was added. After the mixture was stirred for 8 h, the TLC detection showed that the reaction was completed. The reaction solution was concentrated, and 20 mL of saturated sodium bicarbonate solution was added to the residue. Bubbles were released, and a solid was produced. The mixture was then extracted with dichloromethane (3 × 10 mL). The organic phases were combined and dried over anhydrous sodium sulfate. After filtration, the filtrate was concentrated and separated by silica gel column chromatography (elution: $V_{\text{dichloromethane}}/V_{\text{methanol}}$ = 50/1-10/1) to obtain 70 mg of compound **I-5** as a white solid, with a yield of 19.30%. ESI-HRMS m/z: 577.3134 $[M+H]^+$; $^1H$-NMR (600 MHz, DMSO-$d_6$) δppm: 10.91 (s, 1H), 7.32 (d, *J* = 7.8 Hz, 2H), 7.25 (d, *J* = 8.1 Hz, 3H), 7.15-7.08 (m, 2H), 6.69 (dd, *J* = 7.5, 1.9 Hz, 1H), 6.04 (t, *J* = 5.9 Hz, 1H), 5.13 (dd, *J* = 12.8, 5.3 Hz, 1H), 4.36 (d, *J* = 5.9 Hz, 2H), 4.30 (d, *J* = 16.3 Hz, 1H), 4.12 (d, *J* = 16.3 Hz, 1H), 3.44 (s, 2H), 3.30 (s, 4H), 2.91-2.79 (m, 1H), 2.61 (s, 1H), 2.47-2.42 (m, 1H), 2.30 (s, 5H), 2.12-2.04 (m, 1H), 1.40 (d, *J* = 1.6 Hz, 9H). $^{13}C$-NMR (151 MHz, DMSO-$d_6$) δppm: 173.34, 171.65, 163.58, 154.25, 145.39, 139.06, 133.65, 129.44, 128.18, 128.04, 127.52, 127.29, 126.83, 121.10, 114.99, 114.72, 79.22, 63.19, 62.14, 58.01, 55.38, 52.78, 51.40, 46.67, 44.34, 31.39, 28.52 (3C), 28.40, 22.62.

Example 14: Synthesis of 3-(3-methyl-5-((4-(morpholinomethyl)benzyl)amino)-1-oxo-3,4-dihydrophthalazin-2(1*H*)-yl) piperidin-2,6-dione (compound **I-1**)

The synthesis method referred to Example 13. The product **I-1** was a white solid (130 mg, yield 33.10%). ESI-HRMS m/z: 478.2449 $[M+H]^+$; $^1H$-NMR (600 MHz, DMSO-$d_6$) δppm: 10.92 (s, 1H), 7.30 (d, *J* = 8.0 Hz, 2H), 7.24 (d, *J* = 8.1 Hz, 2H), 7.13-7.07 (m, 2H), 6.67 (dd, *J* = 7.9, 1.5 Hz, 1H), 6.04 (t, *J* = 6.0 Hz, 1H), 5.12 (dd, *J* = 13.0, 5.0 Hz, 1H), 4.34 (d, *J* = 6.0 Hz, 2H), 4.28 (d, *J* = 16.2 Hz, 1H), 4.10 (d, *J* = 16.2 Hz, 1H), 3.55 (t, *J* = 4.6 Hz, 4H), 3.41 (s, 2H), 2.84 (ddd, *J* = 17.1, 13.8, 5.4 Hz, 1H), 2.57 (dd, *J* = 16.9, 3.7 Hz, 1H), 2.49 (s, 3H), 2.43 (dd, *J* = 13.3, 4.4 Hz, 1H), 2.32 (s, 4H), 2.07 (dtd, *J* = 12.7, 5.2, 2.6 Hz, 1H). $^{13}C$-NMR (151 MHz, DMSO-$d_6$) δppm: 173.35, 171.66, 163.59, 145.38, 139.00, 136.62, 129.42 (2C), 128.18, 128.04, 127.26 (2C), 121.10, 114.99, 114.73, 66.64, 62.66, 58.01, 55.38, 53.63 (2C), 51.40, 46.68, 44.34, 31.39, 22.62.

Example 15: Synthesis of 3-(3-methyl-1-oxo-5-((4-((4-phenylpiperazin-1-yl)methyl)benzyl) amino)-3,4-dihydrophthalazin-2(1*H*)-yl)piperidin-2,6-dione (compound **I-3**)

The synthesis method referred to Example 13. The product **I-3** was a white solid (90 mg, yield 40.65%). ESI-HRMS m/z: 553.2922 $[M+H]^+$; $^1H$-NMR (600 MHz, DMSO-$d_6$) δppm: 10.92 (s, 1H), 7.32 (d, *J* = 7.8 Hz, 2H), 7.27 (d, *J* = 8.1 Hz, 2H), 7.19 (dd, *J* = 8.7, 7.2 Hz, 2H). 7.14-7.08 (m, 2H), 6.92-6.88 (m, 2H), 6.78-6.74 (m, 1H), 6.68 (dd, *J* = 8.0, 1.4 Hz, 1H), 6.05 (t, *J* = *6.0* Hz, 1H), 5.13 (dd, *J* = 12.7, 5.0 Hz, 1H), 4.36 (d, *J* = 5.9 Hz, 2H), 4.29 (d, *J* = 16.3 Hz, 1H), 4.14-4.09 (m, 1H), 3.48 (s, 2H), 3.17 (d, *J* = 5.2 Hz, 1H), 3.11 (t, *J* = 5.0 Hz, 4H), 2.84 (ddd, *J* = 17.0, 13.7, 5.3 Hz, 1H), 2.57 (dd, *J* = 16.7, 3.5 Hz, 1H), 2.53-2.52 (m, 1H), 2.47 (s, 3H), 2.46-2.39 (m, 1H), 2.07 (dqd, *J* = 12.6, 5.0, 2.7 Hz, 1H), 1.24 (s, 2H). $^{13}C$-NMR (151 MHz, DMSO-$d_6$) δppm: 173.35, 171.66, 163.59, 151.49, 145.40, 139.01, 136.89, 129.44, 129.36 (2C), 128.19, 128.06, 127.30 (2C), 126.85, 121.11, 119.23, 115.83 (2C), 114.99, 114.74, 62.27, 58.02, 53.01 (2C), 51.41, 48.64 (2C), 46.70, 44.35, 31.39, 22.62, 22.57.

Example 16: Synthesis of 3-(5-((4-(4-(4-fluorophenyl)piperazin-1-yl)methyl)benzyl) amino)-3-methyl-1-oxo-3,4-dihydrophthalazin-2(1*H*)-yl)piperidin-2,6-dione (compound **I-6**)

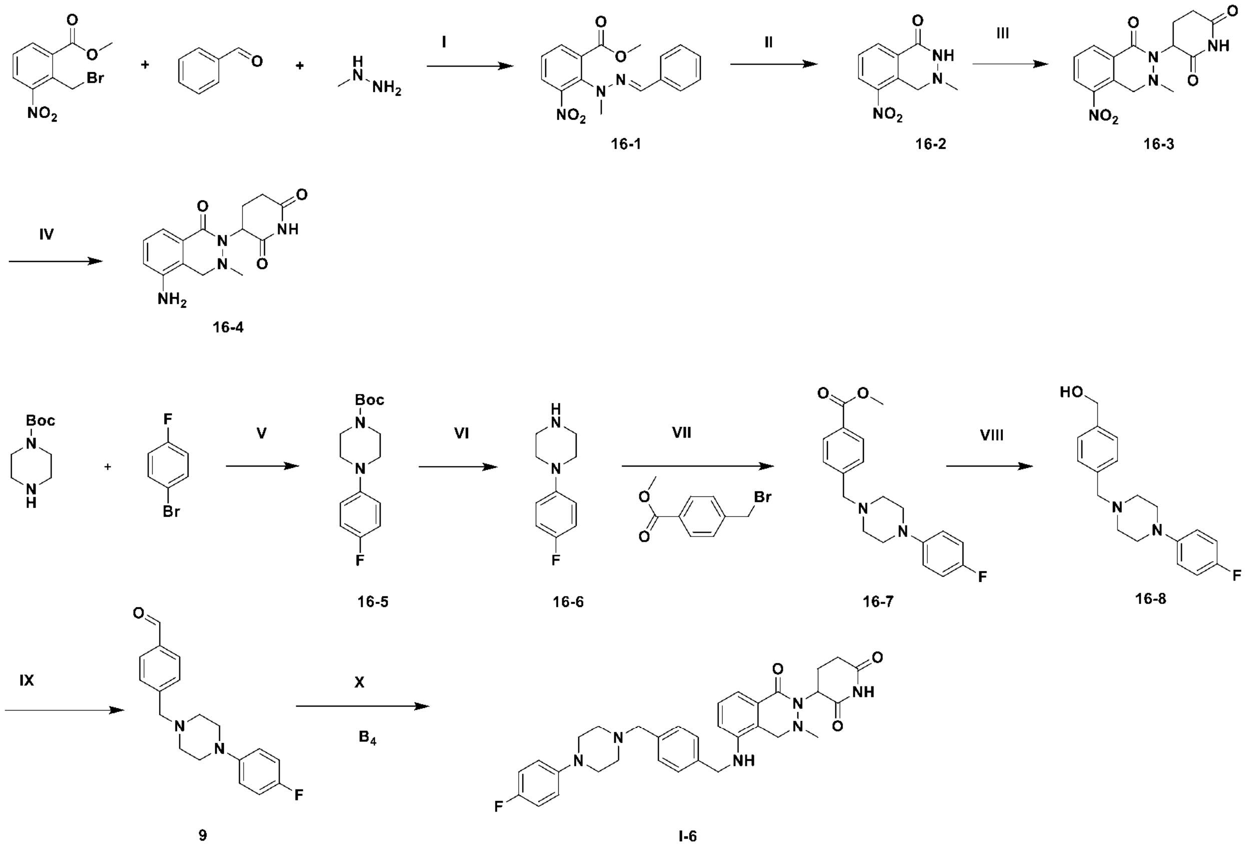

16-1 16-2 16-3 16-4 16-5 16-6 16-7 16-8 9 I-6

[16-1] Synthesis of methyl (*E*)-2-((2-benzylidene-1-methylhydrazineyl)methyl)-3-nitrobenzoate (compound <**16-1**>)

10.50 g (72.84 mmol) of methylhydrazine sulfate and 11.60 g (109.26 mmol) of anhydrous sodium carbonate were weighed and suspended in 200 mL of methanol. The mixture was stirred and heated to 60 °C. One hour later, 7.29 mL (72.84 mmol) of benzaldehyde and 20.00 g (72.84 mmol) of methyl 2-bromomethyl-3-nitrobenzoate were added to the reaction system, and the mixture was stirred at 60 °C for another 3 hours. After the TLC detection showed that the reaction of methyl 2-bromomethyl-3-nitrobenzoate was completed, the solid was removed by filtration. The filtrate was concentrated and purified by silica gel column chromatography (eluent: $V_{\text{petroleum ether}}/V_{\text{ethyl acetate}}$ = 15/1) to give 17.0 g of compound **16-1** as an orange-yellow solid, with a yield of 71.1%. ESI-MS m/z: 328.12 $[M+H]^+$.

[16-2] Synthesis of 3-methyl-5-nitro-3,4-dihydrophthalazin-1(2*H*)-one (compound <**16-2**>):

17.0 g of compound **16-1** (52.00 mmol) was weighed and added into 50 mL of a mixed solvent ($V_{\text{n-brutanol}}/V_{\text{glacial acetic acid}}$ = 1/1), and stirred until it was completely dissolved. 354 mg (2.60 mmol) of anhydrous zinc chloride was added. The mixture was heated to 120 °C and reacted for 6 hours. Heating and stirring were stopped, and the mixture was allowed to stand overnight. A yellow solid precipitated and was filtered. The obtained solid was washed with petroleum ether, and dried to give compound **16-2** (5.90 g, yield 54.8%). ESI-HRMS m/z: 206.0560 $[M+H]^+$.

[16-3] Synthesis of 3-(3-methyl-5-nitro-1-oxo-3,4-dihydrophthalazin-2(1*H*)-yl)piperidin-2,6-dione (compound <**16-3**>)

5.00 g of compound **16-2** (24.14 mmol) was weighed and dissolved in 50 mL of anhydrous tetrahydrofuran under nitrogen protection. The mixture was stirred in an ice bath and 36 mL of LiHMDs (36.00 mmol, 1 N in THF) was added. After the addition was completed, the mixture was stirred in an ice bath for 30 minutes. Then, 6.95 g (36.22 mmol) of 3-bromopiperidin-2,6-dione dissolved in 20 mL of anhydrous tetrahydrofuran was added. The mixture was allowed to warm naturally to room temperature and reacted for 3 hours. After TLC detection showed that the reaction ceased to proceed, the reaction was quenched by adding saturated ammonium chloride solution. After layer separation, the aqueous phase was

extracted with ethyl acetate (3 × 10 mL). The organic phases were combined, dried over anhydrous sodium sulfate, and filtered, and the filtrate was concentrated and purified by silica gel column chromatography (eluent: $V_{dichloromethane}/V_{methanol}$ = 50/1) to give 4.01 g of compound **16-3** as a white to slightly yellow solid, with a yield of 52.10%. ESI-HRMS m/z: 318.3001 [M]+. $^1$H-NMR (600 MHz, DMSO-$d_6$) δppm: 11.00 (s, 1H), 8.36 (dd, *J* = 8.2, 1.3 Hz, 1H), 8.26 (dd, *J* = 7.7, 1.3 Hz, 1H), 7.74 (t, *J* = 8.0 Hz, 1H), 5.20 (dd, *J* = 12.8, 5.1 Hz, 1H), 4.68 (d, *J* = 17.2 Hz, 1H), 4.51 (d, *J* = 18.5 Hz, 1H), 2.86 (ddd, *J* = 17.2, 13.8, 5.4 Hz, 1H), 2.60 (t, *J* = 4.0 Hz, 1H), 2.57 (d, *J* = 3.7 Hz, 2H), 2.45 (d, *J* = 10.2 Hz, 1H), 2.43-2.38 (m, 1H), 2.12 (ddq, *J* = 10.5, 5.3, 2.6 Hz, 1H).

[16-4] Synthesis of 3-(5-amino-3-methyl-1-oxo-3,4-dihydrophthalazin-2(1*H*)-yl)piperidin-2,6-dione (compound <**16-4**>)

4.00 g (12.58 mmol) of compound **16-3** was weighed and added to 40 mL of methanol, and stirred until some of the solid was dissolved. Approximately 133.86 mg (1.26 mmol) of 5% Pd-C was added. The reactor was then purged with hydrogen gas and connected to a hydrogen-filled balloon. The reaction was allowed to proceed overnight at room temperature. The solid was completely dissolved on the next day. TLC detection showed that the reaction was completed. The wet palladium-on-carbon catalyst was removed and recovered by filtration with diatomaceous earth. The filtrate was concentrated and purified by silica gel column chromatography (eluent: $V_{dichloromethane}/V_{methanol}$ = 50/1-10/1) to give 1.21 g of compound **16-4** as a white solid, with a yield of 33.34%. ESI-HRMS m/z: 289.1269 $[M+H]^+$; $^1$H-NMR (600 MHz, DMSO-$d_6$) δppm: 10.90 (s, 1H), 7.13-7.06 (m, 2H), 6.86 (dd, *J* = 7.6, 1.5 Hz, 1H), 5.19 (s, 2H), 5.11 (dd, *J* = 12.7, 5.1 Hz, 1H), 4.18 (d, *J* = 16.2 Hz, 1H), 3.97 (d, *J* = 16.2 Hz, 1H), 2.83 (ddd, *J* = 17.1, 13.8, 5.3 Hz, 1H), 2.58-2.53 (m, 1H), 2.47 (s, 3H), 2.45-2.37 (m, 1H), 2.05 (dtd, *J* = 12.8, 5.2, 2.6 Hz, 1H).

[16-5] Synthesis of tert-butyl 4-(4-fluorophenyl)piperazine-1-carboxylate (compound <**16-5**>)

2.00 g (11.43 mmol) of p-bromofluorobenzene, 3.14 g (13.71 mmol) of 1-tert-butyloxycarbonylpiperazine, 0.52 g (0.57 mmol) of $Pd_2(dba)_3$ and 0.82 g (1.72 mmol) of X-Phos were weighed and placed in a three-necked flask under nitrogen protection. 20 mL of anhydrous dioxane and 17.2 mL of $NaO^tBu$ (1 N in THF) were added, and the mixture was heated to 80 °C and reacted for 2 hours. TLC detection showed the reaction was completed. Heating was stopped, and the mixture was filtered through diatomaceous earth. The filtrate was washed with water. After layer separation, the aqueous phase was extracted with ethyl acetate (2 × 10 mL). The organic phases were combined and dried over anhydrous sodium sulfate, and filtered, and the filtrate was concentrated and separated by silica gel column chromatography (eluent: $V_{petroleum\ ether}/V_{ethyl\ acetate}$ = 20/1) to give 2.8 g of intermediate **16-5** as a yellow oily liquid, with a yield of 87.50%. ESI-MS m/z: 281.17 $[M+H]^+$; $^1$H-NMR (600 MHz, DMSO-$d_6$) δppm: 7.05 (dd, *J* = 9.8, 7.9 Hz, 2H), 6.98-6.92 (m, 2H), 3.46 (t, *J* = 5.1 Hz, 4H), 3.05-2.98 (m, 4H), 1.42 (s, 9H).

[16-6] Synthesis of 1-(4-fluorophenyl)piperazine (compound <**16-6**>)

2.80 g (9.99 mmol) of compound **16-5** was weighed and dissolved in 10 mL of dichloromethane, added with 10 mL of trifluoroacetic acid, stirred, and reacted at room temperature for 1 hour. TLC detection showed that the reaction was completed. The reaction solution was evaporated to dryness, and the residue was washed with saturated sodium bicarbonate and extracted with dichloromethane (2 × 10 mL). The organic phases were combined and dried over anhydrous sodium sulfate. After filtration, the filtrate was evaporated to dryness to obtain 1.93 g of crude product **16-6,** with a yield of 107.34%, which was used directly in the next reaction without purification. ESI-MS m/z: 181.12 $[M+H]^+$; $^1$H-NMR (600 MHz, DMSO-$d_6$) δppm: 7.06-7.01 (m, 2H), 6.94-6.89 (m, 2H), 2.98 (s, 1H), 2.97 (d, *J* = 5.2 Hz, 4H), 2.84 (dd, *J* = 6.2, 3.8 Hz, 4H).

[16-7] Synthesis of 3-(5-((4-(4-(4-fluorophenyl)piperazin-1-yl)methyl)benzyl)amino)-3-methyl-1-oxo-3,4-dihydrophthalazin-2(1*H*)-yl)piperidin-2,6-dione (compound **I-6**)

The process for synthesizing compound **I-6** from intermediate **16-6** was the same as that for synthesizing **I-5** from starting material 1-tert-butyloxycarbonylpiperazine in Example 13. The product **I-6** was a white solid (110 mg, yield 30.21%). ESI-HRMS m/z: 571.2827 $[M+H]^+$; $^1$H-NMR (600 MHz, $CDCl_3$)δppm: 8.18 (s, 1H), 7.47 (dd, *J* = 7.8, 1.1 Hz, 1H), 7.36 (d, *J* = 7.7 Hz, 2H), 7.32 (d, *J* = 8.1 Hz, 2H), 7.27 (d, *J* = 7.9 Hz, 1H), 6.98-6.93 (m, 2H), 6.89-6.84 (m, 3H), 5.23 (dd, *J* = 12.4, 5.0 Hz, 1H), 4.35 (s, 3H), 3.91-3.82 (m, 1H), 3.60 (s, 2H), 3.14 (t, *J* = 4.9 Hz, 4H), 2.90-2.82 (m, 1H), 2.71 (ddd, *J* = 17.2, 13.6, 5.1 Hz, 2H), 2.63 (d, *J* = 16.8 Hz, 8H), 2.20 (dtd, *J* = 12.9, 4.9, 2.8 Hz, 1H). $^{13}$C-NMR (151 MHz, DMSO-$d_6$) δppm: 173.35, 171.66, 163.59, 157.21-155.65, 148.40, 145.40, 139.00, 136.88, 129.44 (2C), 128.19, 128.06, 127.30 (2C), 121.11, 117.57, 117.52, 115.76, 115.62, 115.00, 114.74, 62.22, 58.01, 52.99 (2C), 51.40, 49.43 (2C), 46.70, 44.34, 31.39, 22.62.

Example 17: Synthesis of 3-(5-((4-(4-(4-cyanophenyl)piperazin-1-yl)methyl)benzyl) amino)-3-methyl-1-oxo-3,4-dihydrophthalazin-2(1*H*)-yl)piperidin-2,6-dione (compound **I-7**)

The synthesis method referred to Example 16, except that p-bromofluorobenzene in [16-5] was replaced with 4-bromobenzonitrile. The resulting product **I-7** was a white solid (80 mg, yield 26.09%). ESI-HRMS m/z: 578.2874 $[M+H]^+$; $^1$H-NMR (400 MHz, DMSO-$d_6$) δppm: 10.94 (s, 1H), 7.58 (d, $J$ = 8.9 Hz, 2H), 7.34 (d, $J$ = 7.9 Hz, 2H). 7.29 (d, $J$ = 7.9 Hz, 2H), 7.16-7.08 (m, 2H), 7.04-6.96 (m, 2H), 6.70 (dd, $J$ = 7.0, 2.4 Hz, 1H), 6.07 (t, $J$ = 6.0 Hz, 1H), 5.15 (dd, $J$ = 12.6, 5.1 Hz, 1H), 4.37 (d, $J$ = 5.9 Hz, 2H), 4.31 (d, $J$ = 16.2 Hz, 1H), 4.13 (d, $J$ = 16.4 Hz, 1H), 3.51 (s, 2H), 3.32 (s, 4H), 2.91-2.79 (m, 1H), 2.61 (t, $J$ = $^{13}$C-NMR (151 MHz, DMSO-$d_6$) δppm: 173.34, 171.66, 163.60, 153.59, 151.35, 149.70, 145.40, 133.77, 129.63, 129.38, 128.20, 128.05, 127.55, 127.34, 121.13, 120.54, 119.34, 116.40, 115.88, 115.01, 114.73, 114.51, 58.02, 55.38, 51.43, 46.70, 44.34, 40.42, 40.28, 40.14, 40.00, 39.86, 39.72, 39.58, 34.19, 31.40, 29.50, 24.99, 22.63, 14.43.

Example 18: Synthesis of 3-(5-((4-(4-(3-fluoro-4-cyanophenyl)piperazin-1-yl)methyl) benzyl)amino)-3-methyl-1-oxo-3,4-dihydrophthalazin-2(1*H*)-yl)piperidin-2,6-dione (compound **I-8**)

The synthesis method referred to Example 16, except that p-bromofluorobenzene in [16-5] was replaced with 2-fluoro-4-bromobenzonitrile. The resulting product **I-8** was a white solid (130 mg, yield 32.55%). ESI-HRMS m/z: 596.2780 $[M+H]^+$; $^1$H-NMR (600 MHz, DMSO-$d_6$) δppm: 10.92 (s, 1H), 7.58 (t, $J$ = 8.5 Hz, 1H), 7.32 (d, $J$ = 7.9 Hz). Hz, 2H), 7.26 (d, $J$ = 7.9 Hz, 2H), 7.14-7.08 (m, 2H), 6.92 (dd, $J$ = 14.2, 2.4 Hz, 1H), 6.83 (dd, $J$ = 9.0, 2.4 Hz, 1H), 6.68 (dd, $J$ = 7.7, 1.6 Hz, 1H), 6.05 (t, $J$ = 6.0 Hz, 1H), 5.13 (dd, $J$ = 12.7, 5.1 Hz, 1H), 4.36 (d, $J$ = 5.9 Hz, 2H), 4.29 (d, $J$ = 16.2 Hz, 1H), 4.11 (d, $J$ = 16.3 Hz, 1H), 3.48 (s, 2H), 3.36 (t, $J$ = 5.0 Hz, 4H), 2.84 (ddd, $J$ = 17.0, 13.8, 5.3 Hz, 1H), 2.57 (dt, $J$ = 17.1, 3.7 Hz, 1H), 2.44 (t, $J$ = 5.4 Hz, 5H), 2.07 (dtd, $J$ = 12.8, 5.2, 2.6 Hz, 1H). $^{13}$C-NMR (151 MHz, DMSO-$d_6$) δppm: 173.35, 171.66, 165.53-163.88, 163.59, 155.67, 155.60, 145.38, 139.12, 134.42, 134.40, 129.46, 128.19, 128.04, 127.32, 121.11, 115.89, 115.00, 114.74, 110.54, 100.69, 100.53, 86.48, 86.38, 61.99, 58.02, 55.38, 52.42, 51.40, 46.72, 46.69, 44.35, 31.39, 22.62.

Example 19: Evaluation of activity of compounds against proliferation of hematological tumor cells

The cell models used in this example were human hematologic tumor cells, including NCI-H929, RPMI-8226, MV-4-11, U937 and WSU-DLCL-2. Some of the compounds of the present invention were selected to determine their inhibitory activity against the proliferation of NCI-H929, RPMI-8226, MV-4-11, U937, and WSU-DLCL-2 cells.

1. Experimental materials and instruments

1.1 Experimental materials and instruments

| Materials and Reagents | Vendor | Cat# |
|---|---|---|
| NCI-H929 | Pricella | CL-0509 |
| RPMI-8226 | Pricella | CL-0564 |
| MV-4-11 | Pricella | CL-0572 |
| U937 | Pricella | CL-0239 |
| WSU-DLCL-2 | Pricella | CL-0774 |
| CCK-8 | Dojindo | CK04 |
| RPMI- 1640 | GIBCO | C11875500BT |
| Fetal Bovine Serum (FBS) | Hyclone | SH30406.05 |
| DMSO | Sigma | D8418 |
| Consumables and Instrument | Vendor | Cat# |
| 96-well polypropylene plate | Corning | 3599 |
| Plate shaker | Thermo | 4625-1 CECN/THZ Q |
| Centrifuge | Eppendorf | 5810R |
| Envision 2104 multi-label Reader | PerkinElmer | 74785 |
| Echo | Labcyte | 550 |

2. Experimental procedure

(1) Preparation of solutions

① The selected compounds were dissolved in DMSO to prepare 10 mM stock solutions. Compounds used within three months should be stored at room temperature in a desiccator; others could be stored at -20°C for long-term storage.
② The above stock solutions and the positive reference compound pomalidomide were diluted with DMSO. Their initial concentrations were 10 μM. In the experiments of NCI-H929 and RPMI-8226 cells, 3-fold serial dilution with 11 concentration points was performed, with the lowest concentration of 0.00047 μM. The resulting solutions were shaken on a shaker for 5 minutes.

(2) Applying drugs to cells

① The NCI-H929, RPMI-8226, MV-4-11, U937, and WSU-DLCL-2 cells in logarithmic growth phase were inoculated in a 96-well plate, 20,000 to 30,000 cells per well, and the plate was pre-cultured for 24 hours in an incubator (at 37°C and 5% $CO_2$).

② The culture medium in the plate was replaced, and the compounds and the positive control with corresponding concentrations were added to the plated at a volume of 50 μl.

③ The plate was incubated for 144 hours in an incubator, then 15 μl of CCK-8 solution was added to each well, and incubated in the incubator for another 1-4 hours.

④ The absorbance at 450 nm was measured using a microplate reader.

(3) Data processing

① Calculating % Inhibition:

% Inhibition = 100 - (Signalcmpd - SignalAve_PC) / (SignalAve_VC - SignalAve_PC) × 100.

② Calculating $IC_{50}$ value of compound and plotting dose-response curve:
The $IC_{50}$ value was calculated by fitting the % Inhibition and the logarithm of compound concentration to a nonlinear regression (dose response-variable slope) by using GraphPad 6.0.

$$Y = Bottom + (Top - Bottom) / (1 + 10^{\wedge}((LogIC_{50} - X) * HillSlope))$$

X: log of inhibitor concentration; Y: % Inhibition.

3. Experimental results

After calculating % Inhibition, a dose-response curve was plotted with the logarithm of the test compound concentration as the x-axis and the average cell viability as the y-axis, and the $IC_{50}$ value was obtained by fitting.
Table 1 shows the test results of the above-mentioned compounds and the positive reference compound pomalidomide on NCI-H929, RPMI-8226, MV-4-11, U937, and WSU-DLCL-2 cells. The experimental results indicate that, including pomalidomide, the compounds all exhibited good inhibitory effects on the proliferation of NCI-H929, RPMI-8226, MV-4-11, U937, and WSU-DLCL-2 cells. Among them, **I-5, 1-6, I-7,** and **I-8** showed significantly better inhibitory effects than the control drug pomalidomide on these cells.

Table 1. Antiproliferative activity of compounds against NCI-H929, RPMI-8226, MV-4-11, U937 and WSU-DLCL-2 cells ($IC_{50}$ values)

| 编号 | $IC_{50}$ (μM) | | | | |
|---|---|---|---|---|---|
| | H-929 | RPMI-8226 | MV-4-11 | U937 | WSU-DLCL-2 |
| Pomalidomide | 3.905 | 0.729 | >10 | 2.53 | >10 |
| I-1 | ND | ND | 3.43 | 1.32 | 0.358 |
| I-2 | 0.311 | 8.31 | >10 | 1.33 | 1.06 |
| I-3 | 1.300 | ND | 1.39 | 1.11 | 0.279 |
| I-4 | 0.116 | ND | 0.183 | 0.31 | 1.21 |
| I-5 | 0.243 | 0.641 | 0.174 | 0.666 | 0.108 |
| I-6 | 0.032 | 1.26 | 0.0311 | 0.0522 | 0.048 |
| I-7 | 0.028 | 0.442 | 0.0155 | 0.0348 | 0.053 |
| I-8 | 0.0039 | 0.308 | 0.0058 | 0.0088 | 0.017 |
| I-9 | >10 | 0.457 | 0.390 | >10 | 0.230 |
| I-10 | 5.85 | 0.380 | 0.215 | >10 | 0.70 |
| I-11 | 2.54 | 2.97 | 0.294 | 4.13 | 2.65 |
| I-12 | >10 | 0.499 | 0.874 | 2.30 | 3.69 |
| 1-13 | 0.850 | 0.204 | >10 | 1.11 | 0.88 |

ND: Not detected. The $IC_{50}$ value for compounds with an inhibition rate <50% at 10 μM concentration was not obtained by fitting.

Example 20: In vivo activity of compounds against human multiple myeloma RPMI-8226 transplanted tumor

In this example, human multiple myeloma RPMI-8226 cell line was used as the cell model. The inhibitory effects of compounds on the growth of NOD/SCID mouse transplanted tumors of RPMI-8226 tumor cells were determined. The evaluation methods and results are described below.

1. Experimental materials and instruments

1.1 Experimental consumables for bioactivity evaluation

| Materials and Reagents | Vendor | Cat# |
|---|---|---|
| RPMI-8226 | Pricella | CL-0564 |
| RPMI1640 | GIBCO | C11875500BT |
| Fetal bovine serum (FBS) | Hyclone | SH30406.05 |
| DMSO | Sigma | D8418 |
| NOD/SCID mice | SiPeiFu | -- |
| **Consumables and instrument** | **Vendor** | **Cat#** |
| T75 cell culture flask | Corning | 430641 |

2. Establishment of tumor model

Human multiple myeloma RPMI-8226 cells were routinely cultured in RPMI1640 containing 10% fetal bovine serum at 37°C in a 5% $CO_2$ incubator. After three passages in vitro, when the cells reached over 80% confluence and the required confluence rate, the cells were digested and harvested. After washing with PBS, the cells were counted and the

cell concentration was adjusted to approximately $5 \times 10^7$ cells/mL. The cells were then placed in 4 mL centrifuge tubes on ice for later use.

Female NOD/SCID mice aged 4-5 weeks were selected and subcutaneously inoculated with RPMI-8226 cells ectopically. The mice were held in a lateral position, the forelimb axilla was disinfected with 75% alcohol, and 100 μL of the cell suspension was injected subcutaneously into the axilla using a 1 mL syringe ($1.5 \times 10^7$ cells/mouse/100 μL).

3. Animal grouping and administration

Once the tumors grew to 90-150 $mm^3$, the animals were randomly grouped, 8 mice per group, and administered according to different administration methods as follows:

Model control group: administered with the same volume of Vehicle (DMSO: 0.5% sodium carboxymethyl cellulose: distilled water = 1:1:8) daily by gavage;

Test groups: administered with compound **I-4** and compound **I-8** solutions at 10 mg/kg (mouse body weight) daily by gavage, respectively;

Positive control group: administered with pomalidomide solution at 10 mg/kg (mouse body weight) daily by gavage.

The route of administration was oral gavage, and the administration frequency was once daily, for 12 consecutive days. The first day of administration was defined as Day 1 of the experiment. Tumor volume changes in mice were measured and recorded every two days. Tumor volume was measured using a vernier caliper, in which the major axis (a) and minor axis (b) of the tumor were measured, and the tumor volume was calculated as: tumor volume V ($mm^3$) = a x b^2/2. After the experiment, the mice were dissected and the tumor weight was measured. The data were entered and statistically analyzed using GraphPad Prism 6 software. The data are expressed as mean ± SEM (Standard Error of Mean), and one-way ANOVA was used. $P \leq 0.05$ was considered statistically significant.

4. Experimental results

As shown in Figures 1A and 1B and Table 2, Figure 1A shows the changes in mouse tumor volume on Days 0, 2, 4, 6, 8, 10, and 12, and Figure B shows the dissected tumors from each group after the experiment. Experimental results show that compounds **I-4** and **I-8** (10 mg/kg, QD) significantly inhibit the growth of RPMI-8226 transplanted tumors. At the same dose, **1-4** and **I-8** show better inhibitory effects on tumor growth than the positive control drug pomalidomide. As shown in Figure 1C, no significant changes in mouse body weight occurred in any group during the experiment, and no obvious toxic side effects were observed.

Table 2. Inhibitory effects of compounds **I-4** and **I-8** on human multiple myeloma RPMI-8226 xenograft tumors

| Group | Control | Pomalidomide (10 mg/kg, QD) | **I-4** (10 mg/kg, QD) | **I-8** (10 mg/kg, QD) |
|---|---|---|---|---|
| Volume ($mm^3$), 0 days | 202.3 | 180.0 | 189.0 | 257.5 |
| Volume ($mm^3$), 12 days | 1524.8 | 870.4 | 541.1 | 520.3 |
| Tumor inhibition rate (TGI%) | -- | 47.80% | 73.38% | 80.13% |

Example 21: In vivo antitumor activity of compounds against human multiple myeloma NCI-H929 transplanted tumors

In this example, the human multiple myeloma NCI-H929 cell line was used as the cell model. The inhibitory effects of compounds on the growth of NOD/SCID mouse transplanted tumors of NCI-H929 tumor cells were determined. The evaluation methods and results are described below.

1. Experimental materials and instruments

1.1 Experimental consumables for bioactivity evaluation

| Materials and Reagents | Vendor | Cat# |
|---|---|---|
| NCI-H929 | Pricella | CL-0509 |
| RPMI1640 | GIBCO | C11875500BT |
| Fetal bovine serum (FBS) | Hyclone | SH30406.05 |
| DMSO | Sigma | D8418 |
| NOD/SCID mice | SiPeiFu | - |
| Consumables and instrument | Vendor | Cat# |
| T75 cell culture flask | Corning | 430641 |

2. Establishment of tumor model

Human multiple myeloma NCI-H929 cells were routinely cultured in RPMI1640 containing 10% fetal bovine serum at 37°C in a 5% $CO_2$ incubator. After three passages in vitro, when the cells reached over 80% confluence and the required confluence rate, the cells were digested and harvested. After washing with PBS, the cells were counted and the cell concentration was adjusted to approximately $5 \times 10^7$ cells/mL. The cells were then placed in 4 mL centrifuge tubes on ice for later use.

Female NOD/SCID mice aged 4-5 weeks were selected and subcutaneously inoculated with NCI-H929 cells ectopically. The mice were held in a lateral position, the forelimb axilla was disinfected with 75% alcohol, and 100 μL of the cell suspension was injected subcutaneously into the axilla using a 1 mL syringe ($1.5 \times 10^7$ cells/mouse/100 μL).

3. Animal grouping and administration

After the tumors grew to 90-150 $mm^3$, the animals were randomly divided into groups, 8 mice in each group, and administered according to different administration methods as follows:

Model control group: Administered with the same volume of Vehicle (DMSO: 0.5% sodium carboxymethyl cellulose: distilled water = 1:1:8) daily by gavage;

Control drug group 1: administered with pomalidomide solution at 10 mg/kg (mouse body weight) daily by gavage;

Control drug group 2: administered with CC-220 solution at 3 mg/kg (mouse body weight) daily by gavage;

Control drug group 3: administered with CC-220 solution at 10 mg/kg (mouse body weight) daily by gavage;

Experimental compound group 4: administered with compound **I-4** solution at 3 mg/kg (mouse body weight) daily by gavage;

Experimental compound group 5: administered with compound **I-4** solution at 10 mg/kg (mouse body weight) daily by gavage;

Experimental compound group 6: administered with compound **I-8** solution at 3 mg/kg (mouse body weight) daily by gavage;

Experimental compound group 7: administered with compound **I-8** solution at 10 mg/kg (mouse body weight) daily by gavage;

The route of administration was oral gavage, and the administration frequency was once daily, for 10 consecutive days. The first day of administration was defined as Day 1 of the experiment. Tumor volume changes in mice were measured and recorded every two days. Tumor volume was measured using a vernier caliper, in which the major axis (a) and minor axis (b) of the tumor were measured, and the tumor volume was calculated as tumor volume V ($mm^3$) = a x b^2/2. After the experiment, the mice were dissected and the tumor weight was measured. The data were entered and statistically analyzed using GraphPad Prism 6 software. The data are expressed as mean ± SEM (Standard Error of Mean), and one-way ANOVA was used. $P \leq 0.05$ was considered statistically significant.

4. Experimental results

As shown in Figure 2A and Table 3, the experimental results indicate that compounds 1-4 and **I-8** (3 mg/kg and 10 mg/kg, QD) significantly inhibited the growth of NCI-H929 transplanted tumors. At the same dose, **I-4** and **I-8** (10 mg/kg, QD) show significantly better inhibitory effects on tumor growth than the control drug pomalidomide (Pom, 10 mg/kg, QD). At the same dose, **I-8** (3 mg/kg, QD) is more effective than the control drug CC-220 (3 mg/kg, QD). As shown in Figure 2B, during the experiment, the CC-220 (10 mg/kg, QD) group showed a decreasing trend in body weight, while other groups did not show a significant decreasing trend in body weight, and no obvious toxic side effects were observed.

Table 3. Inhibitory effects of compounds **I-4** and **I-8** on human multiple myeloma NCI-H929 xenograft tumors

| Compounds | Initial volume ($mm^3$) | Average volume ($mm^3$) (Day 10) | Tumor inhibition rate TGI (%) | *p* value (vs Vehicle) | *p* value (vs Pom) |
|---|---|---|---|---|---|
| Control | 143.8 | 2090.3 | - | - | - |
| Pomalidomide (10 mg/kg, QD) | 185.6 | 573.1 | 80.09% | <0.01 | - |
| CC-220 (3 mg/kg, QD) | 214.6 | 643.1 | 77.98% | <0.01 | ns |
| CC-220 (10 mg/kg, QD) | 207.2 | 205.5 | 100.09% | <0.01 | <0.01 |
| **I-4** (3 mg/kg, QD) | 191.6 | 843.1 | 66.53% | <0.01 | <0.01 |
| **I-4** (10 mg/kg, QD) | 204.8 | 344.5 | 92.82% | <0.01 | <0.01 |
| **I-8** (3 mg/kg, QD) | 193.5 | 500.1 | 84.25% | <0.01 | ns |
| **I-8** (10 mg/kg, QD) | 211.3 | 197.3 | 100.72% | <0.01 | <0.01 |

Example 22: In vivo activity of compound in combination with dexamethasone against human multiple myeloma NCI-H929 transplanted tumor

In this example, human multiple myeloma NCI-H929 cell line was selected as the cell model to determine the inhibitory effects of compound and dexamethasone alone, as well as combination thereof on the growth of NOD/SCID mouse transplanted tumors of NCI-H929 tumor cells. The evaluation methods and results are described below.

1. Experimental materials and instruments

1.1 Experimental consumables for bioactivity evaluation

| Materials and Reagents | Vendor | Cat# |
|---|---|---|
| NCI-H929 | Pricella | CL-0509 |
| RPMI1640 | GIBCO | C11875500BT |
| Fetal bovine serum (FBS) | Hyclone | SH30406.05 |
| DMSO | Sigma | D8418 |
| NOD/SCID mice | SiPeiFu | - |
| Consumables and instrument | Vendor | Cat# |
| T75 cell culture flask | Corning | 430641 |

2. Establishment of tumor model

Human multiple myeloma NCI-H929 cells were routinely cultured in RPMI1640 containing 10% fetal bovine serum at 37°C in a 5% $CO_2$ incubator. After three passages in vitro, when the cells reached over 80% confluence and the required confluence rate, the cells were digested and harvested. After washing with PBS, the cells were counted and the cell concentration was adjusted to approximately $5 \times 10^7$ cells/mL. The cells were then placed in 4 mL centrifuge tubes on ice for later use.

Female NOD/SCID mice aged 4-5 weeks were selected and subcutaneously inoculated with NCI-H929 cells ectopically. The mice were held in a lateral position, the forelimb axilla was disinfected with 75% alcohol, and 100 μL of the

cell suspension was injected subcutaneously into the axilla using a 1 mL syringe ($1.5 \times 10^7$ cells/mouse/100 μL).

3. Animal grouping and administration

After the tumors grew to 90-150 $mm^3$, the animals were randomly divided into groups, 8 mice in each group, and administered according to different administration methods as follows:

Model control group: Administered with the same volume of Vehicle (DMSO: 0.5% sodium carboxymethyl cellulose: distilled water = 1:1:8) daily by gavage;

Test group 1: administered with dexamethasone (Dex) solution at 3 mg/kg (mouse body weight) daily by gavage;

Test group 2: administered with **I-4** solution at 3 mg/kg (mouse body weight) daily by gavage;

Test group 3: administered with **I-8** solution at 3 mg/kg (mouse body weight) daily by gavage;

Test group 4: administered with dexamethasone at 3 mg/kg (mouse body weight) and **1-4** solution at 3 mg/kg (mouse body weight) daily by gavage;

Test group 5: administered with dexamethasone at 3 mg/kg (mouse body weight) and **I-8** solution at 3 mg/kg (mouse body weight) daily by gavage;

The route of administration was oral gavage, and the administration frequency was once daily, for 10 consecutive days. The first day of administration was defined as Day 1 of the experiment. Tumor volume changes in mice were measured and recorded every two days. Tumor volume was measured using a vernier caliper, in which the major axis (a) and minor axis (b) of the tumor were measured, and the tumor volume was calculated as tumor volume V ($mm^3$) = a x b^2/2. After the experiment, the mice were dissected and the tumor weight was measured. The data were entered and statistically analyzed using GraphPad Prism 6 software. The data are expressed as mean ± SEM (Standard Error of Mean), and one-way ANOVA was used. $P \leq 0.05$ was considered statistically significant.

4. Experimental results

As shown in Figure 3, Table 4-1 and Table 4-2, the experimental results indicate that compounds **I-4** and **I-8** (3 mg/kg, QD) alone effectively inhibit the growth of NCI-H929 transplanted tumors, while dexamethasone (3 mg/kg, QD) shows weaker tumor-inhibiting activity. The combined use of **I-4** (3 mg/kg, QD) and dexamethasone (3 mg/kg, QD) significantly inhibits the growth of NCI-H929 transplanted tumors. Meanwhile, the inhibitory effects of **I-4** (3 mg/kg, QD) and **I-8** (3 mg/kg, QD) respectively in combination with dexamethasone (3 mg/kg, QD) are significantly superior to those of monotherapy, demonstrating a significant synergistic effect.

Table 4-1. Inhibitory effects of compounds **I-4** and **I-8** respectively in combination with dexamethasone on human multiple myeloma NCI-H929 xenograft tumors

| Compounds | Initial volume ($mm^3$) | Average volume ($mm^3$) (Day 10) | Tumor inhibition rate TGI (%) |
|---|---|---|---|
| Control | 185.7 | 1768.6 | - |
| Dex (3 mg/kg, QD) | 185.7 | 1048.6 | 45.48% |
| I-4 (3 mg/kg, QD) | 183.9 | 689.9 | 68.03% |
| I-8 (3 mg/kg, QD) | 183.3 | 458.1 | 82.64% |
| I-4 (3 mg/kg, QD) + Dex (3 mg/kg, QD) | 197.0 | 372.0 | 88.94% |
| I-8 (3 mg/kg, QD) + Dex (3 mg/kg, QD) | 200.3 | 213.0 | 99.20% |

Table 4-2. Inhibitory effects of compounds **I-4** and **I-8** respectively in combination with dexamethasone on human multiple myeloma NCI-H929 xenograft tumors

| Compounds | *p* value (vs Vehicle) | *p* value (combination vs monotherapy) |
|---|---|---|
| Dex (3 mg/kg, QD) | <0.01 | - |
| I-4 (3 mg/kg, QD) | <0.01 | - |

(continued)

| Compounds | p value (vs Vehicle) | p value (combination vs monotherapy) | |
|---|---|---|---|
| I-8 (3 mg/kg, QD) | <0.01 | - | |
| I-4 (3 mg/kg, QD) + Dex (3 mg/kg, QD) | <0.01 | vs 1-4 | <0.01 |
| | | vs Dex | <0.01 |
| I-8 (3 mg/kg, QD) + Dex (3 mg/kg, QD) | <0.01 | vs 1-8 | <0.01 |
| | | vs Dex | <0.01 |

Example 23: In vivo activity of compound combination therapy against human multiple myeloma NCI-H929 transplanted tumor

In this example, the human multiple myeloma NCI-H929 cell line was selected as the cell model to determine the inhibitory effects of compounds, bortezomib (Bort), and tazemetostat (Taze), as single drugs and in combination, on the growth of NOD/SCID mouse transplanted tumors of NCI-H929 tumor cells. The evaluation methods and results are described below.

1. Experimental materials and instruments

1.1 Experimental consumables for bioactivity evaluation

| Materials and Reagents | Vendor | Cat# |
|---|---|---|
| NCI-H929 | Pricella | CL-0509 |
| RPMI1640 | GIBCO | C11875500BT |
| Fetal bovine serum (FBS) | Hyclone | SH30406.05 |
| DMSO | Sigma | D8418 |
| NOD/SCID mice | SiPeiFu | - |
| Consumables and instrument | Vendor | Cat# |
| T75 cell culture flask | Corning | 430641 |

2. Establishment of tumor model

Human multiple myeloma NCI-H929 cells were routinely cultured in RPMI1640 containing 10% fetal bovine serum at 37°C in a 5% $CO_2$ incubator. After three passages in vitro, when the cells reached over 80% confluence and the required confluence rate, the cells were digested and harvested. After washing with PBS, the cells were counted and the cell concentration was adjusted to approximately $5 \times 10^7$ cells/mL. The cells were then placed in 4 mL centrifuge tubes on ice for later use.

Female NOD/SCID mice aged 4-5 weeks were selected and subcutaneously inoculated with NCI-H929 cells ectopically. The mice were held in a lateral position, the forelimb axilla was disinfected with 75% alcohol, and 100 μL of the cell suspension was injected subcutaneously into the axilla using a 1 mL syringe ($1.5 \times 10^7$ cells/mouse/100 μL).

3. Animal grouping and administration

After the tumors grew to 90-150 $mm^3$, the animals were randomly divided into groups, 8 mice in each group, and administered according to different administration methods as follows:

Model control group: Administered with the same volume of Vehicle (DMSO: 0.5% sodium carboxymethyl cellulose: distilled water = 1:1:8) daily by gavage;

Test group 1: administered with **I-8** solution at 3 mg/kg (mouse body weight) daily by gavage;

Test group 2: administered with bortezomib (Bort) solution at 1 mg/kg (mouse body weight) twice weekly by gavage;

Test group 3: administered with tazemetostat solution at 100 mg/kg (mouse body weight) daily by gavage;

Test group 4: administered with **I-8** at 3 mg/kg (mouse body weight) daily by gavage and bortezomib (Bort) solution at 1 mg/kg (mouse body weight) twice weekly by gavage;

Test group 5: administered with **I-8** at 3 mg/kg (mouse body weight) daily by gavage and tazemetostat (Taze) solution at 100 mg/kg (mouse body weight) daily by gavage;

The route of administration was oral gavage, and the administration frequency was once daily, for 10 consecutive days. The first day of administration was defined as Day 1 of the experiment. Tumor volume changes in mice were measured and recorded every two days. Tumor volume was measured using a vernier caliper, in which the major axis (a) and minor axis (b) of the tumor were measured, and the tumor volume was calculated as tumor volume V ($mm^3$) = a x b^2/2. After the experiment, the mice were dissected and the tumor weight was measured. The data were entered and statistically analyzed using GraphPad Prism 6 software. The data are expressed as mean $\pm$ SEM (Standard Error of Mean), and one-way ANOVA was used. $P \leq 0.05$ was considered statistically significant.

4. Experimental results

As shown in Figure 4, Table 5-1 and Table 5-2, Figure 4A shows the changes in tumor volume in mice on Days 0, 2, 4, 6, 8, 10, and 12; Figure 4B shows the tumors dissected from each group after the experiment; and Figure 4C shows the masses of the tumors dissected from each group. The experimental results indicate that compound **I-8** (3 mg/kg, QD) alone effectively inhibits the growth of NCI-H929 transplanted tumors, while bortezomib (Bort, 1 mg/kg, BIW) and tazemetostat (Taze, 100 mg/kg, QD) alone show weaker tumor-inhibiting abilities. The combined use of **I-8** (3 mg/kg, QD) and bortezomib (1 mg/kg, BIW) significantly induces the regression of NCI-H929 transplanted tumors, and the combined use of **I-8** (3 mg/kg, QD) and tazemetostat (100 mg/kg, QD) significantly inhibits the growth of NCI-H929 transplanted tumors. Furthermore, the inhibitory effects of the combined uses are significantly superior to those of monotherapy, demonstrating a significant synergistic effect.

Table 5-1. Inhibitory effects of compound **I-8** respectively in combination with bortezomib and tazemetostat on human multiple myeloma NCI-H929 xenograft tumors

| Compounds | Initial volume ($mm^3$) | Average volume ($mm^3$) (Day 10) | Tumor inhibition rate TGI (%) |
|---|---|---|---|
| Control | 140.1 | 1800.3 | - |
| **I-8** (3 mg/kg, QD) | 120.7 | 498.6 | 77.24% |
| Bort (1 mg/kg, BIW) | 100.6 | 1249.7 | 30.78% |
| Taze (100 mg/kg, QD) | 110.8 | 1558.0 | 12.83% |
| **I-8** (3 mg/kg, QD) + Bort (3 mg/kg, BIW) | 114.5 | 91.6 | 101.37% |
| **I-8** (3 mg/kg, QD) + Taze (3 mg/kg, QD) | 141.4 | 212.0 | 95.75% |

Table 5-2. Inhibitory effects of compound **I-8** respectively in combination with bortezomib and tazemetostat on human multiple myeloma NCI-H929 xenograft tumors

| Compounds | Tumor volume *p*-value (vs Vehicle) | Tumor weight *p*-value (vs Vehicle) | *p*-values of tumor volume and weight (combined use vs monotherapy) | |
|---|---|---|---|---|
| I-8 (3 mg/kg,QD) | <0.01 | <0.01 | - | |
| Bort (1 mg/kg, BIW) | <0.01 | >0.05 | - | |
| Taze (100 mg/kg, QD) | >0.05 | >0.05 | - | |
| I-8 (3 mg/kg, QD) + Bort (3 mg/kg, BIW) | <0.01 | <0.01 | vs I-8 | <0.01 |
| | | | vs Bort | <0.01 |
| I-8 (3 mg/kg ,QD) + Taze (3 mg/kg, QD) | <0.01 | <0.01 | vs I-8 | <0.01 |
| | | | vs Taze | <0.01 |

Example 24: Evaluation of activity of compounds against lenalidomide and pomalidomide resistance

In this example, lenalidomide-resistant human multiple myeloma cells, including NCI-H929 (NCI-H929-Lenalidomide-R) and RPMI-8226 (RPMI-8226-Lenalidomide-R), and pomalidomide-resistant human multiple myeloma cells, including NCI-H929 (NCI-H929-pomalidomide-R) and RPMI-8226 (RPMI-8226-pomalidomide-R), were selected as cell models to determine the inhibitory activity of compound **I-8** of the present invention against the proliferation of the resistant cells.

1. Experimental materials and instruments

1.1 Experimental materials and instruments

| Materials and Reagents | Vendor | Cat# |
|---|---|---|
| NCI-H929-Pomalidomide-R | - | - |
| RPMI-8226-Pomalidomide-R | - | - |
| NCI-H929- Lenalidomide-R | - | - |
| RPMI-8226- Lenalidomide-R | - | - |
| CCK-8 | Dojindo | CK04 |
| RPMI-1640 | GIBCO | C11875500BT |
| Fetal Bovine Serum (FBS) | Hyclone | SH30406.05 |
| DMSO | Sigma | D8418 |
| **Consumables and Instrument** | **Vendor** | **Cat#** |
| 96-well polypropylene plate | Corning | 3599 |
| Plate shaker | Thermo | 4625-1 CECN/THZ Q |
| Centrifuge | Eppendorf | 5810R |
| Envision 2104 multi-label reader | PerkinElmer | 74785 |
| Echo | Labcyte | 550 |

2. Experimental procedure

(1) Preparation of solution

① **I-8** was dissolved in DMSO to prepare a 10 mM stock solution. Compounds used within three months should be stored at room temperature in a desiccator; others could be stored at -20°C for long-term storage.
② The **I-8** stock solution and the reference compounds lenalidomide and pomalidomide were all diluted with DMSO. Their initial concentrations were 10 μM. In the experiments of lenalidomide-resistant human multiple myeloma cells (NCI-H929 (NCI-H929-Lenalidomide-R) and RPMI-8226 (RPMI-8226-Lenalidomide-R) and pomalidomide-resistant human multiple myeloma cells (NCI-H929 (NCI-H929-pomalidomide-R) and RPMI-8226 (RPMI-8226-pomalidomide-R), 3-fold serial dilution with 11 concentration points was performed, with the lowest concentration of 0.0047 μM. The resulting solutions were shaken on a shaker for 5 minutes.

(2) Applying drugs to cells

① The NCI-H929-Lenalidomide-R, RPMI-8226-Lenalidomide-R, NCI-H929-pomalidomide-R, and RPMI-8226-pomalidomide-R cells in logarithmic growth phase were inoculated in a 96-well plate, 20,000 to 30,000 cells per well, and the plate was pre-cultured for 24 hours in an incubator (at 37°C and 5% $CO_2$).
® The culture medium in the plate was replaced, and the compounds and the positive controls with corresponding concentrations were added to the plated at a volume of 50 μL.

③ The plate was incubated for 144 hours in an incubator, then 15 μL of CCK-8 solution was added to each well, and the plate was incubated in the incubator for another 1-4 hours.
④ The absorbance at 450 nm was measured using a microplate reader.

(3) Data processing

① Calculating % Inhibition:

% Inhibition = 100 - (Signalcmpd - SignalAve_PC) / (SignalAve_VC - SignalAve_PC) × 100.

② Calculating $IC_{50}$ value of compound and plotting dose-response curve:
The $IC_{50}$ value was calculated by fitting the % Inhibition and the logarithm of compound concentration to a nonlinear regression (dose response-variable slope) by using GraphPad 6.0.

$$Y = Bottom + (Top - Bottom) / (1 + 10\text{^}((LogIC_{50} - X) * HillSlope))$$

X: log of inhibitor concentration; Y: % Inhibition.

3. Experimental results

After calculating % Inhibition, a dose-response curve was plotted with the logarithm of the test compound concentration as the x-axis and the average cell viability as the y-axis, and the $IC_{50}$ value was obtained by fitting.

Table 6 shows the test results of **I-8** on NCI-H929-Lenalidomide-R, RPMI-8226-Lenalidomide-R, NCI-H929-pomalidomide-R, and RPMI-8226-pomalidomide-R cells. The experimental results indicate that the $IC_{50}$ values of pomalidomide and lenalidomide against these cells are greater than 100 μM, and **I-8** has good inhibitory effects on these cells.

Table 6. Antiproliferative activity of compounds against NCI-H929-Lenalidomide-R, RPMI-8226-Lenalidomide-R, NCI-H929-pomalidomide-R, and RPMI-8226-pomalidomide-R cells ($IC_{50}$ Values)

| No. | $IC_{50}$ (μM) | | | |
|---|---|---|---|---|
| | NCI-H929-Lenalidomide-R | RPMI-8226-Lenalidomide-R | NCI-H929-pomalidomide-R | RPMI-8226-pomalidomide-R |
| Pomalidomide | >100 | >100 | >100 | >100 |
| Lenalidomide | >100 | >100 | >100 | >100 |
| **I-8** | 0.055 | 0.246 | 0.095 | 0.314 |

The above description merely relates to preferred embodiments of the present invention and is not intended to limit the present invention. It should be noted that those skilled in the art can make various improvements and modifications without departing from the technical principles of the present invention, and these improvements and modifications should also be considered within the scope of protection of the present invention.

## Claims

1. A compound represented by formula (I), or pharmaceutically acceptable salt, prodrug, stable isotope derivative, isomer, solvate, or polymorph thereof:

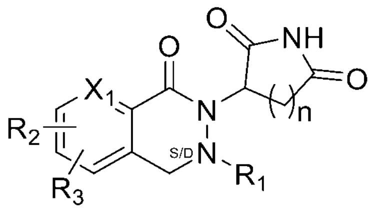

(I)

wherein:

$X_1$ is N or CH; n is 1 or 2;
$R_1$ is selected from the group consisting of H, $C_{1-10}$ linear or branched alkyl and $C_{3-10}$ cycloalkyl;
S/D is a single bond or a double bond, and when it is a double bond, $R_1$ is absent;
$R_2$ and $R_3$ are each independently selected from the group consisting of H, D, halogen, substituted or unsubstituted alkyl, substituted or unsubstituted alkenyl, substituted or unsubstituted alkynyl, substituted or unsubstituted cycloalkyl, substituted or unsubstituted aryl, substituted or unsubstituted heteroaryl, substituted or unsubstituted heterocyclyl, -S-alkyl, -CN, $-NO_2$, $-N_3$, $-CH(Ph)_2$, perfluoro-$C_1$-$C_4$ alkyl, perfluoro-$C_1$-$C_4$ alkoxy, $-NR_4R_5$, $-OR_4$, $-COR_4$, $-CO_2R_4$, $-CONR_4R_5$, $-C(=NR_4)NR_5R_6$, $-NR_4COR_5$, $-NR_4CO_2R_5$, $-SO_2R_4$, $-NR_4SO_2NR_5R_6$, and $-NR_4SO_2R_5$;
$R_4$, $R_5$, and $R_6$ are each independently H, D, alkyl, heteroalkyl, alkenyl, alkynyl, cycloalkyl, saturated or unsaturated heterocyclyl, aryl, or heterocyclylaryl; wherein ($R_4$ and $R_5$) and/or ($R_5$ and $R_6$), together with the atoms to which they are connected, may each form a ring, and the ring is selected from the group consisting of optionally substituted or unsubstituted cycloalkyl, saturated or unsaturated heterocyclyl, aryl, and heterocyclylaryl.

**2.** The compound according to claim 1, or pharmaceutically acceptable salt, prodrug, stable isotope derivative, isomer, solvate, or polymorph thereof, which has one or more of the following characteristics:

(a) $X_1$ is CH, n is 2;
(b) $R_1$ is selected from the group consisting of H and $C_{1-6}$ linear or branched alkyl, preferably H and $C_{1-4}$ linear or branched alkyl, preferably $C_{1-4}$ linear or branched alkyl, preferably $CH_3$;
(c) $R_2$ is selected from the group consisting of H and halogen, preferably H and F, preferably H;
(d) S/D is a single bond;
(e) $R_3$ is selected from the group consisting of H, D, halogen, substituted or unsubstituted alkyl, substituted or unsubstituted alkenyl, substituted or unsubstituted alkynyl, substituted or unsubstituted cycloalkyl, substituted or unsubstituted aryl, substituted or unsubstituted heteroaryl, substituted or unsubstituted heterocyclyl, -S-alkyl, -CN, $-NO_2$, $-N_3$, $-CH(Ph)_2$, perfluoro-$C_1$-$C_4$ alkyl, perfluoro-$C_1$-$C_4$ alkoxy, $-NR_4R_5$, $-OR_4$, $-COR_4$, $-CO_2R_4$, $-CONR_4R_5$, $-C(=NR_4)NR_5R_6$, $-NR_4COR_5$, $-NR_4CO_2R_5$, $-SO_2R_4$, $-NR_4SO_2NR_5R_6$, and $-NR_4SO_2R_5$;

$R_4$, $R_5$, and $R_6$ are each independently H, D, alkyl, heteroalkyl, alkenyl, alkynyl, cycloalkyl, saturated or unsaturated heterocyclyl, aryl, or heterocyclylaryl; wherein ($R_4$ and $R_5$) and/or ($R_5$ and $R_6$), together with the atoms to which they are connected, may each form a ring, and the ring is selected from the group consisting of optionally substituted or unsubstituted cycloalkyl, saturated or unsaturated heterocyclyl, aryl, and heterocyclylaryl.

**3.** The compound according to claim 2, or pharmaceutically acceptable salt, prodrug, stable isotope derivative, isomer, solvate, or polymorph thereof, wherein $R_3$ is selected from the group consisting of H, D, halogen, substituted or unsubstituted $C_1$-$C_4$ alkyl, substituted or unsubstituted $C_2$-$C_6$ alkenyl, substituted or unsubstituted $C_2$-$C_6$ alkynyl, substituted or unsubstituted $C_3$-$C_8$ cycloalkyl, substituted or unsubstituted aryl, substituted or unsubstituted heteroaryl, substituted or unsubstituted heterocyclyl, $-S-R_4$, -CN, $-NO_2$, perfluoro-$C_1$-$C_4$ alkyl, perfluoro-$C_1$-$C_4$ alkoxy, $-NR_4R_5$, $-OR_4$, $-COR_4$, $-CO_2R_4$, $-CONR_4R_5$, $-C(=NR_4)NR_5R_6$, $-NR_4COR_5$, $-NR_4CO_2R_5$, $-SO_2R_4$, $-NR_4SO_2NR_5R_6$, and $-NR_4\ SO_2R_5$;

$R_4$, $R_5$, and $R_6$ are each independently H, D, alkyl, heteroalkyl, alkenyl, alkynyl, cycloalkyl, saturated or unsaturated heterocyclyl, aryl, or heterocyclylaryl; wherein ($R_4$ and $R_5$) and/or ($R_5$ and $R_6$), together with the atoms to which they are connected, may each form a ring, and the ring is selected from the group consisting of optionally substituted or unsubstituted cycloalkyl, saturated or unsaturated heterocyclyl, aryl, and heterocycly-

laryl;

preferably, $R_3$ is selected from the group consisting of H, halogen, -OH, -S-$R_4$, -CN, -$NO_2$, and -$NR_4R_5$, preferably H, halogen, -$NO_2$, and -$NR_4R_5$, wherein $R_4$ and $R_5$ are each independently H, D, $C_{1-4}$ alkyl, or $R_4$ and $R_5$, together with the atoms to which they are connected, form a ring, and the ring is selected from the group consisting of optionally substituted or unsubstituted cycloalkyl, saturated or unsaturated heterocyclyl, aryl, and heterocyclylaryl.

**4.** The compound according to any one of claims 1 to 3, or pharmaceutically acceptable salt, prodrug, stable isotope derivative, isomer, solvate, or polymorph, wherein $R_3$ is selected from the group consisting of H, D, halogen, substituted or unsubstituted $C_1$-$C_4$ alkyl, substituted or unsubstituted $C_2$-$C_6$ alkenyl, substituted or unsubstituted $C_2$-$C_6$ alkynyl, substituted or unsubstituted $C_3$-$C_8$ cycloalkyl, substituted or unsubstituted aryl, substituted or unsubstituted heteroaryl, substituted or unsubstituted heterocyclyl, -S-$R_4$, -CN, -$NO_2$, perfluoro-$C_1$-$C_4$ alkyl, perfluoro-$C_1$-$C_4$ alkoxy, -$NR_4R_5$, -$OR_4$, -$COR_4$, -$CO_2R_4$, -$CONR_4R_5$, -C(=$NR_4$)$NR_5R_6$, -$NR_4COR_5$, -$NR_4CO_2R_5$, -$SO_2R_4$, -$NR_4SO_2NR_5R_6$, and -$NR_4SO_2R_5$;

$R_4$, $R_5$, and $R_6$ are each independently H, D, alkyl, heteroalkyl, alkenyl, alkynyl, cycloalkyl, saturated or unsaturated heterocyclyl, aryl, or heterocyclylaryl; wherein ($R_4$ and $R_5$) and/or ($R_5$ and $R_6$), together with the atoms to which they are attached, may each form a ring as represented by formula (II),

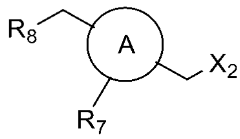

(II)

wherein $X_2$ is selected from the group consisting of NH or $CH_2$ and O, preferably $X_2$ is N;

ring A is a 5- to 6-membered aromatic ring containing 0-3 heteroatoms N, S or O, preferably a benzene ring;

$R_7$ is selected from the group consisting of H, D, halogen, alkyl, heteroalkyl, alkenyl, alkynyl, cycloalkyl, saturated or unsaturated heterocyclyl, aryl and heterocyclylaryl, preferably, $R_7$ is selected from the group consisting of H, D and halogen;

$R_8$ is selected from the group consisting of H, D, substituted or unsubstituted alkyl, substituted or unsubstituted alkenyl, substituted or unsubstituted alkynyl, substituted or unsubstituted cycloalkyl, substituted or unsubstituted aryl, substituted or unsubstituted heteroaryl, substituted or unsubstituted heterocyclyl, -S-alkyl, -CN, -$NO_2$, -$N_3$, -$CH(Ph)_2$, perfluoro-$C_1$-$C_4$ alkyl, perfluoro-$C_1$-$C_4$ alkoxy, -$NR_9R_{10}$, -$OR_9$, -$COR_9$, -$CO_2R_9$, -$CONR_9R_{10}$, -C(=$NR_9$)$NR_9R_{10}$, -$NR_9COR_{10}$, -$NR_9CO_2R_{10}$, -$SO_2R_9$, -$NR_9SO_2NR_{10}R_{11}$, and -$NR_9SO_2R_{10}$;

$R_9$, $R_{10}$, and $R_{11}$ are each independently H, D, alkyl, heteroalkyl, alkenyl, alkynyl, cycloalkyl, saturated or unsaturated heterocyclyl, aryl, or heterocyclylaryl; wherein ($R_9$ and $R_{10}$) and/or ($R_{10}$ and $R_{11}$), together with the atoms to which they are connected, may each form a ring, and the ring is selected from the group consisting of optionally substituted or unsubstituted cycloalkyl, saturated or unsaturated heterocyclyl, aryl, and heterocyclylaryl;

preferably, $R_8$ is selected from the group consisting of H, D, substituted or unsubstituted 5-to 9-membered heterocyclyl and -$NR_9R_{10}$, $R_9$ and $R_{10}$ are each independently H, D and $C_{1-4}$ alkyl, or $R_9$ and $R_{10}$ together with the atoms to which they are connected form a substituted or unsubstituted 5- to 9-membered cycloalkyl; preferably, the 5- to 9-membered heterocyclyl contains 1, 2 or 3 N atoms; preferably, the 5- to 9-membered heterocyclyl is selected from the group consisting of morpholino and piperazine;

preferably, $R_3$ is selected from the group consisting of H, halogen, -OH, -SH, -CN, -$NO_2$, -$NH_2$, -NH($C_{1-4}$ alkyl) and

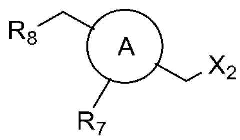

(formula (II)),

preferably H, halogen, -$NO_2$, -$NH_2$, -NH($CH_3$) and

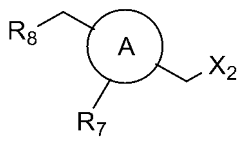

(formula (II)),

and the $R_7$, $R_8$, $X_2$ and ring A are as defined in this claim;
the ring represented by formula (II) is further preferably selected from the following:

ring A is a benzene ring;
$R_7$ is H;
$X_2$ is NH;
$R_8$ is a structure represented by formula (III):

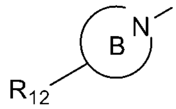

(III)

wherein ring B is an N-substituted non-aromatic ring, which is selected from the group consisting of any of the following structures:

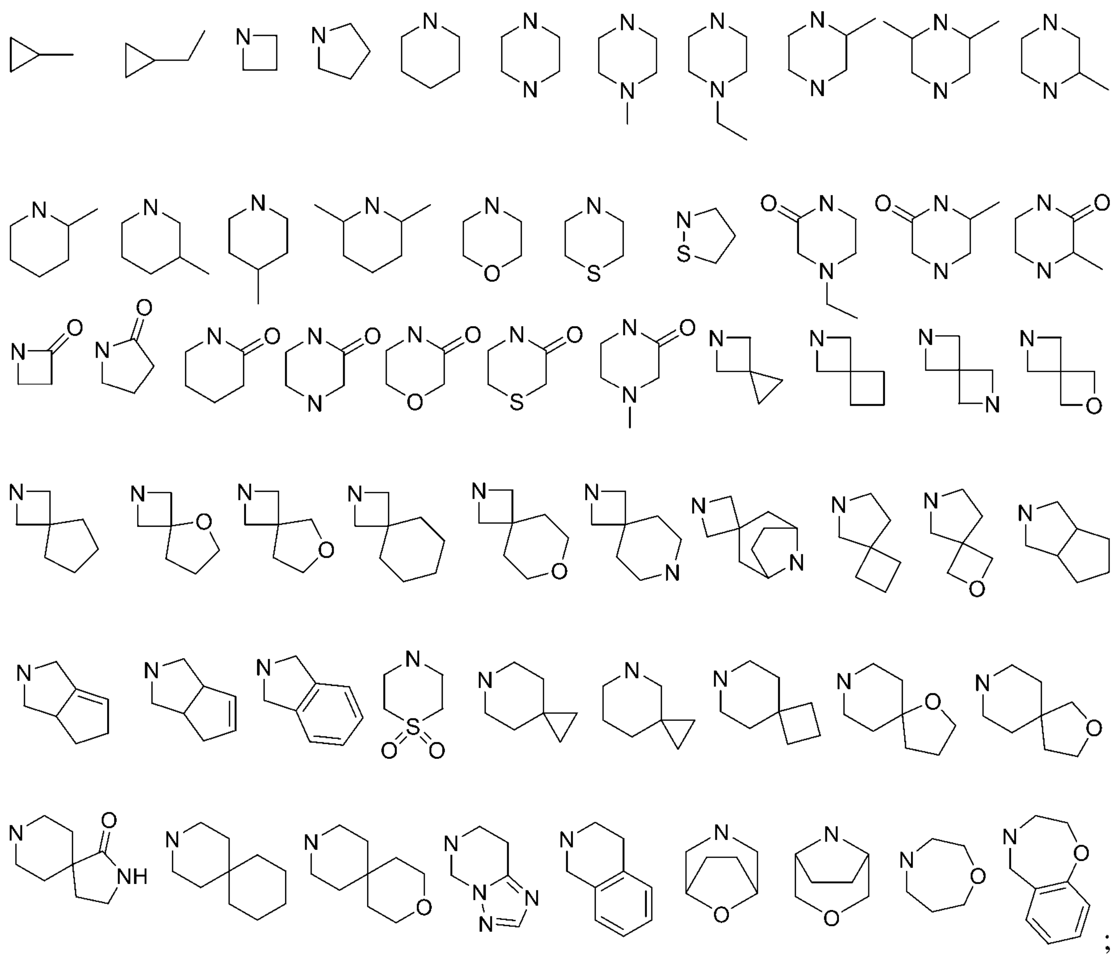

;

preferably, ring B is selected from the group consisting of

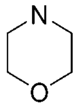 and 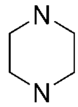 ,

preferably

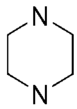 ;

$R_{12}$ is selected from the group consisting of H, D, halogen, -CN, -$NO_2$, alkyl, heteroalkyl, alkenyl, alkynyl, cycloalkyl, saturated or unsaturated heterocyclyl, aryl and heterocyclylaryl; wherein if the alkyl, heteroalkyl, alkenyl, alkynyl, cycloalkyl, saturated or unsaturated heterocyclyl, aryl or heterocyclylaryl is substituted, it is optionally substituted by at least one of substituent $R_{13}$, and $R_{13}$ is halogen, lower alkyl, lower alkoxy, cyano, or nitro;

preferably, $R_{12}$ is selected from the group consisting of H, D, halogen, -C(=O)-$C_{1-6}$ alkyl, phenyl, and 5- to 6-membered heteroaryl, wherein the phenyl and 5- to 6-membered heteroaryl are optionally substituted with one or more $R_{13}$, wherein the $R_{13}$ is selected from the group consisting of halogen, $C_{1-4}$ alkyl, cyano, and nitro;

preferably, $R_{12}$ is selected from the group consisting of H, D, halogen, -C(=O)-$C_{1-4}$ alkyl, and phenyl, wherein the phenyl is optionally substituted with one, two, or three $R_{13}$, wherein the $R_{13}$ is selected from the group consisting of F and cyano;

preferably, $R_{12}$ is selected from the group consisting of -C(=O)OC($CH_3$)$_3$, phenyl,

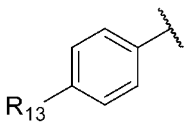 and 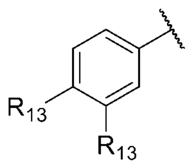 ,

wherein $R_{13}$ is selected from the group consisting of F and cyano;

in formula (III), ring B is preferably a piperazine ring substituted with lower alkyl, alkenyl, alkynyl, halogen, nitro, etc., or a substituted piperazine as represented by formula (IV).

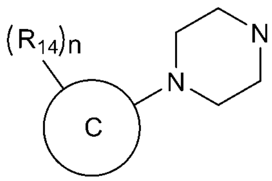

(IV)

wherein, ring C is a 5- to 6-membered aromatic ring, or an aromatic heterocyclyl containing 0-3 heteroatoms N, S, or O;

$R_{14}$ is selected from the group consisting of H, D, halogen, lower alkyl, lower alkoxy, cyano, nitro, and hydroxyl;

n = 0, 1, 2, 3, 4, or 5;

preferably, in formula (III), ring B is a piperazine ring substituted with -C(=O)OC($CH_3$)$_3$ or a substituted piperazine as represented by formula (IV);

preferably, the structural unit

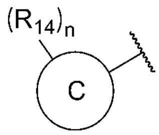

is selected from the group consisting of

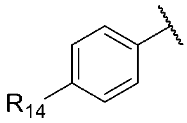 and 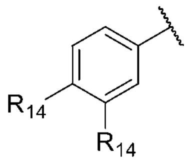 ;

preferably, ring C is phenyl;
preferably, $R_{14}$ is selected from the group consisting of H, F, and cyano, preferably F and cyano;
preferably, the structural unit

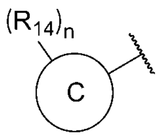

is selected from the group consisting of phenyl,

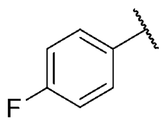 , 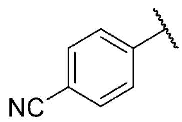 and 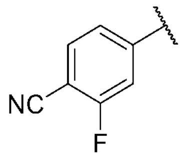 .

**5.** The compound according to any one of claims 1 to 4, or pharmaceutically acceptable salt, prodrug, stable isotope derivative, isomer, solvate, or polymorph thereof, wherein the compound is the compound represented by formula (1-1),

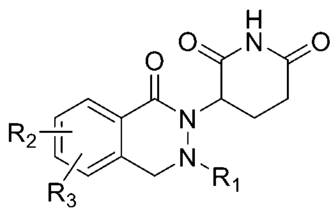

(I-1)

$R_1$, $R_2$, and $R_3$ are as defined in any one of claims 1 to 4;
preferably, the structural unit

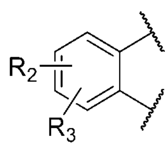

is selected from the group consisting of

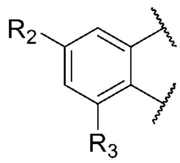 and 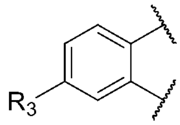 ;

preferably, the structural unit

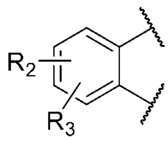 is 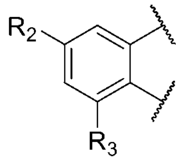 ;

preferably, $R_3$ is selected from the group consisting of H, halogen, $-NO_2$, and $-NR_4R_5$, wherein $R_4$ and $R_5$ are as defined in any one of claims 1 to 4;
preferably, $R_3$ is selected from the group consisting of H, $-NO_2$, $-NH_2$, and -NH($C_{1-4}$ alkyl);
preferably, $R_3$ is selected from the group consisting of H, $-NO_2$, $-NH_2$, and $-NH(CH_3)$;
preferably, $R_3$ is $-NR_4R_5$, wherein $R_4$ and $R_5$ are as defined in any one of claims 1 to 4;
preferably, $R_3$ is selected from the group consisting of $-NH_2$ and $-NH(CH_3)$;
preferably, $R_3$ is $-NO_2$;
preferably, $R_3$ is

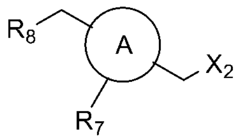
,

wherein $X_2$, ring A, $R_7$ and $R_8$ are as defined in any one of claims 1 to 4;
preferably, $R_8$ is selected from the group consisting of H, D, substituted or unsubstituted 5-to 9-membered heterocyclyl, and

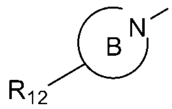
,

wherein ring B and $R_{12}$ are as defined in any one of claims 1 to 4;
preferably, the 5- to 9-membered heterocyclyl contains 1, 2 or 3 N atoms; preferably, the 5-to 9-membered heterocyclyl is selected from the group consisting of morpholino and piperazine;
preferably, the 5- to 9-membered heterocyclyl is optionally substituted with 1, 2 or 3 groups selected from the group consisting of H, D, halogen, nitro, $C_{1-4}$ alkyl, -C(=O)O$C_{1-4}$ alkyl and

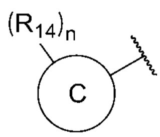
, '

wherein ring C, $R_{14}$ and n are as defined in any one of claims 1 to 4;
preferably, $R_{12}$ is substituted with groups selected from the group consisting of H, D, halogen, nitro, $C_{1-4}$ alkyl, -C(=O)O$C_{1-4}$ alkyl and

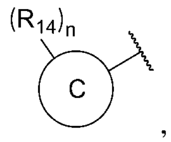

wherein ring C, $R_{14}$ and n are as defined in any one of claims 1 to 4; preferably, the compound is the compound represented by formula (II-1)

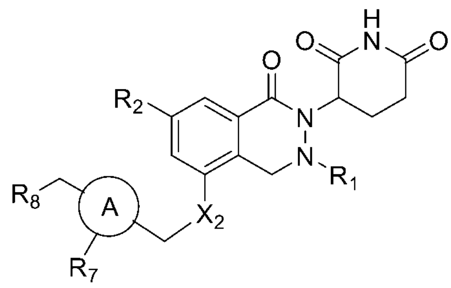

(II-1)

wherein, $R_1$, $R_2$, $R_7$, $R_8$, $X_2$, and ring A are as defined in any one of claims 1 to 4 or as defined in this claim; preferably, the compound is the compound represented by formula (III-1),

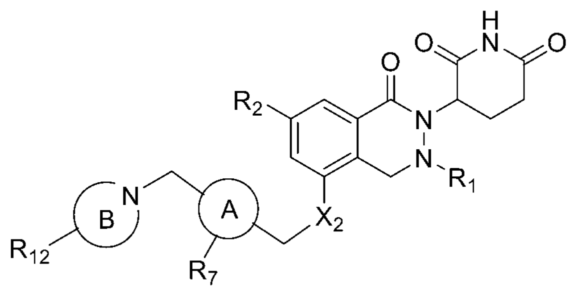

(III-1)

wherein, $R_1$, $R_2$, $R_7$, $R_{12}$, $X_2$, ring A, and ring B are as defined in any one of claims 1 to 4 or as defined in this claim; preferably, the compound is the compound represented by formula (IV-1),

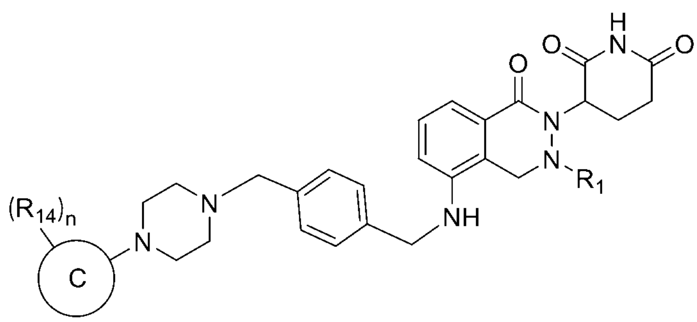

(IV-1)

wherein, $R_1$, ring C, $R_{14}$, and n are as defined in any one of claims 1 to 4 or as defined in this claim.

**6.** The compound according to any one of claims 1 to 5, or pharmaceutically acceptable salt, prodrug, stable isotope derivative, isomer, solvate, or polymorph thereof, wherein the compound is selected from the group consisting of:

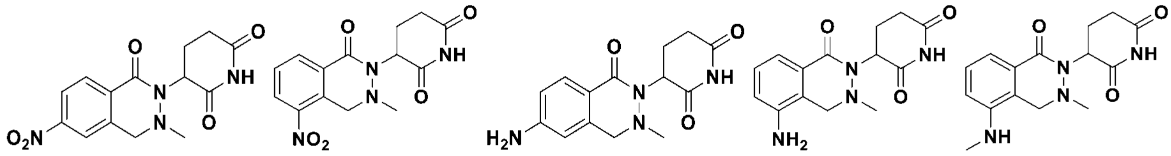

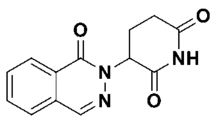
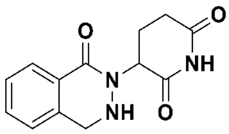
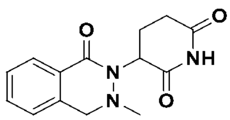
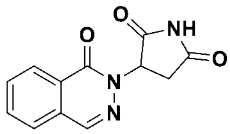
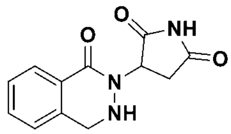

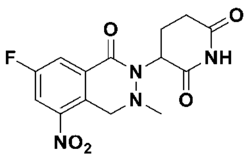
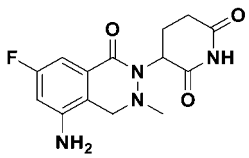
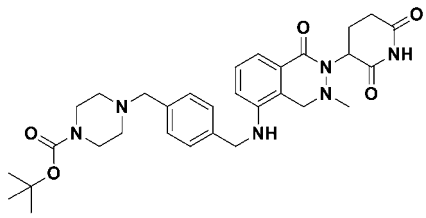

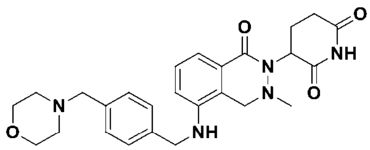
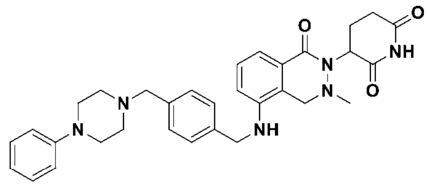

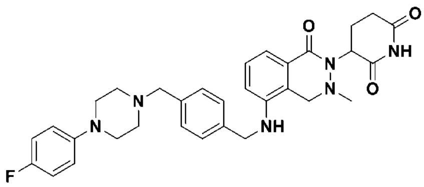

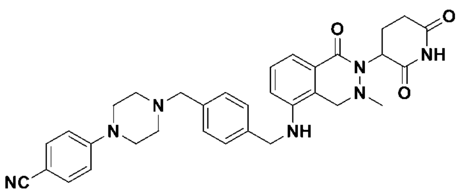 and 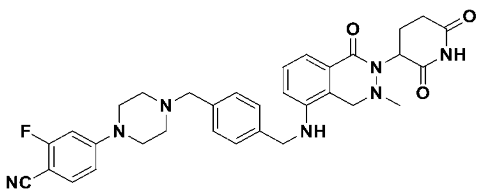 .

7. A method for preparing the compound according to any one of claims 1 to 6, which comprises:

Synthetic scheme A: reacting the compound represented by chemical formula $A_1$ with benzaldehyde and methylhydrazine to prepare $A_2$; cyclizing $A_2$ under the catalysis of a Lewis acid (e.g., $ZnCl_2$) to form $A_3$; substituting $A_3$ with 3-bromocycloglutarimide to generate the target product $A_4$, wherein: $R_2$ and $R_3$ are defined as in any one of claims 1 to 6;

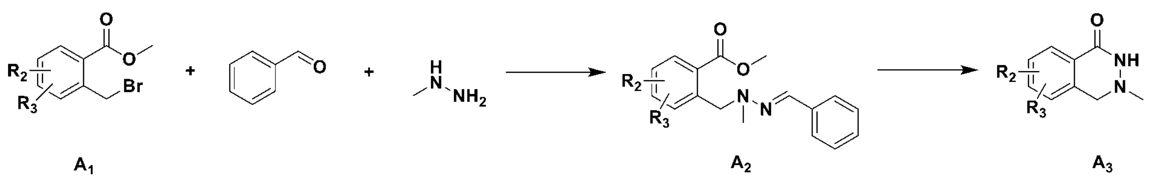

$A_1$ $A_2$ $A_3$

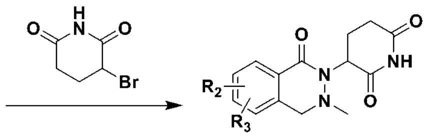

$A_4$

;

or

Synthetic scheme B: firstly, using 2-formylbenzoic acid $B_1$ as the starting material and performing a condensation reaction with hydrazine hydrate under heating conditions to obtain phthalazinone $B_2$; subjecting $B_2$ to a substitution reaction with dimethyl 2-bromoglutarate ($B_{10}$) to give $B_3$; then performing a cyclization of $B_3$ under the basic catalysis of sodium amide to give $B_4$; wherein the key intermediate $B_{10}$ is derived from glutaric acid $B_7$ as the starting material via a one-pot process by sequentially carrying out thionyl chloride acylation, liquid bromine substitution, and methanol esterification to yield $B_8$, $B_9$, and $B_{10}$; reducing $B_4$ (e.g., by zinc reduction) to give $B_5$; methylating $B_5$ (e.g., using formaldehyde and formic acid reagent) to give $B_6$,

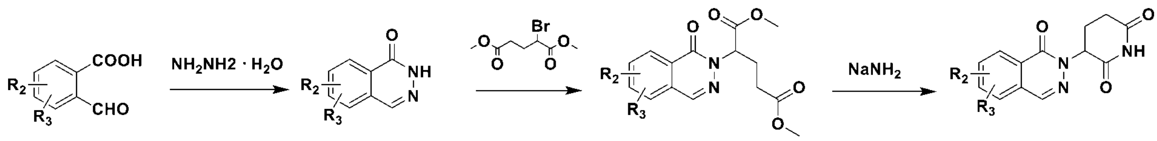


$B_1$ $B_2$ $B_3$ $B_4$

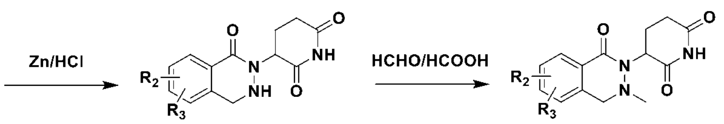


$B_5$ $B_6$

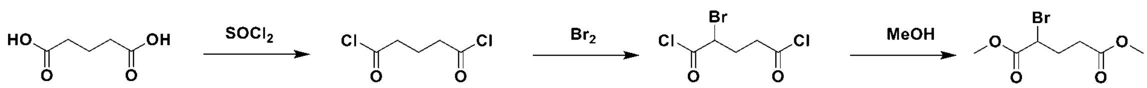


$B_7$ $B_8$ $B_9$ $B_{10}$

;

or,

Synthetic scheme C: synthesizing compound $A_4$ according to Scheme A; substituting the unsaturated nitrogen-containing non-aromatic ring compound R with methyl 4-bromomethylbenzoate to yield $C_5$; firstly fully reducing the methyl benzoate moiety of $C_5$ to generate an alcohol (compound $C_6$), then partially oxidizing $C_6$ to generate an aldehyde $C_7$; finally, conducting a reductive amination reaction between $C_7$ and the aromatic amino group of the aforementioned compound $A_4$ to form an imine therebetween, thereby obtaining the target product, wherein R is the ring B as defined in formula (III),

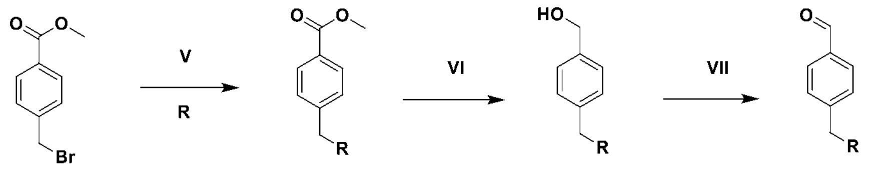


$C_5$ $C_6$ $C_7$

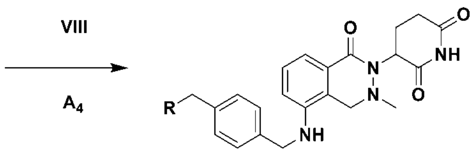


;

or,

Synthetic scheme D: under the conditions of $Pd_2(dba)_3$ as a catalyst, X-Phos as a ligand, and NaOtBu as a base, carrying out a Buchwald-Hartwig carbon-nitrogen coupling reaction between 1-tert-butyloxycarbonylpiperazine and the halogenated aromatic ring compound R to generate compound $D_5$; deprotecting the Boc protecting group on N of compound $D_5$ to obtain $D_6$; the subsequent synthetic steps are consistent with the synthetic route of Scheme C: successively performing methyl 4-bromomethylbenzoate substitution, DIBAL reduction, and manganese dioxide oxidation to obtain compounds $D_7$, $D_8$, and $D_9$, respectively; and conducting a reductive amination reaction between compounds $D_9$ and $A_4$ to obtain the target compound,

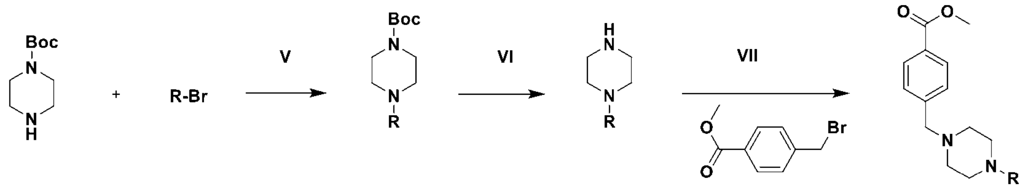


$D_5$ $D_6$ $D_7$

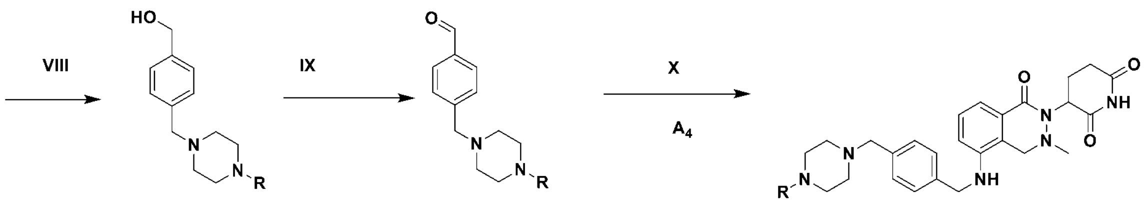


$D_8$ $D_9$

.

**8.** A pharmaceutical composition, which comprises at least one compound according to any one of claims 1 to 6, or pharmaceutically acceptable salt, prodrug, stable isotope derivative, isomer, solvate, or polymorph thereof, and one or more pharmaceutically acceptable carriers or excipients,

preferably, the pharmaceutically acceptable carriers or excipients comprise: ion exchanger, alumina, aluminum stearate, lecithin, serum proteins such as human serum albumin, buffering substances such as phosphates, glycerol, sorbic acid, potassium sorbate, mixture of partial glycerides of saturated vegetable fatty acids, water, salts or electrolytes such as protamine sulfate, disodium hydrogen phosphate, potassium hydrogen phosphate, sodium chloride, zinc salts, colloidal silica, magnesium trisilicate, polyvinylpyrrolidone, cellulose substances, polyethylene glycol, sodium carboxymethyl cellulose, polyacrylate, beeswax, and lanolin.

**9.** A combination drug, comprising the compound according to any one of claims 1 to 6, or pharmaceutically acceptable salt, prodrug, stable isotope derivative, isomer, solvate, or polymorph thereof, and at least one additional drug, wherein the at least one additional drug is a chemotherapeutic agent or immunomodulator (e.g., an immune checkpoint inhibitor, a tyrosine kinase inhibitor, a proteasome inhibitor, an antibiotic, an alkylating agent, an antimetabolite, a hormone, an immunomodulator, an interferon-like agent, or a combination of agents), preferably selected from the group consisting of dexamethasone, bortezomib, and tazemetostat.

**10.** Use of the compound according to any one of claims 1 to 6, or pharmaceutically acceptable salt, prodrug, stable isotope derivative, isomer, solvate, or polymorph thereof, or the pharmaceutical composition according to claim 8, or the combination drug according to claim 9, in the manufacture of a medicament for the treatment and/or prevention of a disease associated with $CRL4^{CRBN}$ E3 ubiquitin ligase; preferably, the disease comprising cancer, pain, neurological disease, and immune system disease.

**11.** Use of the compound according to any one of claims 1 to 6, or pharmaceutically acceptable salt, prodrug, stable isotope derivative, isomer, solvate, or polymorph thereof, or the pharmaceutical composition according to claim 8, or the combination drug according to claim 9, in the manufacture of a medicament for treating and/or preventing a cancerous disease, wherein the cancerous disease comprises: one or more of various types of leukemia, multiple myeloma, various malignant lymphomas such as non-Hodgkin's lymphoma, diffuse large B-cell lymphoma, follicular lymphoma, mantle cell lymphoma, marginal zone lymphoma, Waldenström macroglobulinemia, erythema nodosum, autoimmune diseases such as systemic lupus erythematosus, myelodysplastic syndrome, breast cancer, gastrointestinal cancer, various types of lung cancer (e.g., non-small cell lung cancer), liver cancer, pancreatic cancer, skin cancer, head and neck cancer, melanoma, uterine cancer, ovarian cancer, various endocrine gland cancers (e.g., breast cancer, thyroid cancer, gastric cancer), kidney or ureter cancer, CNS tumor, preferably multiple myeloma.

**12.** The compound according to any one of claims 1 to 6, or pharmaceutically acceptable salt, prodrug, stable isotope derivative, isomer, solvate, or polymorph thereof, or the pharmaceutical composition according to claim 8, or the combination drug according to claim 9, which may be administered via a suitable route, such as oral, sublingual, rectal, parenteral, injection (intradermal, subcutaneous, intramuscular, intravenous, arterial), pulmonary, nasal, tongue, buccal, skin, mucous membrane, conjunctiva, local administration, or implantation.

**13.** Use of the compound according to any one of claims 1 to 6, or pharmaceutically acceptable salt, prodrug, stable isotope derivative, isomer, solvate, or polymorph thereof, in the manufacture of a proteolysis targeting chimera (PROTAC),
preferably, the compound, or pharmaceutically acceptable salt, prodrug, stable isotope derivative, isomer, solvate, or polymorph thereof, is used as a ligand for $CRL4^{CRBN}$ E3 ubiquitin ligase in the manufacture of a proteolysis targeting chimera (PROTAC).

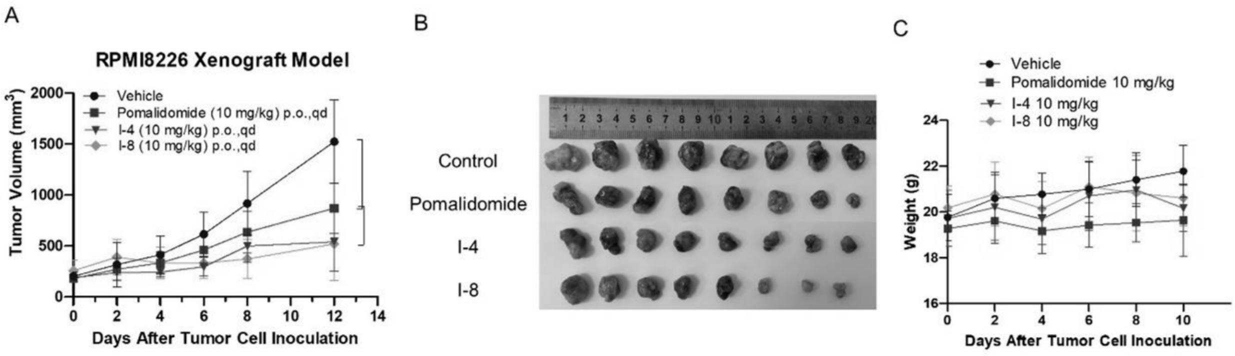


Figure 1

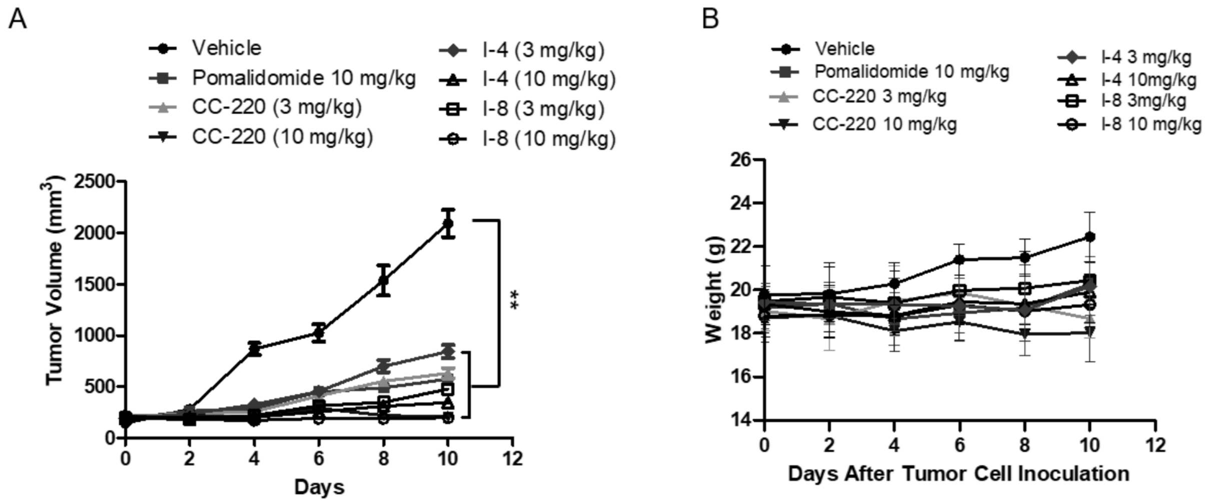


Figure 2

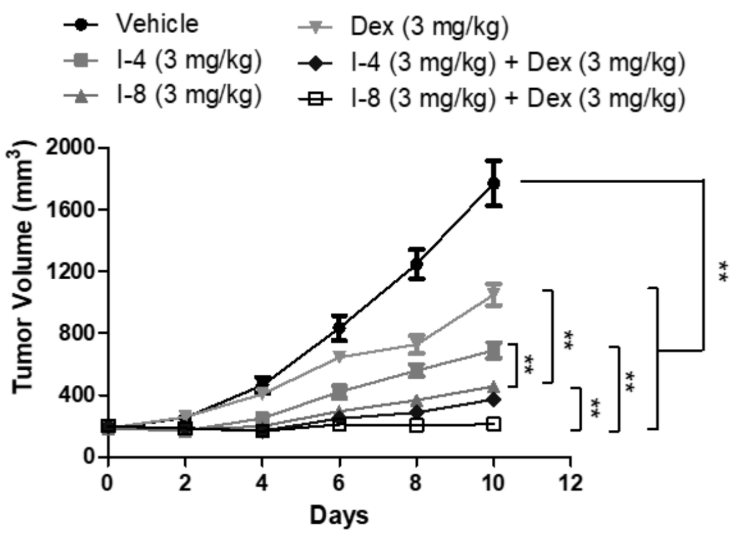


Figure 3

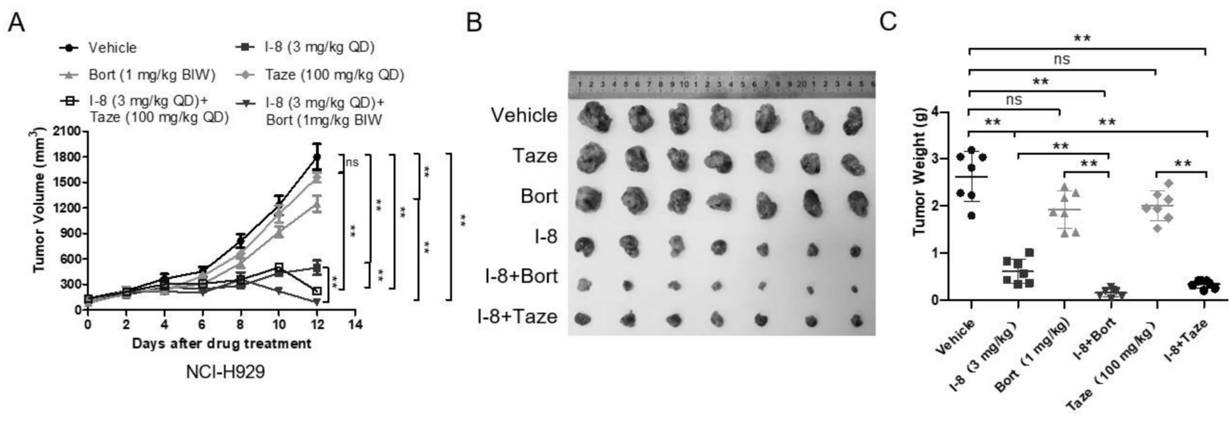
A
Vehicle
I-8 (3 mg/kg QD)
Bort (1 mg/kg BIW)
Taze (100 mg/kg QD)
I-8 (3 mg/kg QD)+ Taze (100 mg/kg QD)
I-8 (3 mg/kg QD)+ Bort (1mg/kg BIW
Tumor Volume (mm3)
Days after drug treatment
NCI-H929
B
Vehicle
Taze
Bort
I-8
I-8+Bort
I-8+Taze
C
Tumor Weight (g)
Vehicle
I-8 (3 mg/kg)
Bort (1 mg/kg)
I-8+Bort
Taze (100 mg/kg)
I-8+Taze

**Figure 4**

## INTERNATIONAL SEARCH REPORT

International application No.
**PCT/CN2024/097038**

**A. CLASSIFICATION OF SUBJECT MATTER**

C07D 401/04(2006.01)i; C07D 403/04(2006.01)i; A61K 31/502(2006.01)i; A61K 31/5377(2006.01)i; A61K 45/06(2006.01)i; A61P 35/00(2006.01)i; A61P 35/02(2006.01)i; A61P 29/00(2006.01)i; A61P 25/00(2006.01)i; A61P 37/02(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

WPABS; CNABS; CNKI; CJFD; CNTXT; WOTXT; 超星读秀, DUXIU; 万方, WANFANG; STN; USTXT; EPTXT: 中国人民解放军军事科学院军事医学研究院, 郑志兵, 李鹏运, 李松, 胡小桐, 肖军海, 钟武, 周辛波, CRBN, E3泛素连接酶, 式(I)结构, cerebion, E3 Ubiquitin Ligase

**C. DOCUMENTS CONSIDERED TO BE RELEVANT**

| Category* | Citation of document, with indication, where appropriate, of the relevant passages | Relevant to claim No. |
|---|---|---|
| PX | CN 116655591 A (ACADEMY OF MILITARY MEDICAL SCIENCES OF THE PLA ACADEMY OF MILITARY SCIENCE) 29 August 2023 (2023-08-29) claims 1-12 | 1-13 |
| PX | CN 117720517 A (TIBET HAISCO PHARMACEUTICAL CO., LTD.) 19 March 2024 (2024-03-19) description, page 5,2 second-to-last line | 1-4 |
| E | CN 118206558 A (TIBET HAISCO PHARMACEUTICAL CO., LTD.) 18 June 2024 (2024-06-18) claim 8 | 1-4 |
| E | CN 118184639 A (NANJING SANHOME PHARMACEUTICAL CO., LTD.) 14 June 2024 (2024-06-14) claim 8 | 1-4 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 July 2024** | **03 September 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/097038**

**C. DOCUMENTS CONSIDERED TO BE RELEVANT**

| Category* | Citation of document, with indication, where appropriate, of the relevant passages | Relevant to claim No. |
|---|---|---|
| E | CN 118146224 A (TIBET HAISCO PHARMACEUTICAL CO., LTD.) 07 June 2024 (2024-06-07)<br>claim 9 | 1-4 |
| X | WO 2022250224 A1 (INNOCURE THERAPEUTICS, INC.) 01 December 2022 (2022-12-01)<br>claims 1-3, and description, paragraphs 1086-1099 | 1-6, 8-13 |
| X | WO 2022250350 A1 (INNOCURE THERAPEUTICS, INC.) 01 December 2022 (2022-12-01)<br>description, paragraphs 29 and 30 | 1-4 |
| X | WO 2023081476 A1 (RANOK THERAPEUTICS (HANGZHOU) CO., LTD.; YING WEIWEN) 11 May 2023 (2023-05-11)<br>description, page 62 | 1-4 |
| X | WO 2023016518 A1 (SICHUAN HAISCO PHARMACEUTICAL CO., LTD.) 16 February 2023 (2023-02-16)<br>description, page 68, line 6 | 1-4 |
| X | CN 113801098 A (SHANGHAI JEMINCARE PHARMACEUTICALS CO., LTD. et al.) 17 December 2021 (2021-12-17)<br>claim 17 | 1-4 |

Form PCT/ISA/210 (second sheet) (July 2022)

**INTERNATIONAL SEARCH REPORT**
**Information on patent family members**

International application No.
**PCT/CN2024/097038**

| Patent document cited in search report | | | Publication date (day/month/year) | Patent family member(s) | | | Publication date (day/month/year) |
|---|---|---|---|---|---|---|---|
| CN | 116655591 | A | 29 August 2023 | None | | | |
| CN | 117720517 | A | 19 March 2024 | None | | | |
| CN | 118206558 | A | 18 June 2024 | None | | | |
| CN | 118184639 | A | 14 June 2024 | WO | 2024125437 | A1 | 20 June 2024 |
| CN | 118146224 | A | 07 June 2024 | None | | | |
| WO | 2022250224 | A1 | 01 December 2022 | JP | 2024519628 | A | 21 May 2024 |
| | | | | US | 2023295105 | A1 | 21 September 2023 |
| | | | | KR | 20220165709 | A | 15 December 2022 |
| | | | | EP | 4116298 | A1 | 11 January 2023 |
| | | | | EP | 4116298 | A4 | 28 June 2023 |
| | | | | KR | 102474999 | B1 | 07 December 2022 |
| | | | | CN | 116323619 | A | 23 June 2023 |
| WO | 2022250350 | A1 | 01 December 2022 | KR | 20230011467 | A | 20 January 2023 |
| | | | | KR | 20220159886 | A | 05 December 2022 |
| | | | | KR | 102489160 | B1 | 18 January 2023 |
| WO | 2023081476 | A1 | 11 May 2023 | WO | 2023077441 | A1 | 11 May 2023 |
| WO | 2023016518 | A1 | 16 February 2023 | TW | 202321219 | A | 01 June 2023 |
| | | | | EP | 4385985 | A1 | 19 June 2024 |
| CN | 113801098 | A | 17 December 2021 | US | 2023131252 | A1 | 27 April 2023 |
| | | | | CR | 20230007 | A | 01 June 2023 |
| | | | | KR | 20230024328 | A | 20 February 2023 |
| | | | | TW | 202146400 | A | 16 December 2021 |
| | | | | TWI | 781651 | B | 21 October 2022 |
| | | | | MX | 2022015562 | A | 30 January 2023 |
| | | | | IL | 298962 | A | 01 February 2023 |
| | | | | CL | 2022003472 | A1 | 02 June 2023 |
| | | | | EP | 4166550 | A1 | 19 April 2023 |
| | | | | EP | 4166550 | A4 | 07 August 2024 |
| | | | | CA | 3186981 | A1 | 16 December 2021 |
| | | | | US | 2023242510 | A1 | 03 August 2023 |
| | | | | US | 11767312 | B2 | 26 September 2023 |
| | | | | CO | 2022017802 | A2 | 17 March 2023 |
| | | | | AU | 2021286738 | A1 | 19 January 2023 |
| | | | | BR | 112022025057 | A2 | 31 January 2023 |
| | | | | JP | 2023531390 | A | 24 July 2023 |
| | | | | PE | 20230610 | A1 | 13 April 2023 |
| | | | | WO | 2021249534 | A1 | 16 December 2021 |
| | | | | IN | 202347001743 | A | 13 January 2023 |
| | | | | CN | 113801098 | B | 30 May 2023 |
| | | | | CN | 116082311 | A | 09 May 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202310656044